# EUROPEAN PATENT SPECIFICATION

(11) **EP 4 251 064 B1**
(45) Date of publication and mention of the grant of the patent: **25.02.2026**
(21) Application number: 21831400.3
(22) Date of filing: 30.11.2021
(51) Int. Cl.: A61B 17/11, A61B 17/00, A61B 18/00, A61B 34/20, A61B 5/06, A61B 18/14, A61B 90/00, A61B 5/00, A61B 8/12, A61B 8/00

(54) **CATHETER FOR ENDOVASCULAR TREATMENT OF A BLOOD VESSEL**
KATHETER ZUR ENDOVASKULÄREN BEHANDLUNG EINES BLUTGEFÄSSES
CATHÉTER POUR LE TRAITEMENT ENDOVASCULAIRE D'UN VAISSEAU SANGUIN

(30) Priority: 30.11.2020 US 202063119239 P
(43) Date of publication of application: 04.10.2023
(73) Proprietor: TVA Medical, Inc., Franklin Lakes, NJ 07417 (US)
(72) Inventor: SIMPSON, Breanna, Franklin Lakes, New Jersey 07417 (US); PALMER, Olivia R., Franklin Lakes, New Jersey 07417 (US); MOLL, Andrew, Franklin Lakes, New Jersey 07417 (US); AKERELE-ALE, Oladipo Peter, Franklin Lakes, New Jersey 07417 (US); PALMER, Alex, Franklin Lakes, New Jersey 07417 (US); PEDE, Stephen, Franklin Lakes, New Jersey 07417 (US)
(74) Representative: Hoffmann Eitle
(86) International application number: PCT/US2021/061118
(87) International publication number: WO 2022/115752

(56) References cited:
- WO-A1-2020/242491
- US-A1- 2012 302 935
- US-A1- 2015 209 526

## Description

### TECHNICAL FIELD

The present specification generally relates to catheters for treatment of a blood vessel and, more specifically, catheters for endovascular treatment of a blood vessel.

### BACKGROUND

Endovascular treatments treat various blood vessel disorders from within the blood vessel using long, thin tubes called catheters, which are place inside the blood vessel to deliver the treatment. Endovascular treatments may include, but are not limited to, endovascular arteriovenous fistula (endoAVF) formations, arteriovenous (AV) treatments, and peripheral arterial disease (PAD) treatments. One of the most challenging aspects of endovascular treatment is proper alignment of a treatment portion of a catheter with the correct treatment location of the blood vessel. Additionally, treatments such as endovascular fistula formation may require two catheters positioned within adjacent blood vessels to form a fistula therebetween. However, alignment and position of two separate catheters may also be difficult/cumbersome for a practitioner.

Additionally, imaging systems for visualizing catheter alignment within blood vessels may also provide numerous hurdles to overcome. In particular fluoroscopy equipment is very expensive, accordingly such equipment might not be available outside of an operating room or in rural locations. Moreover, repeated use of fluoroscopy equipment may introduce radiation not only to the patient but also to the physician. Overtime, such repeat exposure may impact the physician's health. Additionally, contrast dyes used in fluoroscopy may not be suitable for patients with certain medical conditions (e.g., chronic kidney disease).

Accordingly, a need exists for alternative systems, methods, and catheters for endovascular treatment of a blood vessel that improve alignment techniques of the catheter within the blood vessel, and or catheters for endovascular treatment of a blood vessel that allow for simpler delivery of treatment to the blood vessel.
US 2015 209526 A1 describes devices and methods for guided vascular access creation.
US 2012 302935 A1 describes devices and methods for forming a fistula, wherein the devices and methods may be used to form a fistula between two blood vessels.
WO 2020 242491 A1 describes systems, methods, and catheters for treatment of a blood vessel and, more specifically, systems, methods, and catheters for endovascular treatment of a blood vessel.

### SUMMARY

The present embodiments address the above referenced problems. In particular, the present disclosure is directed to systems, methods, and catheters to improved visualization and alignment techniques for delivery of treatments (e.g., fistula formation) using one or more catheters to a blood vessel. Additionally, some embodiments are directed to single catheter systems which may reduce complexity of current two catheter systems. The present invention is defined by the appended independent claims. The dependent claims describe optional features and distinct embodiments.

In a first aspect, a system for endovascular treatment of a blood vessel includes a control unit, an ultrasound device, an actuator, and a catheter having a treatment portion. The ultrasound device is communicatively coupled to the control unit. The ultrasound device includes an ultrasound probe having a subject contact surface. The actuator is coupled to the ultrasound probe and is operable to move the subject contact surface of the ultrasound prove relative to a treatment zone of a subject. The control unit is configured to determine a position of the treatment portion of the catheter as the catheter is advanced through the blood vessel, and move the subject contact surface of the ultrasound probe relative to the treatment zone of the subject with the actuator to follow the position of the catheter as the catheter is advanced through the blood vessel.

In a second aspect, the present disclosure includes a system according to the first aspect, further including one or more user input devices communicatively coupled to the control unit, wherein the control unit is further configured to: received user input from the one or more user input device, and switch to a manual operation mode from an automatic following mode to allow for manual control of movement of the ultrasound probe based on input from the one or more user input devices.

In a third aspect, the present disclosure includes a system according to any preceding aspect, further including a display communicatively coupled to the control unit wherein the control unit is further configured to display one or more ultrasound images with the display in real time as the catheter is advanced through the blood vessel.

In a fourth aspect, the present disclosure includes a system according to the third aspect, wherein the control unit is further configured to determine an orientation of the treatment portion of the catheter within the blood vessel and output an indication of the orientation of the treatment portion of the catheter with the display.

In a fifth aspect, the present disclosure includes a system according to any preceding aspect, wherein the ultrasound device is a 3D ultrasound device and the control unit is configured to display two or more of a frontal plane ultrasound image, an axial plane ultrasound image, and sagittal plane ultrasound image.

In a sixth aspect, the present disclosure includes a system according to any preceding aspect, wherein the ultrasound device is a 3D ultrasound device and the control unit is configured to recognize one or more vessels within an ultrasound image of the ultrasound device and display a 3D model of the one or more vessels on the display.

In a seventh aspect, the present disclosure includes a system according to any preceding aspect, further including a media bath configured to be placed over a treatment zone of a subject, wherein the ultrasound device moves within the media bath.

In an eighth aspect, the present disclosure includes a system according to the seventh aspect, wherein the media bath comprises a flexible subject interface, wherein the flexible subject interface conforms to a shape of the treatment zone of the subject.

In a ninth aspect, the present disclosure includes a system according to the seventh aspect or the eighth aspect, wherein the media bath comprises a housing comprising a track, wherein the ultrasound device is moveable along the track.

In a tenth aspect, the present disclosure includes a system according to any preceding aspect, wherein the catheter comprises a housing, a cutting device, and a biasing mechanism coupled to the housing of the catheter and configured to bias the cutting device against a wall of the blood vessel.

In an eleventh aspect, the present disclosure includes a system according to the tenth aspect, wherein the biasing mechanism is a balloon.

In a twelfth aspect, the present disclosure includes a system according to the tenth aspect, wherein the biasing mechanism is an expandable cage.

In a thirteenth aspect, the present disclosure includes a system according to any of the tenth through twelfth aspects, wherein the biasing mechanism comprises one or more expandable wires moveable between a collapsed position and an expanded position wherein at least a portion of the one or more expandable wire are spaced from an outer wall of the housing of the catheter.

In a fourteenth aspect, the present disclosure includes a system according to any of the tenth through the thirteenth aspects, wherein the catheter comprises one or more echogenic markers, wherein the one or more echogenic markers indicate a rotational alignment of cutting device of the catheter.

In a fifteenth aspect, a system for endovascular treatment of a blood vessel includes a control unit, an imaging device, a display, and a catheter having a treatment portion. The imaging device and the display are communicatively coupled to the control unit. The control unit is configured to display an image of the blood vessel, determine a rotational orientation of the treatment portion of the catheter within the blood vessel, and output an indication of the rotational orientation of the treatment portion of the catheter with the display.

In a sixteenth aspect, the present disclosure includes a system according to the fifteenth aspect, wherein the indication comprises an overlay projected over the image of the blood vessel, the overlay providing an indicator of the rotational orientation of the treatment portion of the catheter.

In a seventeenth aspect, the present disclosure includes a system according to the fifteenth aspect or the sixteenth aspect, wherein the imaging device is an ultrasound imaging device.

In an eighteenth aspect, the present disclosure includes a system according to any of the fifteenth aspect through the seventeenth aspect, wherein the imaging device is an intravascular imaging device.

In a nineteenth aspect, the present disclosure includes a system according to any of the fifteenth aspect through the eighteenth aspect, wherein the imaging device is coupled to the catheter at a position distal to the treatment portion.

In a twentieth aspect, the present disclosure includes a system according to any of the fifteenth aspect through the nineteenth aspect, wherein the imaging device is coupled to the catheter at a position proximal to the treatment portion.

In a twenty-first aspect, the present disclosure includes a system according to any of the fifteenth aspect through the twentieth aspect, wherein the imaging device is a 3D ultrasound device and the control unit is configured to display two or more of a frontal plane ultrasound image, an axial plane ultrasound image, and sagittal plane ultrasound image with the display.

In a twenty-second aspect, the present disclosure includes a system according to any of the fifteenth aspect through the twenty-first aspect, wherein the ultrasound device is a 3D ultrasound device and the control unit is configured to recognize one or more vessels within an ultrasound image of the ultrasound device and display a 3D model of the one or more vessels on the display.

In a twenty-third aspect, the present disclosure includes a system according to any of the fifteenth aspect through the twenty-second aspect, wherein the catheter comprises a housing, wherein the treatment portion of the catheter is coupled to the housing of the catheter at a first radial position.

In a twenty-fourth aspect, the present disclosure includes a system according to the twenty-third aspect, wherein the catheter further comprises a biasing mechanism coupled to the housing of the catheter, the biasing mechanism configured to bias the treatment portion of the catheter toward a wall of the blood vessel.

In a twenty-fifth aspect, the present disclosure includes a system according to the twenty-fourth aspect, wherein the biasing mechanism is coupled to the housing of the catheter proximate to the treatment portion.

In a twenty-sixth aspect, the present disclosure includes a system according to any of the fifteenth aspect through the twenty-fifth aspect, wherein the treatment portion comprises a cutting device.

In a twenty-seventh aspect, the present disclosure includes a system according to any of the twenty-fourth aspect through the twenty-sixth aspect when depending on the twenty-fourth aspect, wherein the biasing mechanism is a balloon.

In a twenty-ninth aspect, the present disclosure includes a system according to any of the twenty-fourth aspect through the twenty-sixth aspect when depending on the twenty-fourth aspect, wherein the biasing mechanism comprises one or more expandable wires moveable between a collapsed position and an expanded position wherein at least a portion of the one or more expandable wire are spaced from an outer wall of the housing of the catheter.

In a thirtieth aspect, the present disclosure includes a system according to any of the fifteenth aspect through the twenty-ninth aspect, wherein the catheter comprises one or more echogenic markers, wherein the one or more echogenic markers indicate a rotational alignment of cutting device of the catheter.

In a thirty-first aspect, the present disclosure includes a catheter for endovascular treatment of a blood vessel includes a housing, a treatment portion coupled to the housing, an intravascular imaging device coupled to the housing, and a biasing mechanism coupled to the housing. The biasing mechanism is configured to contact a wall of the blood vessel to bias the treatment portion into contact with the wall of the blood vessel.

In a thirty-second aspect, the present disclosure includes a system according to the thirty-first aspect, wherein the biasing mechanism is configured to contact a first radial portion of the wall of the blood vessel to bias the treatment portion toward a second radial portion of the wall of the blood vessel opposite the first radial portion.

In a thirty-third aspect, the present disclosure includes a system according to the thirty-first aspect or the thirty-second aspect, wherein the intravascular imaging device is coupled to the housing of the catheter at a position distal to the treatment portion.

In a thirty-fourth aspect, the present disclosure includes a system according to the thirty-first aspect or the thirty-second aspect, wherein the intravascular imaging device is coupled to the housing of the catheter at a position proximal to the treatment portion.

In a thirty-fifth aspect, the present disclosure includes a system according to the thirty-first aspect or the thirty-second aspect, wherein the intravascular imaging device is coupled to the housing of the catheter at a position longitudinally aligned with the treatment portion of the catheter.

In a thirty-sixth aspect, the present disclosure includes a system according to any of the thirty-first aspect through the thirty-fifth aspect, wherein the treatment portion comprises an electrode comprising an arc that extends from the housing, and wherein the intravascular imaging device is positioned so as to capture image data of a cross-section of the catheter taken perpendicular to a longitudinal direction of the catheter at a apex of the arc.

In a thirty-seventh aspect, the present disclosure includes a system according to any of the thirty-first aspect through the thirty-sixth aspect, wherein the intravascular imaging device is positioned longitudinally within the treatment portion of the catheter.

In a thirty-eighth aspect, the present disclosure includes a system according to any of the thirty-first aspect through the thirty-seventh aspect, wherein the treatment portion comprises an electrode, wherein the intravascular imaging device is positioned longitudinally with the treatment portion of the catheter, so as to capture image data of a cross-section of the electrode.

In a thirty-ninth aspect, the present disclosure includes a system according to any of the thirty-first aspect through the thirty-eighth aspect, wherein the biasing mechanism is coupled to the housing of the catheter proximate to the treatment portion.

In a fortieth aspect, the present disclosure includes a system according to any of the thirty-first aspect through the thirty-ninth aspect, wherein the treatment portion comprises a cutting device.

In a forty-first aspect, the present disclosure includes a system according to any of the thirty-first aspect through the fortieth aspect, wherein the biasing mechanism is a balloon.

In a forty-second aspect, the present disclosure includes a system according to any of the thirty-first aspect through the fortieth aspect, where in the biasing mechanism is an expandable cage.

In a forty-third aspect, a method for endovascular treatment of a blood vessel includes advancing a catheter within the blood vessel to a treatment location of the blood vessel, aligning a treatment portion of the catheter with the treatment location of the blood vessel, and deploying the catheter with a biasing mechanism coupled to a body of the catheter. The biasing mechanism is configured to contact a first radial portion of the blood vessel to bias the treatment portion of the catheter toward the treatment location of the blood vessel opposite the first radial portion.

In a forty-fourth aspect, the present disclosure includes a method according to the forty-third aspect, wherein the treatment portion comprises a cutting device.

In a forty-fifth aspect, the present disclosure includes a method according to the forty-third aspect or the forty-fourth aspect, wherein the biasing mechanism is a balloon.

In a forty-sixth aspect, the present disclosure includes a method according to the forty-third aspect or the forty-fourth aspect, where in the biasing mechanism is an expandable cage.

In a forty-seventh aspect, the present disclosure includes a method according to any of the forty-third aspect through the forty-sixth aspect, wherein the biasing mechanism is coupled to the housing of the catheter proximate to the treatment portion.

In a forty-eighth aspect, the present disclosure includes a method according to any of the forty-third aspect through the forty-seventh aspect, further including determining a position of the treatment portion of the catheter as the catheter is advanced through the blood vessel with a control unit, moving an imaging device with an actuator to follow the position of the catheter as the catheter is advanced through the blood vessel, and displaying one or more images from the imaging device with a display in real time as the catheter is advanced through the blood vessel.

In a forty-ninth aspect, the present disclosure includes a method according to any of the forty-third aspect through the forty-eighth aspect, further including capturing image data with an imaging device coupled to the catheter, and displaying image data from the imaging device with a display in real time as the catheter is advanced through the blood vessel.

In a fiftieth aspect, the present disclosure includes a method according to any of the forty-third aspect through the forty-ninth aspect, further including determining a rotational alignment of the catheter, and displaying an indication of the rotational alignment of the catheter with the display.

In a fifty-first aspect, the present disclosure includes a method according to any of the forty-third aspect through the fiftieth aspect, further including determining a rotational alignment of the treatment portion of the catheter, and displaying an indication of the rotational alignment of the treatment portion of the catheter with the display.

In a fifty-second aspect, the present disclosure includes a method according to any of the forty-third aspect through the fifty-first aspect, further including automatically adjusting the imaging device to automatically focus the imaging device on the treatment portion of the catheter to adjust image quality using one or more location sensors and/or echogenic markers.

In a fifty-third aspect, the present disclosure includes a system according to any of the fifteenth through thirtieth aspect, wherein the imaging device is an ultrasound device, and the control unit is configured to: recognize an arterial blood flow using a Doppler functionality of the ultrasound device; recognize a venous blood flow using the Doppler Functionality of the ultrasound device; and display a blood vessel map based on the arterial blood flow and the venous blood flow.

In a fifty-fourth aspect, the present disclosure includes a system according to the fifty-third aspect, wherein the arterial blood flow is depicted as a first color and the venous blood flow is depicted as a second color different from the first color in the blood vessel map.

In a fifty-fifth aspect, the present disclosure includes a system according to the fifty-third aspect or the fifty-fourth aspect, wherein the control unit is configured to recognize fistula creation by identifying blood flow between an adjacent artery and vein using the Doppler functionality of the ultrasound device.

In a fifty-sixth aspect, the present disclosure include a control unit for endovascular treatment of a blood vessel with one or more catheters. The control unit includes one or more process and one or more memory modules communicatively coupled to the one or more processors. The control unit is configured to be communicatively coupled to an imaging device and a display. When the one or more processors execute logic stored on the one or more memory modules, the control unit displays the image data from the imaging device of a blood vessel, determines a rotational orientation of a treatment portion of a catheter within a blood vessel, and outputs an indication of the rotational orientation of the treatment portion of the catheter with the display.

In a fifty-seventh aspect, the present disclosure includes a control unit according to the fifty-sixth aspect, wherein the indication comprises an overlay projected over the image of the blood vessel, the overlay providing an indicator of a rotational orientation of the treatment portion of the catheter.

In a fifty-eighth aspect, the present disclosure includes a control unit according to the fifty-sixth aspect or the fifty-seventh aspect, wherein the imaging device is an ultrasound imaging device.

In a fifty-ninth aspect, the present disclosure includes a control unit according to any of the fifty-sixth aspect through the fifty-eighth aspect, wherein the imaging device is an intravascular imaging device.

In a sixtieth aspect, the present disclosure includes a control unit according to any of the fifty-sixth aspect through the fifty-ninth aspect, wherein the imaging device is coupled to the catheter at a position distal to the treatment portion.

In a sixty-first aspect, the present disclosure includes a control unit according to any of the fifty-sixth aspect through the fifty-ninth aspect, wherein the imaging device is coupled to the catheter at a position proximal to the treatment portion.

In a sixty-second aspect, the present disclosure includes a control unit according to any of the fifty-sixth aspect through the sixty-first aspect, wherein the imaging device is a 3D ultrasound device and the control unit is configured to display two or more of a frontal plane ultrasound image, an axial plane ultrasound image, and sagittal plane ultrasound image with the display.

In a sixty-third aspect, the present disclosure includes a control unit according to any of the fifty-sixth aspect through the sixty-second aspect, wherein the ultrasound device is a 3D ultrasound device and the control unit is configured to recognize one or more vessels within an ultrasound image of the ultrasound device and display a 3D model of the one or more vessels on the display.

In a sixty-fourth aspect, the present disclosure includes a control unit according to any of the fifty-sixth aspect through the sixty-third aspect, wherein the control unit is configured to be communicatively coupled to one or more location sensors coupled to the catheter, the one or more location sensors outputting a location signal indicative of a location of the treatment portion, wherein the control unit is configured to determine a location of the treatment portion based on the signal from the one or more location sensors.

In a sixty-fifth aspect, the present disclosure includes a control unit according to any of the fifty-sixth aspect through the sixty-fourth aspect, wherein the imaging device is an ultrasound device, and the control unit is configured to: recognize an arterial blood flow using a Doppler functionality of the ultrasound device; recognize a venous blood flow using the Doppler functionality of the ultrasound device; and display a blood vessel map based on the arterial blood flow and the venous blood flow.

In a sixty-sixth aspect, the present disclosure includes a control unit according to the sixty-fifth aspect, wherein the arterial blood flow is depicted as a first color and the venous blood flow is depicted as a second color different from the first color in the blood vessel map.

In a sixty-seventh aspect, the present disclosure includes a control unit according to any of the fifty-sixth aspect through the sixty-sixth aspect, herein the control unit is configured to recognize fistula creation by identifying blood flow between an adjacent artery and vein using the Doppler functionality of the ultrasound device.

In a sixty-eighth aspect, the present disclosure includes a control unit for endovascular treatment of a blood vessel using one or more catheters. The control unit includes one or more processors and one or more memory modules communicatively coupled to the one or more processors. The control unit is configured to be communicatively coupled to an ultrasound probe having a subject contact surface, and an actuator coupled to the ultrasound probe. When the one or more processors execute logic stored on the one or more memory modules, the control unit determines a position of a treatment portion of a catheter as the catheter is advanced through the blood vessel; and moves a subject contact surface of the ultrasound probe relative to the treatment zone of the subject with the actuator to follow the position of the catheter as the catheter is advanced through the blood vessel.

In a sixty-ninth aspect, the present disclosure includes a control unit according to the sixty-eight aspect, wherein the control unit is configured to be communicatively coupled to one or more user input devices, wherein the control unit is further configured to: receive user input from the one or more user input devices; and switch to a manual operation mode from an automatic following mode to allow for manual control of movement of the ultrasound probe based on input from the one or more user input devices.

In a seventieth aspect, the present disclosure includes a control unit according to the sixty-eight aspect or the sixty-ninth aspect, wherein the control unit is configured to be communicatively coupled to a display, wherein the control unit is further configured to: display one or more ultrasound images with the display in real time as the catheter is advanced through the blood vessel.

In a seventy-first aspect, the present disclosure includes a control unit according to any of the sixty-eighth aspect through the seventieth aspect, wherein the control unit is further configured to determine an orientation of the treatment portion of the catheter within the blood vessel and output an indication of the orientation of the treatment portion of the catheter with the display.

In a seventy-second aspect, the present disclosure includes a control unit according to any of the sixty-eighth aspect through the seventy-first aspect, wherein the ultrasound device is a 3D ultrasound device and the control unit is configured to display two or more of a frontal plane ultrasound image, an axial plane ultrasound image, and sagittal plane ultrasound image.

In a seventy-third aspect, the present disclosure includes a control unit according to any of the sixty-eighth aspect through the seventy-second aspect, wherein the ultrasound device is a 3D ultrasound device and the control unit is configured to recognize one or more vessels within an ultrasound image of the ultrasound device and display a 3D model of the one or more vessels on the display.

In a seventy-fourth aspect, the present disclosure includes a control unit according to any of the sixty-eight aspect through the seventy-third aspect, wherein the catheter comprises one or more echogenic markers, and the control unit is configured to determine a rotational orientation of the catheter based on recognition of the one or more echogenic markers.

In a seventy-fifth aspect, the present disclosure includes a control unit according to any of the sixty-eight aspect through the seventy-fourth aspect, wherein the control unit is configured to be communicatively coupled to one or more location sensors coupled to the catheter, the one or more location sensors outputting a location signal indicative of a location of the treatment portion, wherein the control unit is configured to determine a location of the treatment portion based on the signal from the one or more location sensors.

In a seventy-sixth aspect, the present disclosure includes a catheter for endovascular treatment of a blood vessel, the catheter comprising: a housing; a treatment portion; a fistula forming element; and an intravascular ultrasound imaging device.

In a seventy-seventh aspect, the present disclosure includes the catheter according to the seventy-sixth aspect, wherein the intravascular ultrasound imaging device further comprises an array of solid-state transducers.

In a seventy-eighth aspect, the present disclosure includes the catheter according to any of the seventy-sixth through seventy-seventh aspects, wherein the array of solid-state transducers are disposed distal the fistula forming element.

In a seventy-ninth aspect, the present disclosure includes the catheter according to any of the seventy-sixth through seventy-eighth aspects, wherein the array of solid-state transducers are disposed proximal the fistula forming element.

In an eightieth aspect, the present disclosure includes the catheter according to any of the seventy-sixth through seventy-ninth aspects, wherein the array of solid-state transducers are coupled to the housing of the catheter.

In an eighty-first aspect, the present disclosure includes the catheter according to any of the seventy-sixth through eightieth aspects, wherein the array of solid-state transducers are disposed circumferentially around the housing of the catheter.

In an eighty-second aspect, the present disclosure includes the catheter according to any of the seventy-sixth through eighty-first aspects, wherein the intravascular ultrasound imaging device further comprises one or more flexible circuit elements electrically coupled to the array of solid-state transducers.

In an eighty-third aspect, the present disclosure includes the catheter according to any of the seventy-sixth through eighty-second aspects, wherein the one or more flexible circuit elements further comprise one or more multiplexing application-specific integrated circuits.

In an eighty-fourth aspect, the present disclosure includes the catheter according to any of the seventy-sixth through eighty-third aspects, wherein the one or more flexible circuit elements are positioned within the housing of catheter.

In an eighty-fifth aspect, the present disclosure includes the catheter according to any of the seventy-sixth through eighty-fourth aspects, further comprising an intravascular ultrasound imaging device wire extending proximally from the one or more flexible circuit elements through the housing of the catheter.

In an eighty-sixth aspect, the present disclosure includes the catheter according to any of the seventy-sixth through eighty-fifth aspects, wherein the one or more flexible circuit elements are positioned distal the fistula forming element and proximal the array of solid-state transducers.

In an eighty-seventh aspect, the present disclosure includes the catheter according to any of the seventy-sixth through eighty-sixth aspects, wherein: the one or more flexible circuit elements are positioned proximal the array of solid-state transducers; and the array of solid-state transducers are positioned proximal the fistula forming element.

In an eighty-eighth aspect, the present disclosure includes the catheter according to any of the seventy-sixth through eighty-seventh aspects, wherein the fistula forming element is an electrode configured to ablate tissue.

In an eighty-ninth aspect, the present disclosure includes the catheter according to any of the seventy-sixth through eighty-eighth aspects, wherein: the electrode housing is positioned along the treatment portion of the catheter; and the electrode housing is at least partially positioned within the housing of the catheter.

In a ninetieth aspect, the present disclosure includes the catheter according to any of the seventy-sixth through eighty-ninth aspects, wherein the electrode is housed within the electrode housing when the electrode is in a low-profile configuration.

In a ninety-first aspect, the present disclosure includes the catheter according to any of the seventy-sixth through ninetieth aspects, wherein the electrode is configured to radially extend from the electrode housing and the housing of the catheter when the electrode is in an extended configuration.

In a ninety-second aspect, the present disclosure includes the catheter according to any of the seventy-sixth through ninety-first aspects, wherein the electrode is spring biased from a low-profile configuration, wherein the electrode is housed within the electrode housing, to an extended configuration, wherein the electrode radially extends from the electrode housing and the housing of the catheter.

In a ninety-third aspect, the present disclosure includes the catheter according to any of the seventy-sixth through ninety-second aspects, wherein the intravascular ultrasound imaging device further comprises: an array of solid-state transducers; one or more flexible circuit elements electrically coupled to the array of solid-state transducers; and an intravascular ultrasound imaging device wire extending proximally from the one or more flexible circuit elements through the housing of the catheter.

In a ninety-fourth aspect, the present disclosure includes the catheter according to any of the seventy-sixth through ninety-third aspects, wherein: the electrode housing further comprises a channel extending therethrough; the array of solid-state transducers are positioned distal the electrode housing; the one or more flexible circuit elements are positioned distal the electrode housing; and the intravascular ultrasound imaging device wire extends through the channel extending through the electrode housing.

In a ninety-fifth aspect, the present disclosure includes the catheter according to any of the seventy-sixth through ninety-fourth aspects, further comprising: a first lumen extending through the housing of the catheter; and a second lumen extending through the housing of the catheter, wherein: the electrode further comprises an electrode wire electrically coupled to the electrode and extending proximally from the electrode through the housing of the catheter in the first lumen; and the intravascular ultrasound imaging device wire extends through the second lumen.

In a ninety-sixth aspect, the present disclosure includes the catheter according to any of the seventy-sixth through ninety-fifth aspects, wherein the first lumen further comprises an insulation sleeve.

In a ninety-seventh aspect, the present disclosure includes the catheter according to any of the seventy-sixth through ninety-sixth aspects, further comprising a biasing mechanism coupled to the housing, wherein the biasing mechanism is configured to contact a wall of the blood vessel to bias the treatment portion into contact with the wall of the blood vessel.

In a ninety-eighth aspect, the present disclosure includes the catheter according to any of the seventy-sixth through ninety-seventh aspects, wherein the biasing mechanism is configured to contact a first radial portion of the wall of the blood vessel to bias the treatment portion toward a second radial portion of the wall of the blood vessel opposite the first radial portion.

In a ninety-ninth aspect, the present disclosure includes the catheter according to any of the seventy-sixth through ninety-eighth aspects, wherein the biasing mechanism is a balloon.

In a one-hundredth aspect, the present disclosure includes the catheter according to any of the seventy-sixth through ninety-ninth aspects, wherein the biasing mechanism is an expandable cage.

In a one-hundred-first aspect, the present disclosure includes the catheter according to any of the seventy-sixth through one-hundredth aspects, wherein the biasing mechanism comprises one or more expandable wires moveable between a collapsed position and an expanded position wherein at least a portion of the one or more expandable wires are spaced from an outer wall of the housing of the catheter.

In a one-hundred-second aspect, the present disclosure includes the catheter according to any of the seventy-sixth through one-hundred-first aspects, wherein the housing further comprises a rapid exchange tip at a distal end of the catheter.

In a one-hundred-third aspect, the present disclosure includes the catheter according to any of the seventy-sixth through one-hundred-second aspects, wherein the housing is comprised of one or more polymers.

In a one-hundred-fourth aspect, the present disclosure includes the catheter according to any of the seventy-sixth through one-hundred-third aspects, wherein the housing is comprised of silicone rubber, nylon, polyurethane, polyethylene terephthalate, latex, or thermoplastic elastomers.

In a one-hundred-fifth aspect, the present disclosure includes the catheter according to any of the seventy-sixth through one-hundred-fourth aspects, wherein the housing is further comprised of a braided polymer.

In a one-hundred-sixth aspect, the present disclosure includes a method for forming a fistula in a blood vessel, the method comprising: advancing a catheter within the blood vessel to a treatment location of the blood vessel; generating an image of the blood vessel from data collected from an intravascular ultrasound imaging device of the catheter; aligning a treatment portion of the catheter with the treatment location of the blood vessel; deploying the catheter with a biasing mechanism coupled to a housing of the catheter, wherein the biasing mechanism is configured to contact a first radial portion of the blood vessel to bias the treatment portion of the catheter toward the treatment location of the blood vessel opposite the first radial portion; deploying an electrode from the treatment portion; and ablating the blood vessel at the treatment location.

In a one-hundred-seventh aspect, the present disclosure includes the method according to the one-hundred-sixth aspect, wherein the intravascular ultrasound imaging device further comprises: an array of solid-state transducers; one or more flexible circuit elements electrically coupled to the array of solid-state transducers and positioned within a housing of the catheter; and an intravascular ultrasound imaging device wire extending proximally from the one or more flexible circuit elements through the housing of the catheter.

In a one-hundred-eighth aspect, the present disclosure includes the method according to any of the one-hundred-sixth through one-hundred-seventh aspects, wherein the array of solid-state transducers are disposed circumferentially around the housing of the catheter.

In a one-hundred-ninth aspect, the present disclosure includes the method according to any of the one-hundred-sixth through one-hundred-eighth aspects, wherein the biasing mechanism is a balloon.

In a one-hundred-tenth aspect, the present disclosure includes the method according to any of the one-hundred-sixth through one-hundred-ninth aspects, wherein the biasing mechanism is an expandable cage.

In a one-hundred-eleventh aspect, the present disclosure includes the method according to any of the one-hundred-sixth through one-hundred-tenth aspects, wherein the biasing mechanism comprises one or more expandable wires moveable between a collapsed position and an expanded position wherein at least a portion of the one or more expandable wires are spaced from an outer wall of the housing of the catheter.

In a one-hundred-twelfth aspect, the present disclosure includes a system for forming a fistula between two blood vessels, comprising: a first catheter comprising a first catheter body, an electrode, and a biasing stent, wherein: the electrode is configured to project from a treatment portion of the first catheter and define an active side of the first catheter; the biasing stent longitudinally extends along a length of the first catheter body and is configured to radially extend away from the first catheter body between a proximal point and a distal point; and the biasing stent extends from a non-active side of the first catheter body such that the biasing stent is configured to bias the treatment portion against a first blood vessel wall; and a second catheter.

In a one-hundred-thirteenth aspect, the present disclosure includes the system according to the one-hundred-twelfth aspect, wherein the biasing stent comprises: a first tapered section; a second tapered section; and a third section longitudinally positioned between the first tapered section and the second tapered section.

In a one-hundred-fourteenth aspect, the present disclosure includes the system of any of the one-hundred-twelfth through one-hundred-thirteenth aspects, wherein: the first tapered section is coupled to the first catheter body at a proximal point of the first tapered section, and the first tapered section connects to the third section of the biasing stent at a distal point of the first tapered section; and the second tapered section is coupled to the first catheter body at a distal point of the second tapered section, and the second tapered section connects to the third section of the biasing stent at a proximal point of the second tapered section.

In a one-hundred-fifteenth aspect, the present disclosure includes the system of any of the one-hundred-twelfth through one-hundred-fourteenth aspects, wherein when the biasing stent is in an extended configuration: the distal point of the first tapered section is a greater distance from the first catheter body than the proximal point of the first tapered section; and the proximal point of the second tapered section is a greater distance from the first catheter body than the distal point of the second tapered section.

In a one-hundred-sixteenth aspect, the present disclosure includes the system of any of the one-hundred-twelfth through one-hundred-fifteenth aspects, wherein when the biasing stent is in an extended configuration: the first tapered section slopes from the first catheter body to the third section of the biasing stent; and the second tapered section slopes from the first catheter body to the third section of the biasing stent.

In a one-hundred-seventeenth aspect, the present disclosure includes the system of any of the one-hundred-twelfth through one-hundred-sixteenth aspects, wherein the biasing stent comprises an elliptic cross section.

In a one-hundred-eighteenth aspect, the present disclosure includes the system of any of the one-hundred-twelfth through one-hundred-seventeenth aspects, wherein the biasing stent comprises a semi-elliptic cross section.

In a one-hundred-nineteenth aspect, the present disclosure includes the system of any of the one-hundred-twelfth through one-hundred-eighteenth aspects, wherein the first catheter further comprises a sheath configured to be advanced distally to compress the biasing stent against the first catheter body.

In a one-hundred-twentieth aspect, the present disclosure includes the system of any of the one-hundred-twelfth through one-hundred-nineteenth aspects, wherein the biasing stent comprises nitinol, stainless steel, polyether ether ketone, polyethylene terephthalate, polyimide, or polytetrafluoroethylene.

In a one-hundred-twenty-first aspect, the present disclosure includes the system of any of the one-hundred-twelfth through one-hundred-twentieth aspects, wherein: the biasing stent comprises a plurality of interconnected struts; and the plurality of interconnected struts comprise substantially flat ribbons having a substantially rectangular cross section.

In a one-hundred-twenty-second aspect, the present disclosure includes the system of any of the one-hundred-twelfth through one-hundred-twenty-first aspects, wherein: the biasing stent comprises a plurality of interconnected struts; and the plurality of interconnected struts comprise a circular cross section.

In a one-hundred-twenty-third aspect, the present disclosure includes the system of any of the one-hundred-twelfth through one-hundred-twenty-second aspects, wherein the treatment portion is longitudinally positioned between the proximal point and the distal point.

In a one-hundred-twenty-fourth aspect, the present disclosure includes the system of any of the one-hundred-twelfth through one-hundred-twenty-third aspects, wherein the distal point is positioned on the non-active side of the treatment portion.

In a one-hundred-twenty-fifth aspect, the present disclosure includes the system of any of the one-hundred-twelfth through one-hundred-twenty-fourth aspects, wherein the distal point is positioned proximal a distal tip of the first catheter body.

In a one-hundred-twenty-sixth aspect, the present disclosure includes the system of any of the one-hundred-twelfth through one-hundred-twenty-fifth aspects, wherein the biasing stent is rigidly coupled to the first catheter body at the distal point and the proximal point with an adhesive or a polymer.

In a one-hundred-twenty-seventh aspect, the present disclosure includes the system of any of the one-hundred-twelfth through one-hundred-twenty-sixth aspects, wherein at least one of the proximal point or the distal point at least partially define a track within the first catheter body, and at least a portion of the biasing stent is configured to slide within the track of the first catheter body between an extended position and a low-profile position.

In a one-hundred-twenty-eighth aspect, the present disclosure includes the system of any of the one-hundred-twelfth through one-hundred-twenty-seventh aspects, wherein the first catheter comprises one or more arrays of magnets arranged longitudinally along the first catheter body.

In a one-hundred-twenty-ninth aspect, the present disclosure includes the system of any of the one-hundred-twelfth through one-hundred-twenty-eighth aspects, wherein: the proximal point is positioned proximal a first end of at least one array of the one or more arrays of magnets and the distal point is positioned distal to a second end of the at least one array of the one or more arrays of magnets such that the biasing stent longitudinally spans the at least one array of the one or more arrays of magnets.

In a one-hundred-thirtieth aspect, the present disclosure includes the system of any of the one-hundred-twelfth through one-hundred-twenty-ninth aspects, wherein at least one of the proximal point or the distal point is longitudinally positioned between a first end of at least one array of the one or more arrays of magnets and a second end of the at least one array of the one or more arrays of magnets.

In a one-hundred-thirty-first aspect, the present disclosure includes the system of any of the one-hundred-twelfth through one-hundred-thirtieth aspects, wherein the first catheter is configured to be positioned within a first blood vessel and the second catheter is configured to be positioned within a second blood vessel adjacent to the first blood vessel.

In a one-hundred-thirty-second aspect, the present disclosure includes the system of any of the one-hundred-twelfth through one-hundred-thirty-first aspects, wherein the second catheter further comprises a recessed region defining an active side of the second catheter, wherein the recessed region is configured to receive the electrode of the first catheter.

In a one-hundred-thirty-third aspect, the present disclosure includes the system of any of the one-hundred-twelfth through one-hundred-thirty-second aspects, wherein the second catheter further comprises: a second catheter body; and a second biasing stent, wherein: the second biasing stent longitudinally extends along a length of the second catheter body and is configured to radially extend away from the second catheter body between a proximal point and a distal point; and the second biasing stent extends from a non-active side of the second catheter body such that the second biasing stent is configured to bias the recessed region against a second blood vessel wall.

In a one-hundred-thirty-fourth aspect, the present disclosure includes the system of any of the one-hundred-twelfth through one-hundred-thirty-third aspects, wherein the recessed region is longitudinally positioned between the proximal point and the distal point.

In a one-hundred-thirty-fifth aspect, the present disclosure includes the system of any of the one-hundred-twelfth through one-hundred-thirty-fourth aspects, wherein the second catheter further comprises one or more arrays of magnets arranged longitudinally along the second catheter body.

In a one-hundred-thirty-sixth aspect, the present disclosure includes a catheter, comprising: a catheter body, an electrode, and a biasing stent, wherein: the electrode is configured to project from a treatment portion of the catheter and define an active side of the catheter; the biasing stent longitudinally extends along a length of the catheter body and is configured to radially extend away from the catheter body between a proximal point and a distal point; and the biasing stent extends from a non-active side of the catheter body such that the biasing stent is configured to bias the treatment portion against a first blood vessel wall.

In a one-hundred-thirty-seventh aspect, the present disclosure includes the catheter according to the one-hundred-thirty-sixth aspect, wherein the biasing stent comprises: a first tapered section; a second tapered section; and a third section longitudinally positioned between the first tapered section and the second tapered section.

In a one-hundred-thirty-eighth aspect, the present disclosure includes the catheter of any of the one-hundred-thirty-sixth through one-hundred-thirty-seventh aspects, wherein: the first tapered section is coupled to the catheter body at a proximal point of the first tapered section, and the first tapered section connects to the third section of the biasing stent at a distal point of the first tapered section; and the second tapered section is coupled to the catheter body at a distal point of the second tapered section, and the second tapered section connects to the third section of the biasing stent at a proximal point of the second tapered section.

In a one-hundred-thirty-ninth aspect, the present disclosure includes the catheter of any of the one-hundred-thirty-sixth through one-hundred-thirty-eighth aspects, wherein when the biasing stent is in an extended configuration: the distal point of the first tapered section is a greater distance from the catheter body than the proximal point of the first tapered section; and the proximal point of the second tapered section is a greater distance from the catheter body than the distal point of the second tapered section.

In a one-hundred-fortieth aspect, the present disclosure includes the catheter of any of the one-hundred-thirty-sixth through one-hundred-thirty-ninth aspects, wherein when the biasing stent is in an extended configuration: the first tapered section slopes from the catheter body to the third section of the biasing stent; and the second tapered section slopes from the catheter body to the third section of the biasing stent.

In a one-hundred-forty-first aspect, the present disclosure includes the catheter of any of the one-hundred-thirty-sixth through one-hundred-fortieth aspects, wherein the biasing stent comprises an elliptic cross section.

In a one-hundred-forty-second aspect, the present disclosure includes the catheter of any of the one-hundred-thirty-sixth through one-hundred-forty-first aspects, wherein the biasing stent comprises a semi-elliptic cross section.

In a one-hundred-forty-third aspect, the present disclosure includes the catheter of any of the one-hundred-thirty-sixth through one-hundred-forty-second aspects, further comprising a sheath configured to be advanced distally to compress the biasing stent against the catheter body.

In a one-hundred-forty-fourth aspect, the present disclosure includes the catheter of any of the one-hundred-thirty-sixth through one-hundred-forty-third aspects, wherein the biasing stent comprises nitinol, stainless steel, polyether ether ketone, polyethylene terephthalate, polyimide, or polytetrafluoroethylene.

In a one-hundred-forty-fifth aspect, the present disclosure includes the catheter of any of the one-hundred-thirty-sixth through one-hundred-forty-fourth aspects, wherein: the biasing stent comprises a plurality of interconnected struts; and the plurality of interconnected struts comprise substantially flat ribbons having a substantially rectangular cross section.

In a one-hundred-forty-sixth aspect, the present disclosure includes the catheter of any of the one-hundred-thirty-sixth through one-hundred-forty-fifth aspects, wherein: the biasing stent comprises a plurality of interconnected struts; and the plurality of interconnected struts comprise a circular cross section.

In a one-hundred-forty-seventh aspect, the present disclosure includes the catheter of any of the one-hundred-thirty-sixth through one-hundred-forty-sixth aspects, wherein the treatment portion is longitudinally positioned between the proximal point and the distal point.

In a one-hundred-forty-eighth aspect, the present disclosure includes the catheter of any of the one-hundred-thirty-sixth through one-hundred-forty-seventh aspects, wherein the distal point is positioned on the non-active side of the treatment portion.

In a one-hundred-forty-ninth aspect, the present disclosure includes the catheter of any of the one-hundred-thirty-sixth through one-hundred-forty-eighth aspects, wherein the distal point is positioned proximal a distal tip of the catheter body.

In a one-hundred-fiftieth aspect, the present disclosure includes the catheter of any of the one-hundred-thirty-sixth through one-hundred-forty-ninth aspects, wherein the biasing stent is rigidly coupled to the catheter body at the distal point and the proximal point with an adhesive or a polymer.

In a one-hundred-fifty-first aspect, the present disclosure includes the catheter of any of the one-hundred-thirty-sixth through one-hundred-fiftieth aspects, wherein at least one of the proximal point or the distal point at least partially define a track within the catheter body, and at least a portion of the biasing stent is configured to slide within the track of the catheter body between an extended position and a low-profile position

In a one-hundred-fifty-second aspect, the present disclosure includes the catheter of any of the one-hundred-thirty-sixth through one-hundred-fifty-first aspects, further comprising one or more arrays of magnets arranged longitudinally along the catheter body.

In a one-hundred-fifty-third aspect, the present disclosure includes the catheter of any of the one-hundred-thirty-sixth through one-hundred-fifty-second aspects, wherein: the proximal point is positioned proximal a first end of at least one array of the one or more arrays of magnets and the distal point is positioned distal to a second end of the at least one array of the one or more arrays of magnets such that the biasing stent longitudinally spans the at least one array of the one or more arrays of magnets.

In a one-hundred-fifty-fourth aspect, the present disclosure includes the catheter of any of the one-hundred-thirty-sixth through one-hundred-fifty-third aspects, wherein at least one of the proximal point or the distal point is longitudinally positioned between a first end of at least one array of the one or more arrays of magnets and a second end of the at least one array of the one or more arrays of magnets.

In a one-hundred-fifty-fifth aspect, the present disclosure includes a method of forming a fistula between a first blood vessel and a second blood vessel, comprising: advancing a first catheter into the first blood vessel, wherein the first catheter comprises: a first catheter body, an electrode, and a first biasing stent, wherein: the electrode is configured to project from a treatment portion of the first catheter and define an active side of the first catheter; the first biasing stent longitudinally extends along a length of the first catheter body and is configured to radially extend away from the first catheter body between a proximal point and a distal point; and the first biasing stent extends from a non-active side of the first catheter body such that the first biasing stent is configured to bias the treatment portion against a first blood vessel wall; and ablating tissue with the electrode to form the fistula.

In a one-hundred-fifty-sixth aspect, the present disclosure includes the method according to the one-hundred-fifty-fifth aspect, wherein the treatment portion is longitudinally positioned between the proximal point and the distal point.

In a one-hundred-fifty-seventh aspect, the present disclosure includes the method of any of the one-hundred-fifty-fifth through one-hundred-fifty-sixth aspects, further comprising: advancing a second catheter into the second blood vessel; and aligning the electrode of the first catheter with the second catheter.

In a one-hundred-fifty-eighth aspect, the present disclosure includes the method of any of the one-hundred-fifty-fifth through one-hundred-fifty-seventh aspects, wherein the second catheter comprises a recess, wherein aligning the electrode of the first catheter with the second catheter comprises aligning the electrode with the recess.

In a one-hundred-fifty-ninth aspect, the present disclosure includes the method of any of the one-hundred-fifty-fifth through one-hundred-fifty-eighth aspects, wherein: the first catheter further comprises one or more arrays of magnets arranged longitudinally along the first catheter body; the second catheter further comprises a second catheter body and one or more arrays of magnets arranged longitudinally along the second catheter body; and the electrode of the first catheter is aligned with the second catheter via the one or more arrays of magnets of the first catheter and the one or more arrays of magnets of the second catheter.

In a one-hundred-sixtieth aspect, the present disclosure includes the method of any of the one-hundred-fifty-fifth through one-hundred-fifty-ninth aspects, wherein the second catheter further comprises: a second biasing stent, wherein: the second biasing stent longitudinally extends along a length of the second catheter body and is configured to radially extend away from the second catheter body between a proximal point and a distal point; and the second biasing stent extends from a non-active side of the second catheter body such that the second biasing stent is configured to bias the recess against a second blood vessel wall.

In a one-hundred-sixty-first aspect, the present disclosure includes the method of any of the one-hundred-fifty-fifth through one-hundred-sixtieth aspects, wherein the recess is longitudinally positioned between the proximal point and the distal point.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1 is an illustrative depiction of the vascular anatomy of an arm in which an endovascular treatment may be delivered, according to one or more embodiments shown and described herein;
FIG. 2 depicts a two catheter system, according to one or more embodiments shown and described herein;
FIG. 3 depicts a single catheter system, according to one or more embodiments shown and described herein;
FIG. 4 depicts a cross-section of a single catheter system in an expanded state, according to one or more embodiments shown and described herein;
FIG. 5A depicts a cross-section of a single catheter system in an expanded state, according to one or more embodiments shown and described herein;
FIG. 5B depicts a axial cross-sectional view of the catheter of FIG. 5A deployed within a blood vessel, according to one or more embodiments shown and described herein;
FIG. 6 depicts a cross-section of a single catheter system in an expanded state, according to one or more embodiments shown and described herein;
FIG. 7 depicts a cross-section of a single catheter system in an expanded state, according to one or more embodiments shown and described herein;
FIG. 8A depicts an axial cross-section of the catheter of FIG. 7 having a single biasing spring, according to one or more embodiments shown and described herein;
FIG. 8B depicts an axial cross-section of a catheter having two biasing springs, according to one or more embodiments shown and described herein;
FIG. 8C depicts an axial cross-section of a catheter having three biasing springs, according to one or more embodiments shown and described herein;
FIG. 9 depicts a cross-section of a single catheter system in an expanded state, according to one or more embodiments shown and described herein;
FIG. 10A depicts a cross-section of a single catheter system in an un-expanded state, according to one or more embodiments shown and described herein;
FIG. 10B depicts a cross-section of the single catheter system of FIG. 10A in an expanded state, according to one or more embodiments shown and described herein;
FIG. 11A depicts a single catheter system in an un-expanded state, according to one or more embodiments shown and described herein;
FIG. 11B depicts the single catheter system of FIG. 11A in an expanded state, according to one or more embodiments shown and described herein;
FIG. 12A depicts a single catheter system in an un-expanded state, according to one or more embodiments shown and described herein;
FIG. 12B depicts a single catheter system in an un-expanded state, according to one or more embodiments shown and described herein;
FIG. 13A depicts a cross-section of a single catheter system in an expanded state, according to one or more embodiments shown and described herein;
FIG. 13B depicts an exit point and a re-entry point of the catheter of FIG. 13A, according to one or more embodiments shown and described herein;
FIG. 14A depicts of a vessel with the catheter system of FIGS. 13A and 13B positioned therein, according to one or more embodiments shown and described herein;
FIG. 14B depicts of the vessel of FIG. 14A with a cutting device being progressed from the first vessel into a second vessel and back into the first vessel, according to one or more embodiments shown and described herein;
FIG. 14C depicts the cutting device of FIG. 14B being further advanced, according to one or more embodiments shown and described herein;
FIG. 14D depicts an axial cross-sectional view of the catheter, and first and second vessels of FIG. 14C, according to one or more embodiments shown and described herein;
FIG. 15 schematically depicts communications between various modules of a system for endovascular treatment of a blood vessel, according to one or more embodiments shown and described herein;
FIG. 16A depicts a display illustrating an endovascular treatment of a blood vessel, according to one or more embodiments shown and described herein;
FIG. 16B depicts the display of FIG. 16A with an overlay, according to one or more embodiments shown and described herein;
FIG. 16C depicts the display of FIG. 16A with a catheter deployed to provide an endovascular treatment, according to one or more embodiments shown and described herein;
FIG. 16D depicts a fistula formation, according to one or more embodiments shown and described herein;
FIG. 17 schematically depicts communications between various modules of a system for endovascular treatment of a blood vessel, according to one or more embodiments shown and described herein;
FIG. 18 depicts a perspective view of the system of FIG. 17, according to one or more embodiments shown and described herein;
FIG. 19A depicts a display showing several views from an imaging device, according to one or more embodiments shown and described herein;
FIG. 19B depicts a display showing a 3-Dimensional Model of a vasculature of a subject, according to one or more embodiments shown and described herein;
FIG. 20 illustrates a user input device, according to one or more embodiments shown and described herein;
FIG. 21 illustrates an endovascular treatment being performed on a subject, according to one or more embodiments shown and described herein;
FIG. 22 illustrates a media bath and imaging device, according to one or more embodiments shown and described herein;
FIG. 23A depicts a perspective view of a media bath and an imaging device, according to one or more embodiments shown and described herein;
FIG. 23B illustrates a front view of the media bath and imaging device of FIG. 23A, according to one or more embodiments shown and described herein;
FIG. 24A depicts a display displaying imaging data from an imaging device during a vascular treatment, according to one or more embodiments shown and described herein;
FIG. 24B depicts a display displaying imaging data from an imaging device during a vascular treatment, according to one or more embodiments shown and described herein;
FIG. 24C depicts a display displaying imaging data from an imaging device during a vascular treatment, according to one or more embodiments shown and described herein;
FIG. 24D depicts a display displaying imaging data from an imaging device during a vascular treatment, according to one or more embodiments shown and described herein;
FIG. 25A depicts a cross-section of a single catheter system, according to one or more embodiments shown and described herein;
FIG. 25B depicts a cross-section of a single catheter system, according to one or more embodiments shown and described herein;
FIG. 26A schematically depicts a side view of a catheter including a biasing stent, according to one or more embodiments shown and described herein;
FIG. 26B schematically depicts a top view of the catheter of FIG. 26A, according to one or more embodiments shown and described herein;
FIG. 26C schematically depicts a side view of a sheath distally advanced over the catheter of FIG. 26A , according to one or more embodiments shown and described herein;
FIG. 27A schematically depicts an axial cross-section of the catheter and biasing stent of FIG. 26A, according to one or more embodiments shown and described herein;
FIG. 27B schematically depicts an axial cross-section of the catheter and biasing stent of FIG. 26A, according to one or more embodiments shown and described herein;
FIG. 28A schematically depicts a top view of a catheter including a biasing stent and a track, according to one or more embodiments shown and described herein;
FIG. 28B schematically depicts a cross-sectional view of the catheter of FIG. 28A with the biasing stent in an extended configuration, according to one or more embodiments shown and described herein;
FIG. 28C schematically depicts a cross-sectional view of the catheter of FIG. 28A with the biasing stent in an low-profile configuration, according to one or more embodiments shown and described herein;
FIG. 28D schematically depicts a cross-sectional view of the catheter of FIG. 28A with the biasing stent in an low-profile configuration, according to one or more embodiments shown and described herein;
FIG. 28E schematically depicts a cross-sectional view of the catheter of FIG. 28A with the biasing stent in an extended configuration, according to one or more embodiments shown and described herein;
FIG. 29A schematically depicts a two catheter system, according to one or more embodiments shown and described herein;
FIG. 29B schematically depicts a two catheter system, according to one or more embodiments shown and described herein;
FIG. 30 schematically depicts a side view of another catheter including a biasing stent, according to one or more embodiments shown and described herein; and
FIG. 31 schematically depicts a side view of a single catheter system including a biasing stent, according to one or more embodiments shown and described herein.

### DETAILED DESCRIPTION

Embodiments as described herein are directed the systems, methods, and catheters for endovascular treatment of a blood vessel. Endovascular treatments may include but are not limited to fistula formation, vessel occlusion, angioplasty, thrombectomy, atherectomy, crossing, drug coated balloon angioplasty, stenting (uncovered and covered), lytic therapy. Accordingly, while various embodiments are directed to fistula formation between two blood vessels, other vascular treatments are contemplated and possible. The figures generally depict various systems, methods, and devices that allow an operator to visualize and determine when a catheter has reached the correct location to provide treatment to the blood vessel (e.g., form a fistula between adjacent blood vessels). In particular, determining when a catheter has reached a desired location for treatment may be very challenging to an operator. In particular, visualization systems (e.g., ultrasound, fluoroscopy, etc.) may include the need of equipment that may be difficult to control while also controlling advancement of one or more catheter's through a vasculature of a patient. Additionally, such equipment may be expensive, leading treatment facilities to only include such visualization systems in operating rooms or the like. Accordingly, systems as described herein will make visualization easier and/or more accessible for various applications.

Additionally, using two catheters to form a fistula or otherwise provide a treatment (e.g., advancing a wire from one blood vessel to another) has been described in U.S. Patent No 9,017,323, entitled "Devices and Methods for Forming Fistula," filed November 16, 2011; U.S. Patent No 9,486,276, entitled "Devices and Methods for Fistula Formation," filed October 11, 2013; U.S. Patent Application Publication No. 2014/0276335, entitled Fistula Formation Devices and Methods Therefor," filed March 14, 2014; U.S. Patent Application Publication No. 2015/0258308, filed March 13, 2015; U.S. Patent Application No. 15/019,962, entitled Methods for Treating Hypertension, Filed February 9, 2016; U.S. Patent Application Publication No. 2017/0202616, entitled "Devices and Methods for Forming a Fistula," filed January 15, 2017; U.S. Patent Application Publication No. 2017/0202603, entitled "Systems and Methods for Increasing Blood Flow," Filed January 15, 2017; U.S. Patent Application No. 16/024,241, entitled "Systems and Methods for Adhering Vessels," filed June 29, 2018; and U.S. Patent Application No 16/024,345, entitled "Devices and Methods for Advancing a Wire," filed June 29, 2018. However, manipulating two catheters while simultaneously trying to trying to visualize the positions of both catheters may be cumbersome for a user. Accordingly, various embodiments described herein are directed to reducing the number of catheters to a single catheter while providing visualization to allow a user to readily determine a location of a treatment portion of a catheter. Moreover, flexibility of the catheters, spacing of vessels, thickness of the vessel walls, and/or the tortuous anatomy of the vessels, may make it difficult to provide sufficient coaptation and/or alignment between vessels for fistula formation. Accordingly, embodiments described herein address the one or more aforementioned limitations.

These and additional features will be discussed in greater detail below.

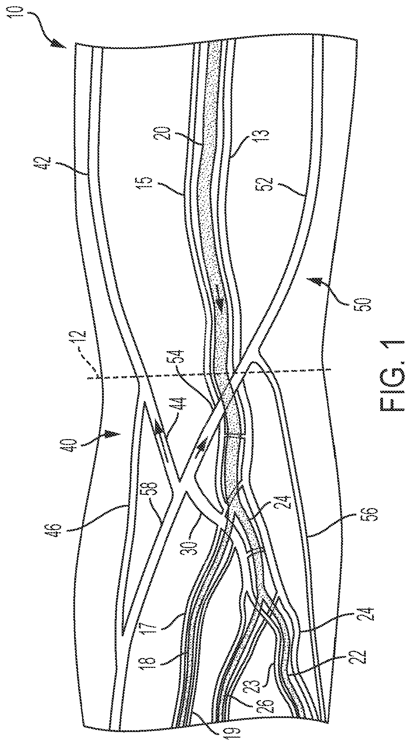

The vasculature of a potential subject (e.g., patient) may be tortuous. Additionally, each subject's vasculature may vary to provide each subject with uniquely positioned blood vessels (e.g., veins and arteries). Accordingly, in some embodiments, prior to a vascular treatment, systems as described may be used to scan a vasculature at an around a treatment portion of a subject to map the vasculature of the subject and/or determine a proper location for treatment (e.g., fistula formation). FIG. 1 illustrates a simplified depiction of the typical vascular anatomy an arm 10 around an elbow joint 12 including one or more blood vessels which may be targeted for vascular treatment. As shown, the brachial artery 20 extends superficially and distally from the upper arm and sinks deeply into the arm near the elbow joint 12, where the brachial artery 20 branches into the radial artery 22 and the ulnar artery 24. The upper portion of the ulnar artery 24 is deeply seated within the arm beneath the superficial flexor muscles (not shown), and leads down the ulnar side of the forearm to the wrist. Further down the arm, typically just below the radial tuberosity of the radius bone (not shown), the interosseous artery 26 branches off from the ulnar artery 24 and eventually feeds into the posterior and anterior interosseous arteries (not shown).

Also shown in FIG. 1 are the cephalic vein 40, including the upper cephalic vein 42, the median cephalic vein 44, and the lower cephalic vein 46, and the basilic vein 50, including the upper basilic vein 52, the medium basilic vein 54, and the lower basilic vein 56. The upper cephalic vein 42 runs along the outer border of the bicep muscle (not shown) and continues down into the forearm as lower cephalic vein 46. The median cephalic vein 44 joins the cephalic vein 40 near the elbow joint 12. The upper basilic vein 52 runs along the inner side of the bicep muscle (not shown) and continues into the forearm as the lower basilic vein 56. The lower basilic vein 56 of the lower arm is sometimes referred to as the common ulnar vein. The median basilic vein 54 (in some instances referred to as the median cubital vein) joins the upper basilic vein 52 and the low basilic vein 56. The median basilic vein 54 and the median cephalic vein 44 are formed at the branching of the median antebrachial vein 58. Near the branching of the median antebrachial vein 58 into the median basilic vein 54 and the medial cephalic vein 44, a perforating branch 30 connects these vessels with the deep veins of the arm through the antebrachial fascia (not shown).

As shown in FIG. 1, perforating branch 30 communicates with a first deep ulnar vein 23 and a second deep ulnar vein 24. These deep ulnar veins 23/24 may run substantially parallel on either side of the ulnar artery 22 between the brachial artery 14 and the interosseous artery 18, and may branch away from ulnar artery 24 distal to the interosseous artery 16. Between the brachial artery 20 and the interosseous artery 26, the deep ulnar veins 23/24 are typically located in close proximity to the ulnar artery 20, and usually less than 2 mm separate the ulnar artery 22 from the deep ulnar veins 23/24. Along the length of the deep ulnar veins 23/24, transverse branches (not shown) may occasionally connect to the deep ulnar veins 23/24. Also shown in FIG. 1 are first brachial vein 13 and second brachial vein 15. The brachial veins 13/15 generally run along the brachial artery 14, and the deep ulnar veins 23/24 feed into the brachial veins 13/15 near the elbow joint. Additionally, a pair of radial veins 17/19 may run along the radial artery 18, and may feed into one or both of the brachial veins 13/15.

In various embodiments, access to the ulnar artery and/or the ulnar vein may be achieved through an access site formed at the wrist or further up the arm into a superficial vein or artery. The catheter(s) may then be advanced through the vasculature to a treatment location. For example, it is often desirable to form a fistula between a vein and an artery proximate to a perforator (e.g., perforating branch 30) to increase blood flow from deep arteries to the superficial veins for such purposes as dialysis. Advancing a catheter from a superficial vein or artery makes accessing the site for fistula formation within the deep arterial/venous system easier.

It is noted that the vasculature within an arm is illustrated for example purposes only. It is contemplated that systems as described herein may be used to treat blood vessels anywhere within a body, human or animal (e.g., bovine, ovine, porcine, equine, etc.). For example, in some embodiments, blood vessels which are targeted and treated may include the femoral artery and femoral vein or the iliac artery and the iliac vein. In other embodiments, treatments between body conduits may not be limited to vein/artery treatments but may include treatment or fistula formation between adjacent veins, adjacent arteries or any other body conduits (e.g., bile ducts, esophagus, etc.).

### Catheters

Generally, systems described herein are directed to endovascular treatment of a blood vessel. For example, systems described herein may be useful in measuring, modifying, and/or ablating tissue to form a fistula. The systems described here typically include one or more catheters. The one or more catheters may comprise one or more treatment portions. For fistula formation procedures, the one or more treatment portions may include one or more fistula-forming elements. The catheters described may further comprise elements to aid in visualization and/or alignment of one or more catheters as described in more detail herein. Any suitable catheter or catheters may be used with the systems described herein to form the fistulas other using the methods described herein.

The catheters may have any suitable diameter for intravascular use, such as, for example, about 4 French, about 5.7 French, about 6.1 French, about 7 French, about 8.3 French, between about 4 French and about 9 French, between about 4 French and about 7 French, between about 4 French and about 6 French, or the like.

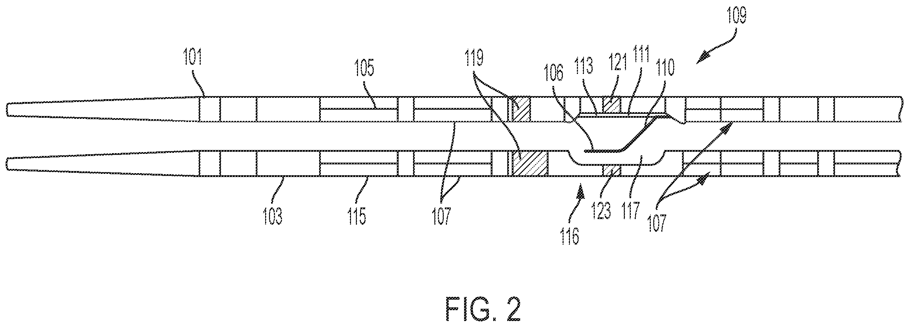

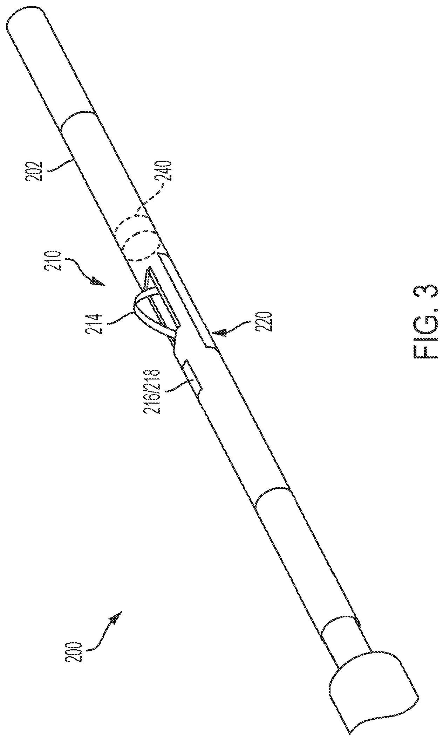

Referring now to FIGS. 2 and 3, various embodiments of one or more catheters are depicted. FIG. 2 generally illustrates one embodiment of a two catheter system while FIG. 3 illustrates an embodiment of a single catheter system. Accordingly, in embodiments incorporating a single catheter system, a second catheter is not necessary for supplying a desired treatment to a blood vessel. However, it is noted that various features of either the two catheter system or the single catheter system may be incorporated into either of the two systems. For example, an electrode such as illustrated in the single catheter system may be the same as an electrode used in the two catheter system.

As noted above, FIG. 2 generally illustrates one embodiment of a two catheter system configured to be used to form a fistula. As shown there, the system may include a first catheter 101 and a second catheter 103. The first catheter 101 may comprise a catheter body 105, one or more magnetic elements 107, and a treatment portion 109. As described herein, embodiments may be directed to fistula formation, accordingly the first catheter may include fistula-forming element 110 that may be used to form a fistula. In some variations, the fistula-forming element 110 may be advanced to project out of an opening 111 in the catheter body 105. The fistula-forming element 110 may comprise an electrode 106 configured to move between a low-profile configuration and an extended configuration in which it extends from the catheter body 105. In some variations the fistula-forming element may be spring-biased toward the extended configuration. That is, the electrode 106 may be configured to self-expand from the low-profile configuration to the extended configuration. Put yet another way, the electrode 106 may be in its natural resting state in the extended configuration. In some variations of electrodes moving between a low-profile configuration and an extended configuration, the electrode may be held in the low-profile configuration during placement of the catheter. For example, in some variations the electrode may be held in the low-profile configuration by the catheter body. The electrode may be released from the low-profile configuration when the electrode has been delivered to the location for fistula formation. For example, in some variations, the electrode may be released by moving the electrode in a proximal direction relative to the housing using a proximal control, as described in U.S. Patent No 9,017,323, entitled "Devices and Methods for Forming Fistula," filed November 16, 2011. In other variations, the electrode may be held in a low-profile configuration by an external radially inward force on the electrode from a vessel wall during delivery, as described in U.S. Patent Application Publication No. 2017/0202616, entitled "Devices and Methods for Forming a Fistula," filed January 15, 2017.

In some variations, the first catheter 101 may comprise a housing 113, which may help protect other components of the first catheter 101 during fistula formation. For example, when the fistula-forming element 110 comprises an electrode 106 configured to ablate tissue, the housing 113 may comprise one or more insulating materials which may shield or otherwise protect one or more components of the first catheter 101 from heat that may be generated by the electrode 106 during use.

As shown in FIG. 2, the second catheter 103 may also comprise a catheter body 115 and one or more magnetic elements 107. In variations where the first catheter 101 comprises a fistula-forming element 110 configured to project out the catheter body 105 of the first catheter 101, such as the variation depicted in FIG. 2, the catheter body 115 of the second catheter 103 may comprise a treatment portion 116 that includes a recess 117 therein, which may be configured to receive the fistula-forming element 110 as it passes through tissue. While shown in FIG. 2 as having a recess 117, it should also be appreciated that in some variations the treatment portion 116 of the second catheter 103 may not include a recess 117. In some variations, the treatment portion 116 of the second catheter 103 may include a fistula-forming element (not shown) in addition to or instead of the fistula-forming element 110 of the first catheter 101. Thus, in some variations, a fistula may be formed by one or more electrodes of one catheter, while in other variations, two catheters each comprising an electrode may simultaneously cut tissue from opposing sides to form a fistula.

In some variations, each of the one or more catheters may include one or more location indicators 119 configured to allow a control unit of the system to determine a location of the treatment portion of the catheter as it is advanced through the vascular of a subject (e.g., patient). For example, in one embodiment, each of the first catheter 101 and the second catheter 103 may include echogenic markers. The echogenic markers may be positioned proximate to the treatment portion of the catheter and may be visible to an imaging device such of an ultrasound imaging device. The echogenic markers may form particular patterns (e.g., a series of different sized echogenic rings with a specific spacing similar to a bar code) which may allow recognition of a particular catheter. Such pattern or ring may include marker bands made from, for example, platinum, iridium, or combinations thereof applied to the catheter proximate to the treatment portion of the catheter. In some embodiments, and as will be described in greater detail below, based on the echogenic markers a control unit, using a imaging device to capture image data of the one or more catheters, may be configured to determine a location of the treatment portion of the one or more catheters. In a two-catheter system such as illustrated in FIG. 2, each of the first and second catheters 101/103 may include echogenic markers which may be identical to or different from one another. Where the echogenic markers on each of the first and second catheters 101/103 vary from one another, a control unit may be able to determine which catheter is which. In some embodiments, echogenic markers may be used to indicate a rotational orientation of the one or more catheters. For example, a pattern of the echogenic marker when viewed under ultrasound may indicate in which direction the treatment portion of the particular catheter is facing.

In some embodiments, in addition to or in lieu of echogenic markers, the catheters 101/103 may include one or more location sensors 121/123, configured to output a signal indicative of a location of the catheter 101/103 (e.g., the treatment portion of the catheter). For example, the location sensor 121/123 may include an active electromagnetic sensor, a passive electromagnetic sensor, a permanent magnet, an RFID device, and or/ an ultrasound transceiver. The location sensor 121 may be coupled to or positioned within the housing of the catheter at a position proximate to the treatment portion of the catheter. For example, a location sensor may be positioned longitudinally within the treatment portion 109/116 of the catheter 101/103. In some embodiments, a location sensor may be positioned proximal to and/or distal from the treatment portion 109/116 of the catheter 101/103. As will be described in greater detail below, a control unit may, based on the signal received from the location sensor 121, determine a location of the treatment portion 109/116 of the catheter 101/103 and follow a location of the catheter 101/103 in real time with an imaging device. It is noted that while the one or more location sensors 121/123 are illustrated as being in close proximity to the treatment portion 109/116, the one or more location sensors may be positioned anywhere along the housing of the catheter 101/103.

It is noted that echogenic markers may be advantageous over electrically powered location sensors due to a need for tethering the location sensor to a power source. Accordingly, some echogenic markers may not require connection to a power source.

FIG. 3 illustrates an embodiment of a system including a single catheter 200. Catheter 200 may be substantially similar to catheter 101 described above. Similar to the first catheter 101 described in regards to FIG. 2 above, the catheter 200 may include a housing 202 and coupled to the housing 202 may be a treatment portion 210. In embodiments wherein endovascular treatment is directed to fistula formation, the treatment portion 210 may include an electrode 214 or other cutting device for forming a fistula. While the illustrated embodiment depicts an electrode 214 having an arc, the electrode 214 may be substantially similar to that described above and to the electrode described above in regards to the two catheter system. Additionally, and as noted above an electrode of the single catheter system may have features such as described in U.S. Patent No 9,017,323, entitled "Devices and Methods for Forming Fistula," filed November 16, 2011, and U.S. Patent Application Publication No. 2017/0202616, entitled "Devices and Methods for Forming a Fistula," filed January 15, 2017.

It is also contemplated that the catheter 200 may include one or more echogenic markers 216 and/or one or more location sensors 218, as described above in regard to FIG. 1. The one or more echogenic markers 216 and the one or more location sensors 218 may be positioned anywhere along the housing 202 of the catheter 200. For example, the one or more echogenic markers 216 may be positioned proximal to distal to, and/or within the treatment portion 210. Similarly, the one or more location sensors 218 may be positioned proximal to distal to, and/or within the treatment portion 210.

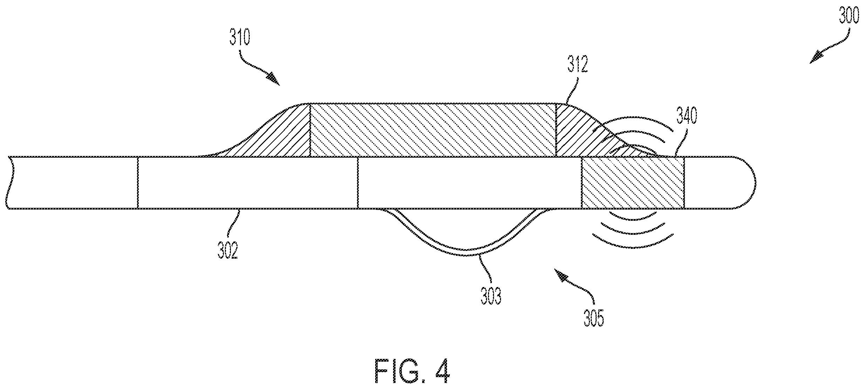

In addition, the catheter 200 may include one or more biasing mechanisms 220. A biasing mechanism 220 may be configured contact a wall of a blood vessel to bias the treatment portion 210 of the catheter 200 into contact with the wall (e.g., at a target treatment location) of the blood vessel. For example, the biasing mechanism 220 may be configured to expand to contact a first radial portion of a host blood vessel to bias the treatment portion 210 toward a second radial portion of the host blood vessel opposite the first radial portion. That is, the biasing mechanism 220 may expand to cause the catheter 200 to move laterally within the host blood vessel to cause the treatment portion 210 (e.g., cutting device, electrode, etc.) to contact a wall of the blood vessel. In some embodiments, the force of the biasing mechanism 220 may alter a shape of the blood vessel to extend the blood vessel in a direction opposite the movement of the biasing mechanism. Accordingly, the one or more biasing mechanisms 220 may be any mechanism configured to move the catheter transversely within a blood vessel to cause the treatment portion of the catheter to contact a treatment location within the blood vessel. Such biasing mechanism 220 may be positioned on opposite sides of the housing 202 from the treatment portion 210 of the catheter 200. Biasing mechanisms 220 may include, but are not limited to, balloons, cages, expandable wires, retracting mechanisms, etc. Various embodiments of biasing mechanisms will be discussed in greater detail with reference to FIGS. 4-13B.

In some embodiments, the catheter 200 may further include an intravascular imaging device 240 positioned within the housing 202 adjacent to the treatment portion 210. For example, the intravascular imaging device 240 may include intravascular ultrasound ("IVUS"), optical coherence tomography ("OCT"), intracardiac ultrasound ("ICE"), or the like. The intravascular imaging device 240 may be configured to provide a cross-sectional image at the position of the intravascular imaging device 240. As will be described in greater detail herein, the intravascular imaging device 240 may be used to determine a position of the catheter 200 within a blood vessel and/or the rotational alignment of the catheter 200, for example, the treatment portion 210 of the catheter 200. The intravascular imaging device 200 may be coupled to the housing 202 at a position distal to the treatment portion 210, proximal to the treatment portion 210, or longitudinally aligned with and/or within the treatment portion 210 of the catheter 200. In some embodiments, the one or more location sensors 220 may be incorporated in or positioned proximate to the intravascular imaging device 240. Various embodiments of the intravascular imaging device will be described in greater detail with reference to FIGS. 4-13B and 25A-25B. It is noted that in some embodiments, there may not be an intravascular imaging device and instead an external imaging device (e.g., an external ultrasound probe) may be used.

Referring now to FIG. 4, a cross section of an embodiment of a catheter 300 having a treatment portion 305 and a biasing mechanism 310 is depicted. In the depicted embodiment, the biasing mechanism 310 may be balloon that may be controllably expanded (e.g., controllably filled with saline). In the illustrated embodiment, the balloon is an asymmetrical balloon 312 that spans longitudinally across the treatment portion 302 of the catheter 300 on the opposite side of the housing 302. For example, in the present embodiment, the treatment portion 302 includes an electrode 303 and the asymmetrical balloon 312 is positioned directly opposite from the electrode 303. In the illustrated embodiment, the catheter 300 includes an intravascular imaging device 340 as described above, positioned distal (i.e., closer to the tip of the catheter) treatment portion. As will be described in greater detail herein, upon expansion of the biasing mechanism 310 the treatment portion 305 may be biased into contact with a treatment location of the blood vessel.

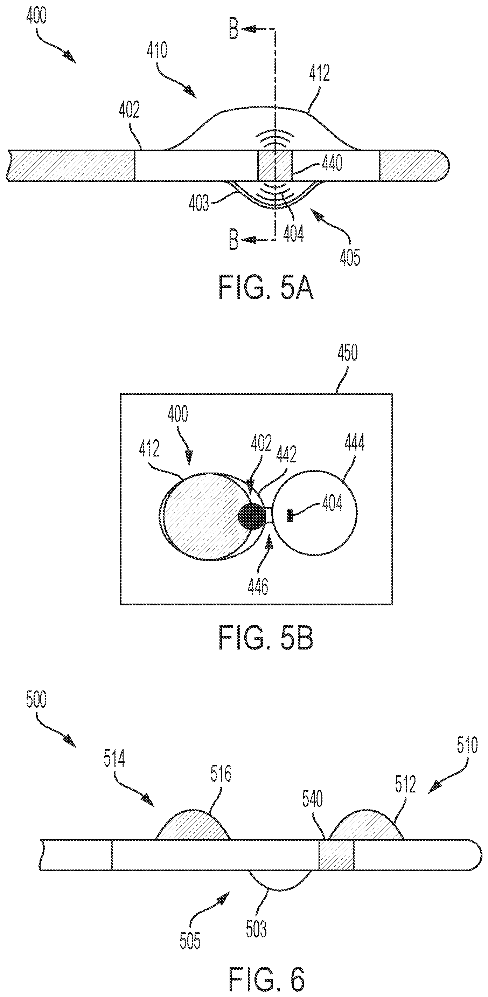

FIGS. 5A and 5B illustrate a similar catheter 400 to that illustrated in FIG. 4. In the illustrated embodiment, the catheter 400 includes a treatment portion 405 and a biasing mechanism 410. As in FIG. 4, the biasing mechanism 410 may be an asymmetrical balloon 412 that spans across the treatment portion of the catheter on an opposite side of the housing 402. For example, in the present embodiment, the treatment portion 405 includes an electrode 403 having an arc that extends from the housing 402 and the asymmetrical balloon 412 is positioned directly opposite from the electrode 403. In the illustrated embodiment, the catheter 300 includes an intravascular imaging device 440 that is positioned within the housing 402 at a mid-point or apex 404 of the arc of the electrode 403. As noted above, the intravascular imaging device 440 may output image data depicting a cross-section of the catheter at the intravascular imaging device. For example, the cross-section may be aligned with the apex 404 of the electrode 403 as indicated by line B-B. FIG. 5B depicts an example image output on a display 450 of the intravascular imaging device 440 wherein the cross-section within a blood vessel 442 is taken at the apex 404 of the electrode 403. By taking the cross-section at the apex 404 of the electrode 404 it may be possible to determine a rotational alignment of the catheter based on the position of the apex 404 of the electrode as determined by the image data of the intravascular imaging device 440. Furthermore, because the intravascular imaging device 440 can continue transmitting during fistula formation with the electrode 403, it may be determined that the electrode has passed into a second blood vessel 444 from the first vessel 442 and that a fistula 446 has been created.

FIG. 6 illustrates another embodiment of a catheter 500 including a first biasing mechanism 510 and a second biasing mechanism 514. The first and second biasing mechanisms may be places proximal to and distal from the treatment portion 405. In such embodiment, the first and second biasing mechanisms may include balloons 512/516 (e.g., asymmetrical balloons). However, it is also contemplated that the first and second biasing mechanisms may be any biasing mechanism discussed herein. It is noted that by placing the balloons distal to and proximal to the treatment portion, it may be easier to isolate the electrode from fluid (e.g., saline) used to fill the balloons 512/516.

Furthermore, catheter 500 may include an intravascular imaging device 540. While the intravascular imaging device 540 is illustrated as being position distal to the treatment portion 505 (e.g., electrode 503), the intravascular imaging device 540 may be positioned anywhere along the catheter 500.

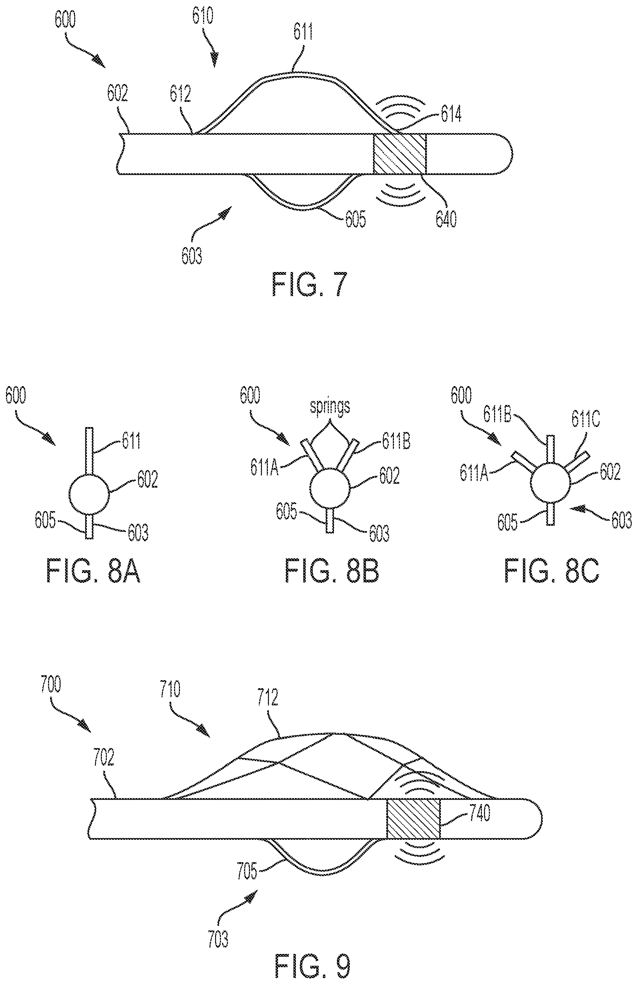

FIG. 7 illustrates another embodiment of a catheter 600 including a treatment portion 603, an intravascular imaging device 640, and a biasing mechanism 610. In such embodiment, the biasing mechanism includes one or more expandable wires such as a biasing spring 611 (e.g., nitinol ribbon, wire, etc.) that is configured to bias the treatment portion 603 into contact with a blood vessel. The biasing spring 610 may be coupled to the housing 602 of the catheter at a first end 612 and a second end 614 proximal and distal to the treatment portion so as to span the treatment portion 603 on an opposite side of the housing 602 from the treatment portion 603. In some embodiments, the biasing mechanism 610 may include a single biasing spring 611 (FIG. 8A), two biasing springs 611A, 611B (FIG. 8B), or three biasing springs 611A, 611B, 611C (FIG. 8C). However, a greater number of biasing springs are contemplated and possible. The biasing springs may extend radially from the housing as illustrated in FIGS. 8A-8C.

As noted above, catheter 600 may include an intravascular imaging device 640. While the intravascular imaging device 640 is illustrated as being position distal to the treatment portion 603 (e.g., electrode 605), the intravascular imaging device 640 may be positioned anywhere along the catheter 600.

FIG. 9 illustrates another embodiment of a catheter 700 including a treatment portion 703, an intravascular imaging device 740, and a biasing mechanism 710. In such embodiment, the biasing mechanism 700 includes a self-expanding cage 712 (e.g., nitinol cage) coupled to the housing of the catheter 700 opposite the treatment portion 703 (e.g., electrode 705). The self-expanding cage 712 is configured to bias the treatment portion 703 into contact with a blood vessel. The self-expanding cage 712 may be coupled to the housing 702 of the catheter 700 opposite the treatment portion 703 so as to span the treatment portion 703.

As noted above, catheter 700 may include an intravascular imaging device 740. While the intravascular imaging device 740 is illustrated as being position distal to the treatment portion 703 (e.g., electrode 705), the intravascular imaging device 740 may be positioned anywhere along the catheter 700.

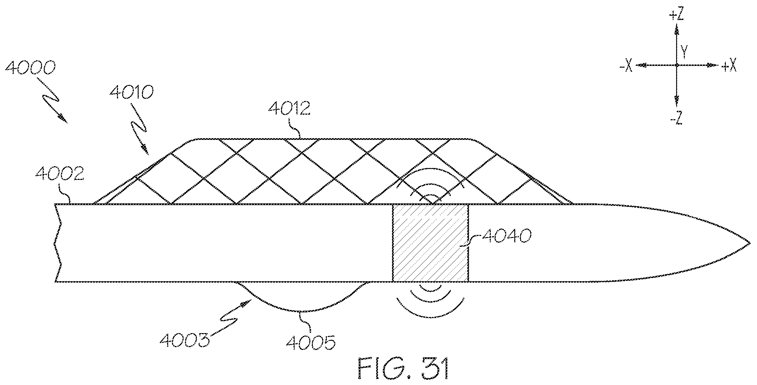

FIG. 31 illustrates another embodiment of a catheter 4000 including a treatment portion 4003, an intravascular imaging device 4040, and a biasing mechanism 4010. In such embodiment, the biasing mechanism 4010 includes a self-expanding biasing stent 4012 (described in greater detail below) coupled to the housing of the catheter 4000 opposite the treatment portion 4003 (e.g., electrode 4005). The biasing stent 4012 is configured to bias the treatment portion 4003 into contact with a blood vessel. The biasing stent 4012 may be coupled to the housing 4002 of the catheter 4000 opposite the treatment portion 4003 so as to span the treatment portion 4003.

As noted above, catheter 4000 may include an intravascular imaging device 4040. While the intravascular imaging device 4040 is illustrated as being position distal to the treatment portion 4003 (e.g., electrode 4005), the intravascular imaging device 4040 may be positioned anywhere along the catheter 4000.

It is noted that each of the embodiments of the biasing mechanism may be positioned in an un-expanded position via a retractable sheath (not shown). In other embodiments, the biasing mechanism may be actuated by an operator.

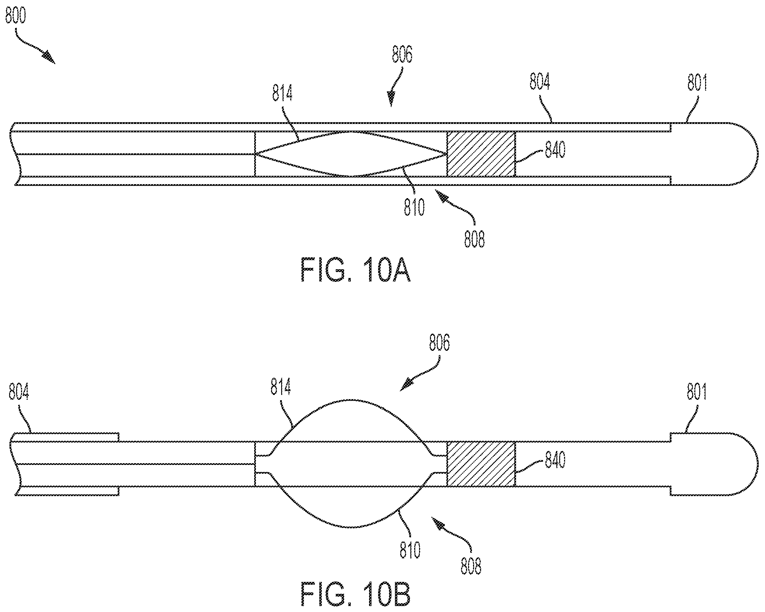

FIGS. 10 and 10B illustrate an embodiment of a catheter 800 including a housing 801, a retractable sheath 804, and a biasing mechanism 806, and a treatment portion 808. The treatment portion 808 may include a spring biased electrode 810, such as described above. The retractable sheath 804 may hold the spring biased electrode 810 in a retracted position. The biasing mechanism 806 may be coupled to the housing 801 at a position proximate to the treatment portion 808. The biasing mechanism 806 may include one or more expandable wires moveable between a collapsed position and an expanded position, wherein at least a portion of the one or more expandable wires are spaced from an outer wall of the housing of the catheter 801. For example, in this embodiment, the biasing mechanism 806 is illustrated as a biasing spring 814 that is retractable to a position within the housing 801 and expandable to a position outside of the housing 801 to bias the treatment portion 808 of the catheter 800. The retractable sheath 804 is configured to hold the biasing mechanism 806 in a retracted position until deployment is desired. Accordingly, retracting of the sheath 804 may deploy both the spring biased electrode 810 and the biasing spring 814 simultaneously.

The catheter 800 may include an intravascular imaging device 840. While the intravascular imaging device 840 is illustrated as being position distal to the treatment portion 808 (e.g., electrode 810), the intravascular imaging device 840 may be positioned anywhere along the catheter 800.

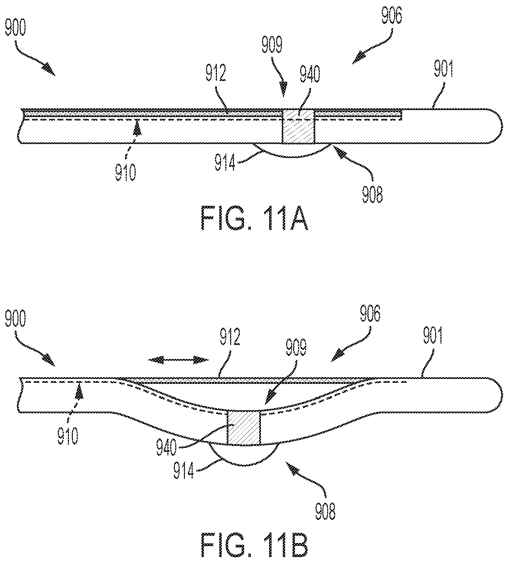

FIGS. 11A and 11B illustrate an embodiment of a catheter 900 including a housing 901, a treatment portion 908, and a biasing mechanism 906. In such embodiment, the housing 901 defines one or more lumens 910 extending therethrough. A deflection wire 912 may extend through the housing 901 to couple to the housing 901 at a position distal the treatment portion 908. The housing 901 may further define an opening 909, such that the housing 901 may deflected around the deflection wire 912. Such deflection may allow the treatment portion 908 to be biased toward a treatment location within a blood vessel. In operation, the catheter 900 may be advanced to a treatment location, the housing 901 may then be pushed distally, while an operator restricts motion of the deflection wire (e.g., by holding a proximal end of the deflection wire 912). As illustrated in FIG. 11B, such motion causes the housing 901 including the treatment portion 908 (e.g., electrode 914) to deflect away from the deflection wire 912. In other embodiments, the operator may instead restrict motion of the housing 901 and pull on the deflection wire 912 to cause the housing 901 to deflect away from the deflection wire 912. In embodiments, the deflection wire 912 may have a greater stiffness than the housing 901.

The catheter 900 may include an intravascular imaging device 940. While the intravascular imaging device 940 is illustrated aligned within the treatment portion 908 and aligned with an apex of the electrode 914, the intravascular imaging device 940 may be positioned anywhere along the catheter 900.

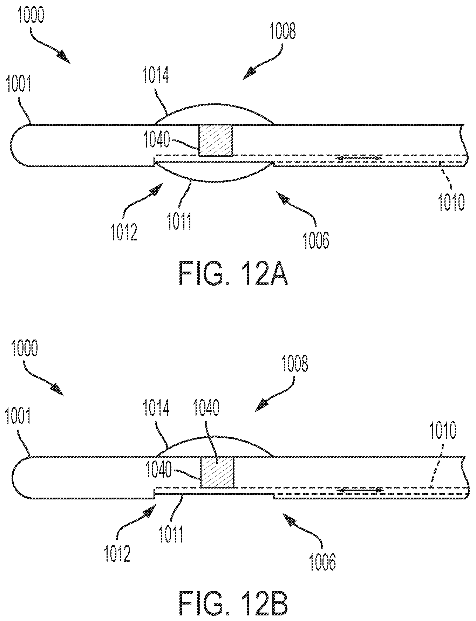

FIGS. 12A and 12B illustrated an embodiment similar to that illustrated in FIGS. 11A and 11B. In particular, FIGS. 12A and 12B depict a catheter 1000 including a housing 1001, a treatment portion 1008, and a biasing mechanism 1006. In such embodiment, the housing 1001 defines one or more lumens 1010 extending therethrough. A deflection wire 1011 may extend through the housing 1001 to couple to the housing 1001 at a position distal the treatment portion 1008. The housing 1001 may further define an opening 1012, such that the housing 1001 through which the deflection wire 1011 moves in and out of to provide a biasing force to bias the treatment portion 1008 into contact with a treatment location of a blood vessel. Accordingly, the deflection wire 1011 has a retracted configuration (as illustrated in FIG. 12A) wherein the deflection wire 1001 is disposed within the housing and an extended configuration wherein the deflection wire 1011 is positioned outside of the housing (as illustrated in FIG. 12B).

In some embodiments, the deflection wire 1011 may comprise a shape memory material wherein in its natural state the deflection wire 1011 deflects out of the housing 1001. For example, the deflection wire 1011 may be a leaf spring. In such embodiments, a user may hold the deflection wire (e.g., with a sheath) in the retracted configuration and when the catheter has reach the desired position, release the deflection wire 1001. In other embodiments, a user may instead manually advance or retract the deflection wire 1011 (e.g., may pulling/pushing a proximal end of the deflection wire 1011), to cause the deflection wire 1011 to retract or extend.

The catheter 1000 may include an intravascular imaging device 1040. While the intravascular imaging device 1040 is illustrated aligned within the treatment portion 1008 and aligned with an apex of the electrode 1014, the intravascular imaging device 1040 may be positioned anywhere along the catheter 1000.

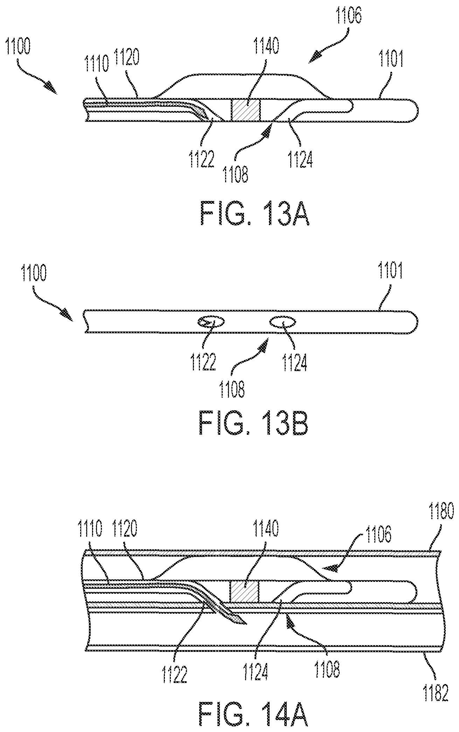

FIGS. 13A-13F illustrate an alternative embodiment of a catheter 1100. In such embodiment, the catheter 1100 includes a housing 1101, a treatment portion 1108, and a biasing mechanism 1106. The biasing mechanism 1106 may include any biasing mechanism such as, for example, an asymmetrical balloon, a cage, a wire(s), or any other deflection mechanism discussed herein. The catheter 1100 may further include an intravascular imaging device 1140.

Referring to FIGS. 13A and 13B, the housing 1101 may define a lumen 1120 extending therethrough. The lumen 1120 may define an exit point 1122 and a re-entry point within the treatment portion 1108 of a catheter 1100. A cutting device 1110 (e.g., a nitinol needle) may be advanced through the lumen 1120 from a proximal position to a distal position, wherein the cutting device 1110 is extended through the exit point 1122 and through the re-entry point 1124. The cutting device 1110 may be produced from a shape memory material configured to bend as it exits the exit point 1122 to align itself for re-entry through the re-entry point 1124. The cutting device 1110 may be an electrode or other cutting device.

The biasing mechanism 1106 may be coupled to the housing 1101 opposite the exit and re-entry points 1122/1124. Accordingly, the biasing mechanism 1106 may bias the exit and re-entry point 1122/1124 into contact with a treatment location within a blood vessel.

In the present embodiment, the intravascular imaging device 1140 may be positioned longitudinally between the exit point and the re-entry point of the housing within the treatment portion 1108 of the catheter 1100. In other embodiments, the intravascular imaging device may be positioned longitudinally proximal or distal from the treatment portion 1108.

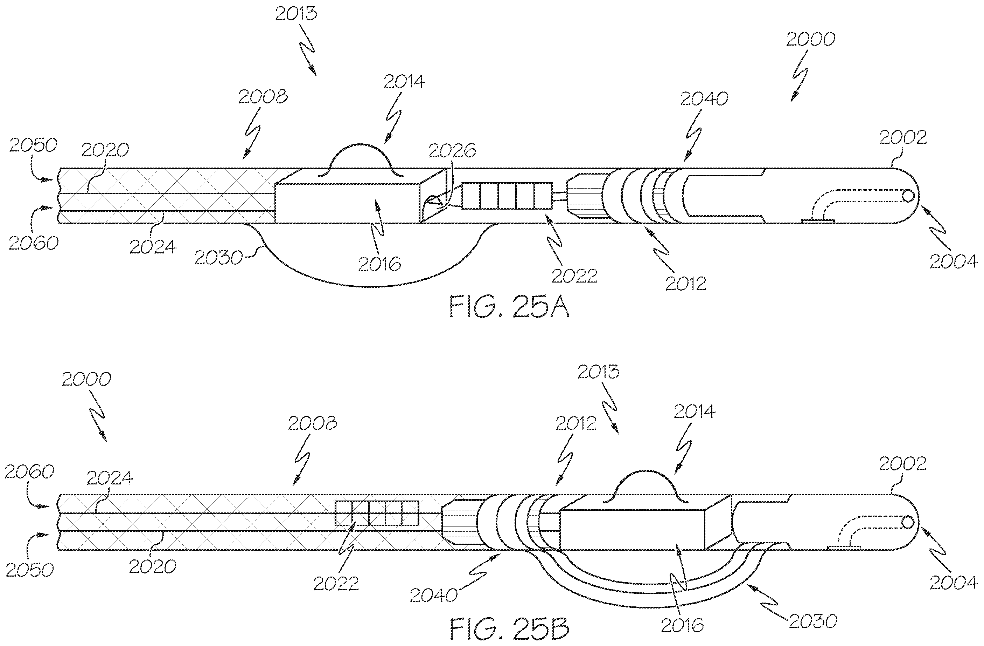

Referring now to FIGS. 25A and 25B, yet another alternative embodiment of a catheter 2000 is schematically depicted. The catheter 2000 is substantially similar to the above-described embodiments unless otherwise noted or apparent. In particular the catheter 2000 includes a housing 2008, a treatment portion 2013, and a biasing mechanism 2030. The biasing mechanism 2030 may include any biasing mechanism such as, for example, an asymmetrical balloon, a cage, a wire(s), or any other deflection mechanism discussed herein. The catheter 2000 may further include an intravascular imaging device 2040 (e.g., such as a solid state intravascular ultrasound device, a mechanical intravascular ultrasound device, or the like, such as described above). In embodiments, the intravascular imaging device 2040 includes an array of solid-state transducers 2012, which may be positioned circumferentially (e.g., 360 degrees) around a support structure. Similar to above embodiments, the treatment portion 2013 may include a fistula forming element 2014, such as an electrode or other cutting element. For example, the fistula forming element 2014 may be a leaf spring electrode, that is configured to arc, such as under a natural spring bias, to extend outside the housing 2008. Extending from the electrode may be an electrode wire 2020, which may extend within the housing 2008 and may be coupled to an energy source.

In the depicted embodiment, the housing 2008 is substantially similar to the above-described embodiments. However, in the depicted embodiment, the housing 2008 includes a rapid exchange tip 2002 at the distal end 2004, for passing over a guidewire. The housing 2008 may be formed of from any combination of polymers, such as, silicone rubber, nylon, polyurethane, polyethylene terephthalate (PET), latex, thermoplastic elastomers, or the like. In some embodiments, the housing 2008 may be a braided polymer. In some embodiments, the housing 2008 may define a dual lumen. For example, a first lumen 2050 within the housing 2008 may house the electrode wire 2020 of the catheter 2000, while a second lumen 2060 of the housing 2008 may house various other components such as components with respect to the intravascular imaging device 2040, the array of solid-state transducers 2012, wires thereof, or the like. In some embodiments, the first lumen 2050 housing the electrode wire 2020 may include an insulation sleeve, to electrically and/or thermally insulate the electrode wire 2020. In some embodiments, the first lumen 2050 may be positioned radially within the second lumen 2060.

As noted above, the treatment portion 2013 of the catheter 2000 may include an electrode. The electrode may have an electrode housing 2016 positioned within the catheter 2000. The electrode housing 2016 may be positioned within the catheter housing 2008 and generally aligned with the treatment portion 2013. The electrode may radially extend from the catheter housing 2008 at the electrode housing 2016. The electrode may be housed within the electrode housing 2016 when the electrode is in the low-profile configuration. The electrode housing 2016 may define a channel 2026 therethrough for passage of wires for the intravascular imaging device 2040, the array of solid-state transducers 2012, and/or the like.

The array of solid-state transducers 2012 may be piezoelectric ultrasound transducers. In some embodiments, the array of solid-state transducers 2012 may extend around the entire circumference of the catheter housing 2008. In some embodiments, the array of solid-state transducers 2012 may extend around a portion of the circumference of the catheter housing 2008. The array of solid-state transducers 2012 may be fixed in or on the catheter 2000 by any suitable method. The array of solid-state transducers 2012 may be embedded within the catheter housing 2008. In other embodiments, the array of solid-state transducers 2012 may be fixed to the outer surface of the catheter housing 2008. In other embodiments, the array of solid-state transducers 2012 may be friction-fit within the catheter housing 2008. The array of solid-state transducers 2012 may be positioned proximal or distal the fistula forming element 2014, as discussed in more detail below.

The array of solid-state transducers 2012 may be coupled to one or more flexible circuit elements housed within the catheter housing 2008, such as, but not limited to, one or more multiplexing application-specific integrated circuits 2022. The one or more multiplexing application-specific integrated circuits 2022 may be positioned near the array of solid-state transducers 2012 and within the catheter housing 2008. The one or more multiplexing application-specific integrated circuits 2022 enables front-end processing of data generated by the array of solid-state transducers 2012. Positioning the one or more multiplexing application-specific integrated circuits 2022 near the array of solid-state transducers 2012 and within the catheter housing 2008 may further reduce the cables and/or wires passing back through the catheter housing 2008 to a back-end processor and/or display device. Particularly, an IVUS wire 2024 may extend from the array of solid-state transducers 2012 and/or the one or more multiplexing application-specific integrated circuits 2022 proximally through the catheter housing 2008.

In embodiments, the array of solid-state transducers 2012 may be positioned between the electrode housing 2016 and the distal end 2004 of the catheter 2000. That is, the array of solid-state transducers 2012 may be positioned between the electrode housing 2016 and the rapid exchange tip 2002. In such embodiments, the one or more multiplexing application-specific integrated circuits 2022 may be positioned between the array of solid-state transducers 2012 and the electrode housing 2016. The channel 2026 of the electrode housing 2016 may allow for the IVUS wire 2024 to extend from the multiplexing application-specific integrated circuits 2022, through the electrode housing 2016, and proximally through the catheter housing 2008.

In some embodiments the electrode housing 2016 may be positioned between the array of solid-state transducers 2012 and the distal end 2004 of the catheter 2000. That is, the electrode housing 2016 may be positioned between the array of solid-state transducers 2012 and the rapid exchange tip 2002. In such embodiments, the array of solid-state transducers 2012 may be positioned between the one or more multiplexing application-specific integrated circuits 2022 and the electrode housing 2016.

FIGS. 14A-14D illustrate a potential method for forming a fistula using catheter 1100 described in FIGS. 13A and 13B. In such embodiments, the catheter 1100 may be advanced within a first blood vessel 1180 (e.g., an artery or vein) to a position wherein the first blood vessel 1180 is positioned proximate to a second target vessel 1182 (e.g., a vein or artery). It is noted that while the catheter may be advanced through either the vein or artery, in some embodiments, it is beneficial to advance the catheter through an artery and form a fistula from the arterial side, as opposed to from the vein side, as going from a higher pressure artery to a lower pressure vein may provide for improved fistula formation. However, in other embodiments, the catheter may instead be advanced through a vein to a desired location. In yet further embodiments, a first catheter may be advanced through an artery and a second catheter may be advanced through a vein, such as in a two catheter system as discussed above.

Referring now to FIG. 14A, the catheter 1100 is advanced to a treatment location within the first blood vessel 1180. Based on imaging from, for example, the intravascular imaging device 1140, an operator may determine that the catheter 1100 is in the correct location for treatment (e.g., fistula formation between an artery and closely situated vein). Once in position, the biasing mechanism 1106 can be actuated to bias the treatment portion 1108 of the catheter into contact a treatment location (e.g., a desired portion of the vessel wall) of the first blood vessel 1180. In such cases, imaging using the intravascular imaging device 1140 may allow the operator to determine the treatment portion 1108 is rotationally aligned with the desired treatment location of the blood vessel. It is noted that in some embodiments, an intravascular imaging device 1140 may include a sensor (e.g., a location sensor) that outputs an indication of the rotational alignment of the intravascular imaging device 1140 which may correlate to or otherwise provide an indication of the rotational alignment of the treatment portion 1108 of the catheter 1100.

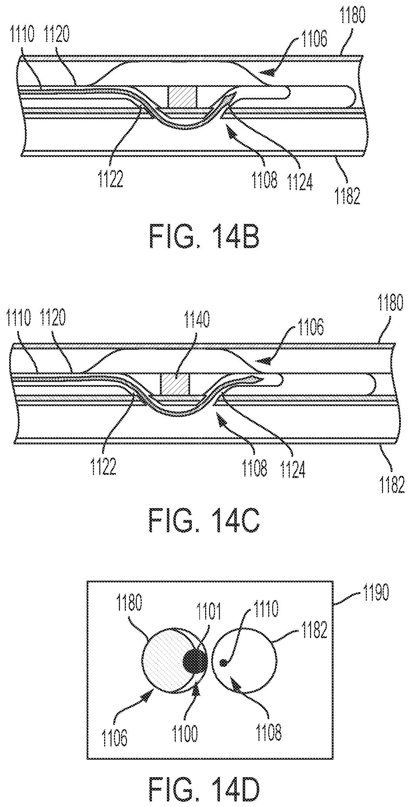

Once in position, the cutting device 1110 may be advanced along the lumen through the exit point 1122 and into the second blood vessel 1182. In FIG. 14B, the cutting device 1110 may continue to be advanced such that the cutting device 1110 crosses back out of the second blood vessel 1182 and into the re-entry point 1124 of the catheter 1100. Referring to FIG. 14C, continuing to advance the cutting device 1110 may more closely sandwich the walls of the first and second blood vessels 1180/1182 between the cutting device 1110 and the housing 1101 of the catheter. FIG. 14D illustrate image data from the intravascular imaging device 1140, which may be displayed on a display 1190 communicatively coupled to the intravascular imaging device 1140. The image data may be an axial cross-section of the catheter 1100 and blood vessels 1180/1182 at the position of the intravascular imaging device 1140. Such cross-section illustrates the biasing mechanism biasing 1106 the treatment portion 1108 of the catheter 1100 toward contact with the treatment location of the blood vessel 1180. Additionally, the cutting device 1110 is illustrated as being positioned within the second blood vessel 1182. Accordingly, it can be confirmed that the cutting device has entered the second blood vessel. When in position, the cutting device 1110 may be activated (e.g., through RF energy) to create a fistula between the first blood vessel 1180 and the second blood vessel 1182. Using Doppler, fluoroscopy, or other imaging functions, it may be confirmed that a fistula has been created by monitoring blood flow between the two vessels through the fistula.

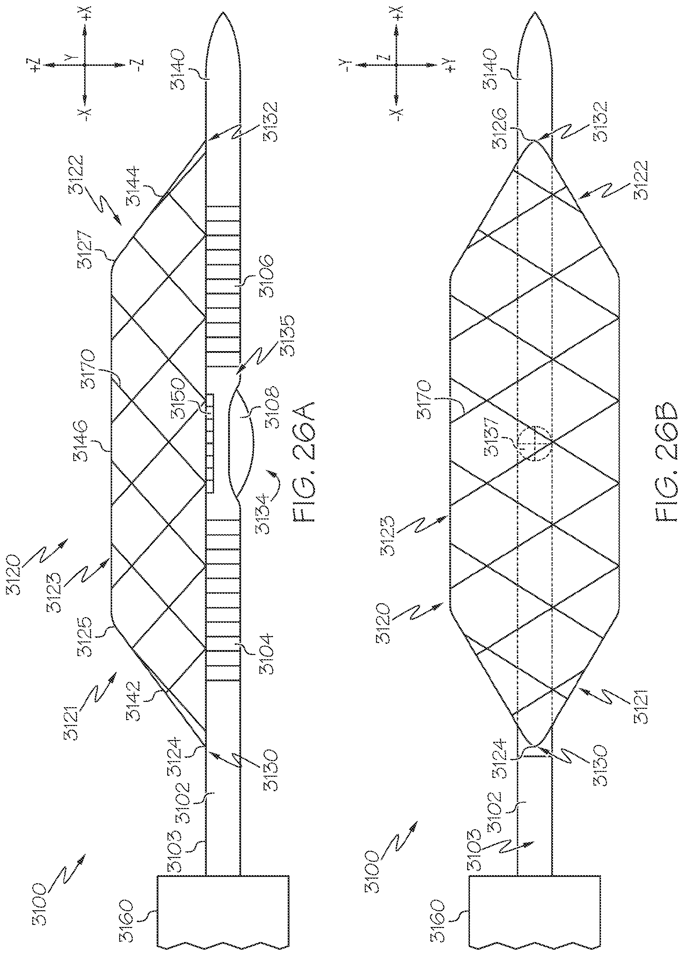

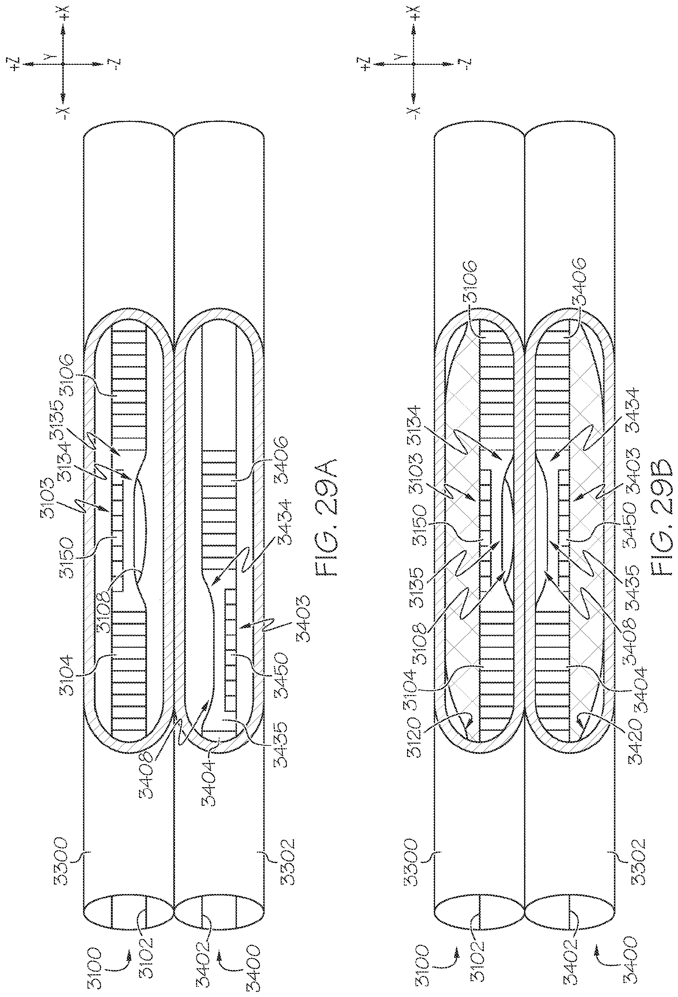

Referring now to FIG. 26A, another catheter 3100 (e.g., a first catheter) of a system for forming a fistula is depicted. As will be described in greater detail herein, the system may further include a second catheter 3400 (FIG. 29A). While the structure of the first catheter 3100 will be discussed in detail, it should be appreciated that the structure of the second catheter 3400 (FIG. 29A) may mirror the first catheter 3100 except where noted. The catheter 3100 generally includes a catheter body 3102, which may include a distal tip 3140 that is particularly configured to aid in advancement of the catheter 3100 through a blood vessel. For example, the distal tip 3140 may be pointed and/or atraumatic for advancement through a blood vessel. The catheter body 3102 may have any desirable cross-sectional shape and any suitable diameter for intravascular use. The catheter 3100 may further include one or more lumens or other passageways extending at least partially along or through the catheter body 3102. For instance, the one or more lumens may extend at least partially longitudinally through the catheter body 3102 in the direction of the x-axis of the coordinate axes of FIG. 26A.

The catheter 3100 may further include a treatment portion 3135 arranged along the catheter body 3102. The treatment portion 3135, as described herein, refers to a portion of the catheter 3100 positioned along the catheter body 3102 that is configured to modify a blood vessel (e.g., cut, ablate, etc.). In particular, in embodiments of the present disclosure, the catheter 3100 may include a treatment portion 3135 that is configured to form one or more fistulas between a first blood vessel 3300 (FIG. 29A) and a second blood vessel 3302 (FIG. 29A). In embodiments, the treatment portion 3135 may be positioned along the catheter body 3102 at a position proximal to (e.g. in the -x direction of the coordinate axes of FIG. 26A) the distal tip 3140 and define an active side 3134. The treatment portion 3135 may comprise one or more openings in the active side 3134 of the treatment portion 3135 that allow for passage of one or more instruments into and/or out of the catheter body 3102 for modifying the vessel. For example, the active side 3134 of the treatment portion 3135 is the portion of the treatment portion 3135 that abuts or faces the area of the vessel where a modification is to be made. For instance, an electrode 3108 may protrude from the active side 3134 of the treatment portion 3135 and extend radially away from a longitudinal centerline of the catheter 3100 to contact a wall of the blood vessel 3300 (see e.g., FIG. 29B).

The electrode 3108 may include an exposed ablation surface, which may be activated to ablate tissue, and a lead wire or other conductor attached thereto. Particularly, when activated, current may be supplied to and/or carried from tissue and fluid via the ablation surface to facilitate ablation or vaporization of tissue to form a fistula. In some embodiments, the electrode 3108 may be a spring wire or leaf spring electrode, which may be movable between a low-profile configuration, in which the electrode 3108 is retained within the catheter 3100, and an extended configuration, in which electrode 3108 projects from a surface of the catheter body 3102. The electrode 3108 may or may not be naturally biased to project from the catheter body 3102. When the electrode 3108 is naturally biased to project from the catheter body 3102, a structure, such as a retractable sheath 3160, may be used to hold or maintain the electrode 3108 in a low-profile configuration until deployment is desired. In some embodiments, the catheter body 3102 may comprise one or more insulating materials (not shown) which may shield or otherwise protect the catheter 3100 and its components from heat generated by the electrode 3108 during use.

Still referring to FIG. 26A, the catheter 3100 includes a non-active side 3103 or region positioned opposite the active side 3134 of the treatment portion 3135. For example, the non-active side 3103 refers to the side of the catheter 3100 devoid of cutting and/or ablation means. The non-active side 3103 of the catheter 3100 extends across the catheter body 3102 and the treatment portion 3135. In other words, the catheter body 3102 and the treatment portion 3135 may both define the non-active side 3103. The non-active side 3103 of the catheter body 3102 and treatment portion 3135 is diametrically opposite the active side 3134 of the treatment portion 3135. Therefore, the non-active side 3103 of the catheter body 3102 and treatment portion 3135 is positioned opposite the electrode 3108. In other words, the non-active side 3103 of the catheter body 3102 and treatment portion 3135 does not abut or face a modification being formed in the blood vessel 3300 (FIG. 29A) via the active side 3134 of the treatment portion 3135. Instead the non-active side 3103 of the catheter body 3102 may be spaced from a modification being formed in the blood vessel 3300 (FIG. 29A) by the electrode 3108 by the diameter or height (e.g. in the direction of the z-axis of the coordinate axes of FIG. 26A) of at least a portion of the catheter body 3102.

Still referring to FIG. 26A, the catheter 3100 may include one or more arrays of magnets arranged longitudinally along the catheter body 3102. For instance, the catheter 3100 may include a first array of magnets 3104 that extends longitudinally along the catheter body 3102 and is positioned proximal (e.g. in the -x direction of the coordinate axes of FIG. 26A) to the treatment portion 3135. The catheter 3100 may include a second array of magnets 3106 that extends longitudinally along the catheter body 3102 and is positioned distal (e.g. in the +x direction of the coordinate axes of FIG. 26A) to the treatment portion 3135. In some embodiments, the second array of magnets 3106 may be positioned longitudinally between (e.g. in the direction of the x-axis of the coordinate axes of FIG. 26A) the treatment portion 3135 and the distal tip 3140 of the catheter body 3102. In embodiments, the catheter 3100 may include a third array of magnets 3150 positioned in the treatment portion 3135 behind the active side 3134 of the treatment portion 3135, such as directly behind the active side 3134. More particularly, the third array of magnets 3150 may be positioned along and/or within the non-active side 3103 of the treatment portion 3135. In embodiments, the catheter 3100 may include the first array of magnets 3104, the second array of magnets 3106, and the third array of magnets 3150 individually or in any combination. It should be appreciated that while the phrase "array of magnets" is used herein, that each of the arrays of magnets 3104, 3106, and 3150 may be configured as a single magnet along the catheter body 3102 and/or treatment portion 3135.

The arrays of magnets 3104, 3106, and 3150 described herein may be permanent magnets comprising one or more hard magnetic materials, such as but not limited to alloys of rare earth elements (e.g., samarium-cobalt magnets or neodymium magnets, such as N52 magnets) or alnico. In some variations, the arrays of magnets 3104, 3106, and 3150 may comprise anisotropic magnets; in other variations, the arrays of magnets 3104, 3106, and 3150 may comprise isotropic magnetics. In some variations, the arrays of magnets 3104, 3106, and 3150 may be formed from compressed powder. In some variations, a portion of the arrays of magnets 3104, 3106, and 3150 (e.g., a permeable backing) may comprise one or more soft magnetic materials, such as but not limited to iron, cobalt, nickel, or ferrite. It should be appreciated that in systems comprising two catheters, either the first catheter 3100 or the second catheter 3400 (FIGS. 29A and 29B) may comprise ferromagnetic elements (i.e., elements attracted to but not generating a permanent magnetic field). For example, in some variations, the first catheter 3100 may include only one or more ferromagnetic elements while the second catheter 3400 (FIGS. 29A and 29B) may comprise one or more permanent magnets. In other variations, the second catheter 3400 (FIGS. 29A and 29B) may include only one or more ferromagnetic elements while the first catheter 3100 may comprise one or more permanent magnets. However, in other variations, one or both of the first catheter 3100 and the second catheter 3400 (FIGS. 29A and 29B) may include any suitable combination of ferromagnetic, permanent, and/or other suitable kinds of magnets.

Generally, the dimensions of the arrays of magnets 3104, 3106, and 3150 described herein may be selected based upon by the size of the catheter 3100 carrying the arrays of magnets 3104, 3106, and 3150, which in turn may be selected based upon the anatomical dimensions of the selected blood vessels through which the catheter 3100 may be advanced. For example, if the catheter 3100 is to be advanced through a blood vessel 3300 (FIGS. 29A and 29B) having an internal diameter of about 3 mm, it may be desirable to configure any array of magnets 3104, 3106, and 3150 to be less than about 3 mm at the widest part of their cross-sections, to reduce the risk of injury to vessel walls during advancement and manipulation of the catheter 3100. Each array of magnets 3104, 3106, and 3150 may have any suitable length (e.g., about 5 mm, about 10 mm, about 15 mm, about 20 mm, and the like), although it should be appreciated that in some instances longer arrays of magnets may limit the flexibility of the catheter 3100 to maneuver through a vessel. In some variations, the arrays of magnets 3104, 3106, and 3150 may include a plurality of square magnets. In other embodiments, each magnet of the arrays of magnets 3104, 3106, and 3150 may have any suitable shape for placement inside or outside of the catheter. Magnets may be cylindrical, semi-cylindrical, tube-shaped, box-shaped, or the like.

In embodiments, the outer surfaces of the arrays of magnets 3104, 3106, and 3150 may be flush or in line with the outer surface of the catheter body 3102. In other embodiments, the magnets 3104, 3106, and 3150 may be positioned radially within the catheter body 3102 away from the outer surface of the catheter body 3102. In other embodiments the outer surfaces of the arrays of magnets 3104, 3106, and 3150 may extend a distance radially beyond the outer surface of the catheter body 3102.

Each array of magnets 3104, 3106, 3150 may be fixed in or on the catheter 3100 by any suitable method. For example, in some variations the one or more arrays of magnets 3104, 3106, and 3150 may be embedded in, adhered to, or friction-fit within the catheter 3100.

Still referring to FIG. 26A, the catheter 3100 may further include a biasing stent 3120. The biasing stent 3120 may generally take any known or desired stent structure. For instance, the biasing stent 3120 generally includes a plurality of interconnected struts 3170. The struts 3170 may form a plurality of cells, which are generally a small, repetitive structure of the biasing stent 3120. The cells may, in turn, form a plurality of rings, and adjacent rings may be attached by connectors.

The biasing stent 3120 may extend longitudinally (e.g. in the direction of the x-axis of the coordinate axes of FIG. 26A) along a length of the catheter body 3102. The biasing stent 3120 may arch radially away from the catheter body 3102 between a proximal point 3130 and a distal point 3132 of the catheter body 3102. The proximal point 3130 may be positioned proximal (e.g. in the -x direction of the coordinate axes of FIG. 26A) a first end of at least one array of the arrays of magnets 3104, 3106, 3150, and the distal point 3132 may be positioned distal (e.g. in the +x direction of the coordinate axes of FIG. 26A) a second end of the at least one array of the arrays of magnets 3104, 3106, 3150. Accordingly, the biasing stent 3120 may be configured to longitudinally span the at least one array of the arrays of magnets 3104, 3106, 3150. In other words, at least one of the first array of magnets 3104, the second array of magnets 3106, and the third array of magnets 3150 may be positioned longitudinally (e.g. in the direction of the x-axis of the coordinate axes of FIG. 26A) between the proximal point 3130 and the distal point 3132. In embodiments the biasing stent 3120 may be arranged to bias the one or more arrays of magnets 3104, 3106, 3150 against a blood vessel wall, as will be discussed in further detail with respect to FIGS. 29A and 29B. In embodiments, the proximal point 3130 and/or the distal point 3132 may be positioned within the one or more arrays of magnets 3104, 3106, 3150.

In embodiments, the biasing stent 3120 may extend radially from the non-active side 3103 of the catheter 3100. In other words, the proximal point 3130 and distal point 3132 may be positioned along the non-active side 3103 of the catheter body 3102. More particularly, in embodiments, a lateral center point 3137 (FIG. 26B) of the biasing stent 3120 may be diametrically opposite the active side 3134 of the treatment portion 3135. The lateral center point 3137 may be a point that is equidistant from the laterally outward (e.g. in the direction of the y-axis of the coordinate axes of FIG. 26B) edges of the biasing stent 3120. In embodiments, the treatment portion 3135 may be positioned longitudinally (e.g. in the direction of the x-axis of the coordinate axes of FIG. 26A) between the proximal point 3130 and the distal point 3132. In embodiments, the biasing stent 3120 may be configured to bias the treatment portion 3135, and more specifically the active side 3134 of the treatment portion 3135, against a blood vessel wall, as will be discussed in further detail with respect to FIGS. 29A and 29B.

In embodiments, the biasing stent 3120 may include a first tapered section 3121, a second tapered section 3122, and a third section 3123 longitudinally positioned between the first tapered section 3121 and the second tapered section 3122. The first tapered section 3121 may be positioned proximal the second tapered section 3122. The first tapered section 3121 may be coupled to the catheter body 3102 at a proximal point 3124 of the first tapered section 3121. Particularly, the proximal point 3124 of the first tapered section 3121 may be coupled to the catheter body 3102 at the proximal point 3130. The first tapered section 3121 may further connect to the third section 3123 of the biasing stent 3120 at a distal point 3125 of the first tapered section 3121. The second tapered section 3122 may be coupled to the catheter body 3102 at a distal point 3126 of the second tapered section 3122. Particularly, the distal point 3126 of the second tapered section 3122 may be coupled to the catheter body 3102 at the distal point 3132. The second tapered section 3122 may further connect to the third section 3123 of the biasing stent 3120 at a proximal point 3127 of the second tapered section 3122.

As mentioned above, the biasing stent 3120 may radially arch away from the catheter body 3102 of the catheter 3100 between the proximal point 3130 and the distal point 3132. In some embodiments the biasing stent 3120 may be fixedly secured to the catheter body 3102 at the proximal point 3130 and the distal point 3132. In other words, the proximal point 3124 of the first tapered section 3121 may be fixedly secured to the catheter body 3102 at the proximal point 3130, and the distal point 3126 of the second tapered section 3122 may be fixedly secured to the catheter body 3102 at the distal point 3132. In such embodiments, the biasing stent 3120 may be fixed to the catheter body 3102 at the proximal point 3130 and the distal point 3132 with a suitable polymer or adhesive, such as glue, laser welding, heat shrunk plastic wrap, or the like.

In embodiments, the struts 3170 of the biasing stent 3120 may have a circular cross section. In embodiments, the struts 3170 of the biasing stent 3120 may be flat ribbons having a substantially rectangular cross section. In embodiments, the biasing stent 3120 may be made of metal, plastic, polymer, metal coated in plastic, a composite of any of said materials, and the like. For instance, the biasing stent 3120 may be nitinol, stainless steel, polyethylene terephthalate, polyether ether ketone, polytetrafluoroethylene, polyimide, and the like. The biasing stent 3120 may be made of a material that exhibits high radiopacity, allowing the biasing stent 3120 to be visualized under fluoroscopy. The biasing stent 3120 may be a material that exhibits shape memory and returns to a set or desired shape.

In some embodiments, the biasing stent 3120 may expand from a low-profile configuration to an extended configuration, depicted in FIG. 26A. In the low-profile configuration, the biasing stent 3120 may be positioned in a non-contacting state or non-biasing state, in which the biasing stent 3120 does not apply a biasing force to the wall of the blood vessel 3300 (FIG. 29A) to bias the catheter 3100 laterally within the blood vessel 3300 (FIG. 29A). In the low-profile configuration, the biasing stent 3120 may be maintained substantially flush with the catheter body 3102 of the catheter 3100. As such, the catheter 3100 may be advanced to a desired location within the blood vessel 3300 (FIG. 29A) without the biasing stent 3120 extending radially from the catheter body 3102 and applying a biasing force to the wall of the blood vessel 3300 (FIG. 29A) to bias the catheter 3100 laterally within the blood vessel 3300 (FIG. 29A). In the extended configuration, at least a portion of the biasing stent 3120 may radially extend outward from the outer surface of the catheter body 3102 to be in a contacting state, in which at least a portion of the biasing stent 3120 applies a biasing force to the wall of the blood vessel 3300 (FIG. 29A). Accordingly, a maximum distance of radial deflection of the biasing stent 3120 from the outer surface of the catheter body 3102, when in the extended configuration, may be greater than the maximum distance of radial deflection of the biasing stent 3120 from the outer surface of the catheter body 3102, when in the low-profile configuration.

When the biasing stent 3120 is in the extended configuration, the distal point 3125 of the first tapered section 3121 is a greater distance from the catheter body 3102 than the proximal point 3124 of the first tapered section 3121. Also, when in the extended configuration, the proximal point 3127 of the second tapered section 3122 is a greater distance from the catheter body 3102 than the distal point 3126 of the second tapered section 3122. Therefore, when the biasing stent 3120 is in the extended configuration, the first tapered section 3121 slopes from the catheter body 3102 to the third section 3123 of the biasing stent 3120, and the second tapered section 3122 slopes from the catheter body 3102 to the third section 3123 of the biasing stent 3120. In embodiments, a radially outer edge 3142 of the first tapered section 3121 may linearly slope from the non-active side 3103 of the catheter body 3102 to a radially outer edge 3146 of the third section 3123 of the biasing stent 3120, and a radially outer edge 3144 of the second tapered section 3122 may linearly slope from the non-active side 3103 of the catheter body 3102 to the radially outer edge 3146 of the third section 3123 of the biasing stent 3120. The sloped first tapered section 3121 and second tapered section 3122 may allow the catheter 3100, with the biasing stent 3120 exposed, to be advanced and retracted in the blood vessel 3300 (FIG. 29A) without causing undue stress to a vessel wall.

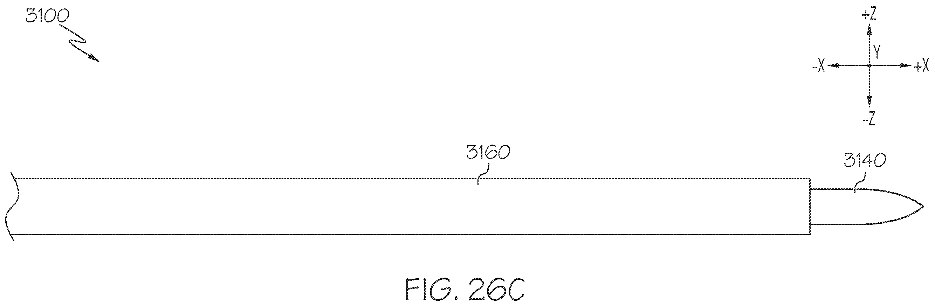

In embodiments, the biasing stent 3120 described herein may be biased toward the extended configuration. That is, the biasing stent 3120 may be configured to self-expand from the low-profile configuration to the extended configuration. Put yet another way, the biasing stent 3120 may be in its natural resting state in the extended configuration, extending a predetermined distance away from the outer surface of the catheter body 3102. In such embodiments, a force may be required to hold the biasing stent 3120 in the low-profile configuration. For instance, referring to FIGS. 26A-26C, a sheath 3160 may be advanced distally (e.g. in the +x direction of the coordinate axes of FIGS. 26A-26C) to maintain the biasing stent 3120 in the low-profile configuration. The sheath 3160 may include an inner lumen having a greater diameter than the catheter body 3102 and the one or more arrays of magnets 3104, 3106, 3150 of the catheter 3100, thereby allowing the sheath 3160 to be advanced distally over the catheter body 3102 and one or more arrays of magnets 3104, 3106, 3150, as depicted in FIG. 26C. Thus, with the sheath 3160 advanced distally over the catheter body 3102 and one or more arrays of magnets 3104, 3106, 3150, the biasing stent 3120 may be compressed by the sheath 3160 against the catheter body 3102. In other words, the biasing stent 3120 may be compressed and maintained in the low-profile configuration within the space between the catheter body 3102 and the sheath 3160. The catheter 3100 within the sheath 3160 may be advanced within the blood vessel 3300 (FIG. 29A) to a desirable location to form a fistula, for instance, at which point the sheath 3160 may be retracted in the proximal direction (e.g. in the -x direction of the coordinate axes of FIGS. 26A-26C), thereby exposing the biasing stent 3120. With the sheath 3160 no longer applying a force to the biasing stent 3120 to maintain the biasing stent 3120 in the low-profile configuration, and due to the natural bias of the biasing stent 3120, the biasing stent 3120 may naturally expand into the extended configuration, as shown in FIG. 26A.

In some embodiments, the biasing stent 3120 may be made of a shape-memory alloy, such as copper-aluminum-nickel and nickel-titanium, that changes shape due to environmental cues, such as temperature. For instance, the active shape of the biasing stent 3120 may be the extended configuration depicted in FIG. 26A. The transition temperature of the shape-memory alloy may be above standard room temperatures. In some embodiments, the transition temperature of the shape-memory alloy may be roughly at internal body temperature. Therefore, outside of a patient, at standard room temperature, the biasing stent 3120 may deform into the low-profile configuration, in which the biasing stent 3120 is in a non-contacting state and does not extend radially away from the outer surface of the catheter body 3102 to apply a biasing force to a wall of the blood vessel 3300 (FIG. 29A). As the temperature of the biasing stent 3120 increases, the biasing stent 3120 may naturally transition from the low-profile configuration to the extended configuration, in which the biasing stent 3120 is in a contacting state and extends radially away from the outer surface of the catheter body 3102 to apply a biasing force to the wall of the blood vessel 3300 (FIG. 29A) to bias the catheter 3100 laterally within the blood vessel 3300 (FIG. 29A). In embodiments, the biasing stent 3120 may transition from the low-profile configuration to the extended configuration at temperatures above 30 °C, temperatures above 32 °C, temperatures above 35 °C, and like temperatures between standard room temperature of 20 °C and internal body temperature at 37 °C.

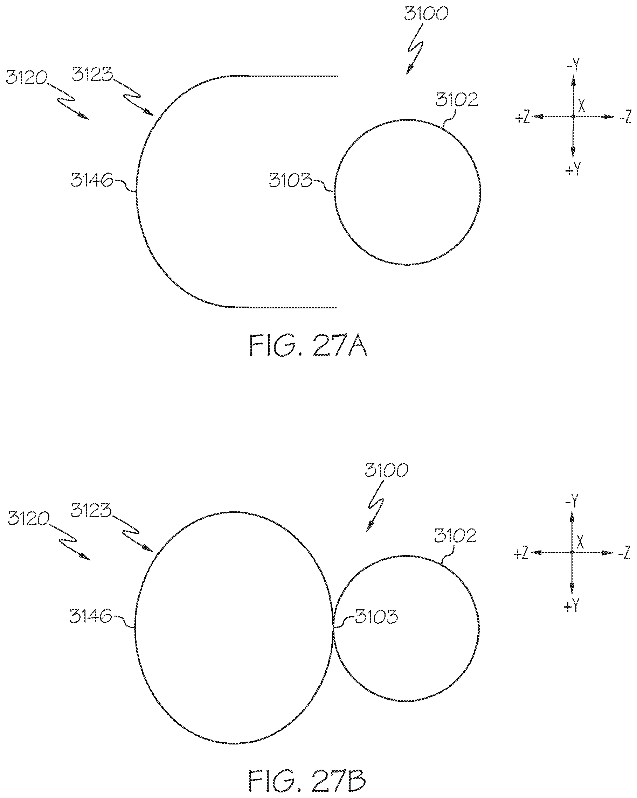

Referring now to FIGS. 27A and 27B, cross sections of the catheter 3100 are depicted. The cross sections are particularly taken through the catheter body 3102 and the third section 3123 of the biasing stent 3120. As depicted in FIG. 27A, in embodiments, the biasing stent 3120 may have a semi-elliptical cross-section. The biasing stent 3120 may form a semi-elliptical cross-section, particularly, when in the extended configuration. As depicted in FIG. 27B, in other embodiments, the biasing stent 3120 may have an elliptical cross section.

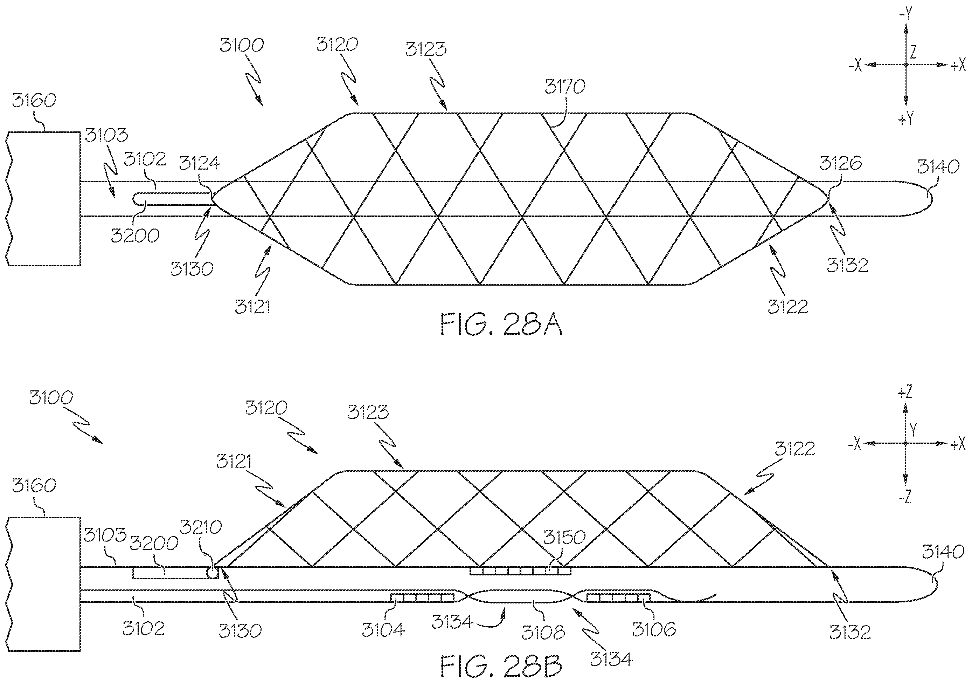

In some embodiments, the biasing stent 3120 described herein may be configured such that the biasing stent 3120 is longitudinally moveable (e.g. in the direction of the x-axis of FIG. 28A) along the catheter body 3102 of the catheter 3100. For instance, and with reference to FIGS. 28A-28C, the biasing stent 3120 may be slidable within a track 3200. The track 3200 may be an opening in the outer surface of the catheter body 3102. In embodiments, the biasing stent 3120 may extend from the track 3200 in the catheter body 3102 at the proximal point 3130. That is, the proximal point 3130 may define at least a portion of the track 3200 in the catheter body 3102. More particularly, the proximal point 3130 may define a distal end of the track 3200. The biasing stent 3120 may move proximally (e.g. in the -x direction of the coordinate axes of FIGS. 28A-28C) within the track 3200 and along the catheter body 3102 from the proximal point 3130.

The proximal point 3124 (FIG. 26A) of the first tapered section 3121 may be positioned in, and slide within, the track 3200. In embodiments, the proximal point 3124 (FIG. 26A) of the first tapered section 3121 may include, or be coupled to, an anchor member 3210. The anchor member 3210 may be sized and shaped such that the anchor member 3210 may slide within the track 3200, and such that the anchor member 3210 may not exit the track 3200. That is, the anchor member 3210 may not be able to exit the track 3200 and extend radially away from the outer surface of the catheter body 3102.

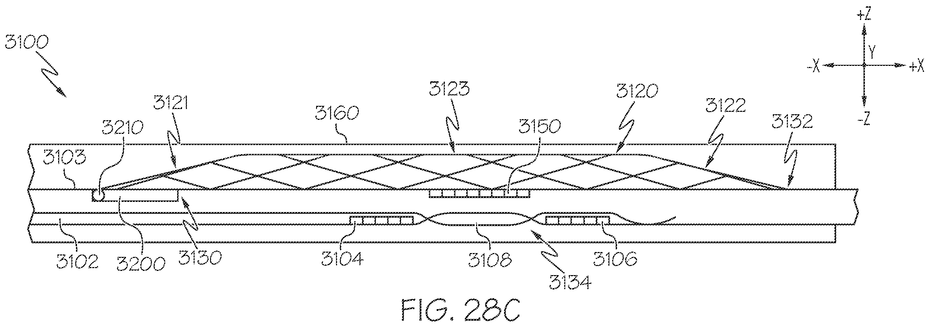

In the low-profile configuration, as depicted in FIG. 28C, the anchor member 3210, and the proximal point 3124 (FIG. 26A) of the first tapered section 3121, may be positioned at a proximal end of the track 3200. The anchor member 3210, and the proximal point 3124 (FIG. 26A) of the first tapered section 3121 may move distally within the track 3200 to a distal end of the track 3200, thereby urging the biasing stent 3120 to radially extend away from the catheter body 3102 between the proximal point 3130 and the distal point 3132 and move into the extended configuration, as depicted in FIG. 28B.

In embodiments, the biasing stent 3120 may be biased to the extended configuration. In other words, the anchor member 3210, and the proximal point 3124 (FIG. 26A) of the first tapered section 3121, may be biased to slide distally within the track 3200. The sheath 3160 may maintain the biasing stent 3120 in the low-profile configuration. For instance, the sheath 3160 may be advanced distally (e.g. in the +x direction of the coordinate axes of FIGS. 28A-28C) over the catheter 3100. The sheath 3160 may apply an inward force on the biasing stent 3120, compressing the biasing stent 3120 such that the anchor member 3210, and the proximal point 3124 (FIG. 26A) of the first tapered section 3121, slides proximally (e.g. in the -x direction of the coordinate axes of FIGS. 28A-28C) within the track 3200. The sheath 3160 may further prevent the anchor member 3210, and the proximal point 3124 (FIG. 26A) of the first tapered section 3121, from sliding distally within the track 3200. Accordingly, the anchor member 3210, and the proximal point 3124 (FIG. 26A) of the first tapered section 3121, may be maintained at a proximal end of the track 3200, and the biasing stent 3120 may be maintained in the low-profile configuration. Moreover, removal of the sheath 3160 proximally (e.g. in the -x direction of the coordinate axes of FIGS. 28A-28C) may allow the anchor member 3210, and the proximal point 3124 (FIG. 26A) of the first tapered section 3121, to slide distally within the track 3200 to the distal end of the track 3200, or in other words, to the proximal point 3130 of the catheter body 3102. Accordingly, the biasing stent 3120 may assume the extended configuration depicted in FIG. 28B.

It should be appreciated that, in embodiments, the biasing stent 3120 may be fixed to the catheter body 3102 at the distal point 3132 and slide within the track 3200 from the proximal point 3130. In other embodiments, the biasing stent 3120 may similarly be fixed to the catheter body 3102 at the proximal point 3130 and slide within a track 3200 from the distal point 3132. In such embodiments, the distal point 3132 may define at least a portion of the track 3200 in the catheter body 3102. More particularly, the distal point 3132 may define a proximal end of the track 3200. The biasing stent 3120 may move distally (e.g. in the +x direction of the coordinate axes of FIGS. 28A-28C) within the track 3200 and along the catheter body 3102 from the distal point 3132. In such embodiments, the biasing stent 3120 may operate as described above, in that the biasing stent 3120 may transition between the low-profile configuration and the extended configuration depending on the position of an anchor member 3210, and the distal point 3126 (FIG. 26A) of the second tapered section 3122. within the track 3200. In some embodiments, the biasing stent 3120 may slide within a first track 3200 from the proximal point 3130 and within a second track 3200 from the distal point 3132.

In the embodiments of the catheter 3100 discussed, the natural bias of the biasing stent 3120 may eliminate the need for a user-actuated control that in a first state holds the biasing stent 3120 in a low-profile configuration for delivery through the vasculature and in a second state allows the biasing stent 3120 to enter the extended configuration for applying a biasing force to the wall of the blood vessel 3300 (FIG. 29A). Although the biasing stent 3120 may be moveable between low-profile and extended configurations, this movement may occur as a natural result of the bias of the biasing stent 3120 in combination with external forces (such as from the sheath 3160), and the biasing stent 3120 remains in a single state throughout use. That is, during both advancement of the catheter 3100 through vasculature and tissue ablation, the catheter 3100 is in a state in which the biasing stent 3120 would be able to be in the extended configuration in the absence of an external force pressing on the biasing stent 3120. Due to the shape-memory of the biasing stent 3120 and the natural bias of the biasing stent 3120, the biasing stent 3120 may return to a set shape (e.g. the extended configuration). Accordingly, the angle, shape, curvature, and distance of radial extension from the catheter body 3102 of the biasing stent 3120 in the extended configuration may be particularly selected. The distance of radial extension from the catheter body 3102 of the biasing stent 3120 may be particularly selected and/or configured based on the size of the catheter 3100 and the size of the blood vessel 3300 (FIG. 29A) the catheter 3100 is advanced through. For instance, the distance of radial extension of the biasing stent 3120 may be selected to bias the catheter 3100 against a blood vessel wall in a blood vessel having a diameter of about 2 mm to about 4 mm, greater than 4 mm, greater than 6 mm, greater than 8 mm, about 10 mm, and the like.

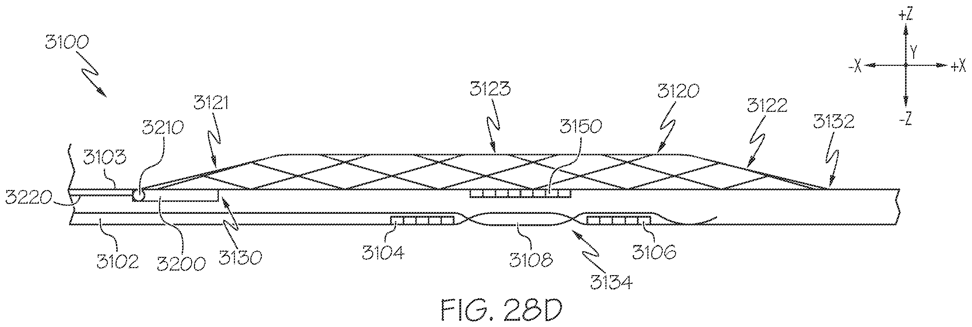

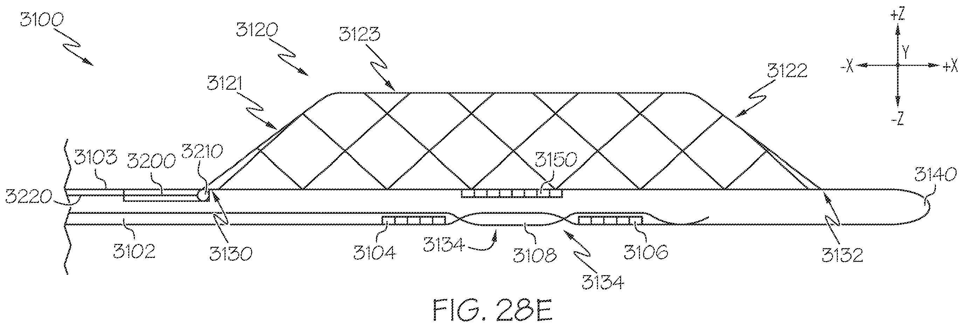

Referring now to FIGS. 28D and 28E, in some embodiments, the biasing stent 3120 may be user-manipulated from the low-profile configuration to the extended configuration. For example, in embodiments in which the biasing stent 3120 is slidable within the track 3200, the anchor member 3210 may be coupled to a rod 3220. The rod 3220 may extend proximally (e.g. in the -x direction of the coordinate axes of FIGS. 28D and 28E) through a lumen of the catheter body 3102 to a hand control, switch, actuator, or other user-manipulated device coupled to the rod 3220. The track 3200 may include an opening at its proximal end to allow the rod 3220 to be advanced through the track 3200. The opening at the proximal end of the track 3200 may be sized such that the anchor member 3210 may be unable to move through the opening. Through actuation of the user-manipulated device, a user may advance the anchor member 3210, and the proximal point 3124 (FIG. 26A) of the first tapered section 3121, distally (e.g. in the +x direction of the coordinate axes of FIGS. 28D and 28E) in the track 3200 and/or retract the anchor member 3210, and the proximal point 3124 (FIG. 26A) of the first tapered section 3121, proximally (e.g. in the direction of the -x direction of the of the coordinate axes of FIGS. 28D and 28E) in the track 3200. In such embodiments, the biasing stent 3120 may be fixed to the catheter body 3102 at the distal point 3132. Accordingly, retraction of the anchor member 3210 in the track 3200 may cause the biasing stent 3120 to collapse into the low-profile configuration, as shown in FIG. 28D, and advancement of the anchor member 3210 in the track 3200 may cause the biasing stent 3120 to expand into the extended configuration, as shown in FIG. 28E.

Referring now to FIGS. 29A and 29B a system and method for forming a fistula between the first blood vessel 3300 and a second blood vessel 3302 with the first catheter 3100 and the second catheter 3400 will now be discussed. Referring first to FIG. 29A, the first catheter 3100 may be advanced within a lumen of the blood vessel 3300. The second catheter 3400 may be placed in a blood vessel 3302 that is adjacent to the blood vessel 3300. The second catheter 3400 may resemble the first catheter 3100 discussed herein. In some embodiments, the second catheter 3400 may not include an electrode 3108. The second catheter 3400 includes a catheter body 3402. The catheter body 3402 further defines a treatment portion 3435 having an active side 3434. The second catheter 3400 further includes a non-active side 3403 defined by the catheter body 3402 and the treatment portion 3435. Specifically, the active side 3434 of the treatment portion 3435 may include a recess 3408 configured to receive the electrode 3108 of the first catheter 3100. The recess 3408 may be particularly shaped, sized, and/or the like to receive the electrode 3108 of the first catheter 3100 therein. In other embodiments, the second catheter 3400 may include an electrode that extends from the treatment portion 3435 and radially away from the catheter body 3402. The second catheter body 3402 of the second catheter 3400 may further include one or more arrays of magnets. For instance, the second catheter 3400 may include a first array of magnets 3404 positioned proximal (e.g. in the -x direction of the coordinate axes of FIGS. 29A and 29B) to the treatment portion 3435, a second array of magnets 3406 positioned distal (e.g. in the +x direction of the coordinate axes of FIGS. 29A and 29B) to the treatment portion 3435, and/or a third array of magnets 3450 positioned along the treatment portion 435, and particularly along the non-active side 3403 of the treatment portion 3435 in some embodiments. In embodiments, the second catheter 3400 may include a second biasing stent 3420 that mirrors the first biasing stent 3120 of the first catheter 3100 in structure and operation. While in embodiments described below, the first catheter 3100 includes the first biasing stent 3120 and the second catheter 3400 includes the second biasing stent 3420, it should be appreciated that only one of the first catheter 3100 and the second catheter 3400 may include a biasing stent.

The one or more arrays of magnets 3104, 3106, 3150 of the first catheter 3100 and the one or more arrays of magnets 3404, 3406, 3450 of the second catheter 3400 may be configured to promote rotational and axial alignment of the catheters 3100 and 3400. Proper axial and rotational alignment between catheters 3100 and 3400 may facilitate alignment of one or more fistula-forming elements, such as the treatment portions 3135, 3435 of the first and second catheters 3100, 3400, respectively. More specifically, proper axial and rotational alignment between the first catheter 3100 and the second catheter 3400 may facilitate alignment of the electrode 3108 with the recess 3408. The one or more arrays of magnets 3104, 3106, 3150 of the first catheter 3100 may be arranged such that the magnetic fields generated by the one or more arrays of magnets 3104, 3106, 3150 are stronger in the direction of the active side 3134 of the treatment portion 3135 (e.g. in the -z direction of the coordinate axes of FIGS. 29A and 29B) than in the direction of the non-active side 3103 of the treatment portion 135 (e.g. in the +z direction of the coordinate axes of FIGS. 29A and 29B). Similarly, the one or more arrays of magnets 3404, 3406, 3450 of the second catheter 3400 may be arranged such that the magnetic fields generated by the one or more arrays of magnets 3404, 3406, 3450 are stronger in the direction of the active side 3434 of the treatment portion 3435 (e.g. in the +z direction of the coordinate axes of FIGS. 29A and 29B) than in the direction of the non-active side 3403 of the treatment portion 3435 (e.g. in the -z direction of the coordinate axes of FIGS. 29A and 29B). In such embodiments, the strength of the magnetic fields in the directions of the active sides 3134, 3434 of the treatment portions 3135, 3435, respectively, may promote rotational alignment between the active side 3134 of the treatment portion 3135 of first catheter 3100 in the first blood vessel 3300 and the active side 3434 of the treatment portion 3435 of the second catheter 3400 in the second blood vessel 3302.

The catheters 3100 and 3400, as depicted in FIG. 29A are axially misaligned, such that the electrode 3108 of the first catheter 3100 is not aligned with the recess 3408 of the second catheter 3400 in the x-direction of the coordinate axes of FIGS. 29A and 29B. Moreover, as depicted in FIG. 29A, for instance, the first catheter 3100 and the second catheter 3400 are in weak coaptation. When in weak coaptation, space may remain between at least one of the active side 3134 of the treatment portion 3135 of the first catheter 3100 and an adjacent wall of the blood vessel 3300 and the active side 3434 of the treatment portion 3435 of the second catheter 3400 and an adjacent wall of the blood vessel 3302. Therefore, in weak coaptation, the active side 3134 of the treatment portion 3135 and the active side 3434 of the treatment portion 3435 are not in close approximation with one another (e.g. in the direction of the z-axis of the coordinate axes of FIGS. 29A and 29B).

Each array of magnets 3404, 3406, 3450 of the second catheter 3400 may be configured to mate with a corresponding array of magnets 3104, 3106, 3150 of the first catheter 3100, and vice versa, such that the first catheter 3100 and the second catheter 3400 may be aligned and coapted. As used herein, the terms "coapted" and/or "strong coaptation" may be understood to mean that the first catheter 3100 and the second catheter 3400 are in close approximation (e.g. in the direction of the z-axis of the coordinate axes of FIGS. 29A and 29B) such that the electrode 3108 of the first catheter 3100 may enter the recess 3408 of the second catheter 3400. As used herein, the term "mate" may be understood to mean a mutual attraction between an array of magnets of the first catheter 3100 and an array of magnets of the second catheter 3400. For instance, the first array of magnets 3104 positioned proximal (e.g. in the -x direction of the coordinate axes of FIGS. 29A and 29B) the treatment portion 3135 of the catheter 3100 may be configured to mate with the first array of magnets 3404 positioned proximal (e.g. in the -x direction of the coordinate axes of FIGS. 29A and 29B) the treatment portion 3435 of the second catheter 3400. The second array of magnets 3106 positioned distal (e.g. in the +x direction of the coordinate axes of FIGS. 29A and 29B) the treatment portion 3135 of the catheter 3100 may be configured to mate with the second array of magnets 3406 positioned distal (e.g. in the +x direction of the coordinate axes of FIGS. 29A and 29B) the treatment portion 3435 of the second catheter 3400. Similarly, the third array of magnets 3150 positioned along the non-active side 3103 of the treatment portion 3135 of the first catheter 3100 may be configured to mate with the third array of magnets 3450 positioned along the non-active side 3403 of the treatment portion 3435 of the second catheter 3400. It should be appreciated that the third array of magnets 3150 of the first catheter 3100 and the third array of magnets 3450 of the second catheter 3400 may be configured and arranged such that when mated, coaptation is promoted between the active side 3134 of the treatment portion 3135 and the active side 3434 of the treatment portion 3435 such that the third array of magnets 3150 and the third array of magnets 3450 remain separated by the treatment portions 3135, 3435 (e.g. in the direction of the z-axis of the coordinate axes of FIGS. 29A and 29B).

During a fistula-forming procedure, however, the one or more arrays of magnets 3104, 3106, and 3150 of the first catheter 3100 and the one or more arrays of magnets 3404, 3406, and 3450 of the second catheter 3400 may be insufficient on their own to align and coapt the first catheter 3100 and the second catheter 3400. For instance, due to limitations in the strength of the one or more arrays of magnets 3104, 3106, 3150 of the first catheter 3100 and the one or more arrays of magnets 3404, 3406, 3450 of the second catheter 3400, limitations on the degree of flexibility of the first catheter 3100 and the second catheter 3400, a distance between the vessels 3300 and 3302, and/or a tortuous anatomy of the first blood vessel 3300 and/or the second blood vessel 3302, the one or more arrays of magnets 3104, 3106, 3150 of the first catheter 3100 and the one or more arrays of magnets 3404, 3406, 3450 of the second catheter 3400 may be unable to mate. Therefore, as depicted in FIG. 29A the first catheter 3100 and the second catheter 3400 may be axially misaligned and/or in weak cooptation.

The biasing stent 3120 of the first catheter 3100 and/or the biasing stent 3420 of the second catheter 3400 may assist the one or more arrays of magnets 3104, 3106, 3150 of the first catheter 3100 and the one or more arrays of magnets 3404, 3406, 3450 of the second catheter 3400 in aligning and coapting the first catheter 3100 and the second catheter 3400. For instance, referring to FIG. 29A, the first catheter 3100 and the second catheter 3400 may be advanced in the blood vessels 3300 and 3302, respectively, with the biasing stent 3120 and/or the biasing stent 3420 in the low-profile configuration to ease distal advancement (e.g. in the +x direction of the coordinate axes of FIGS. 29A and 29B) of the catheters 3100 and 3400. The biasing stent 3120 of the first catheter 3100 and/or the biasing stent 3420 of the second catheter 3400 may be maintained in the low-profile configurations by any of the methods discussed above with respect to FIGS. 26A-26C and 28A-28E, for instance. With the biasing stent 3120 of the first catheter 3100 and/or the biasing stent 3420 of the second catheter 3400 in the low-profile configuration, the first catheter 3100 and the second catheter 3400 may be advanced within the blood vessels 3300 and 3302, respectively, until the treatment portion 3135 of the first catheter 3100 and the treatment portion 3435 of the second catheter 3400 are generally positioned at a desired site for forming a fistula between the blood vessels 3300 and 3302. However, as stated above and depicted in FIG. 29A, the one or more arrays of magnets 3104, 3106, 3150 of the first catheter 3100 and the one or more arrays of magnets 3404, 3406, 3450 of the second catheter 3400 may be unable to align and coapt the catheters 3100 and 3400 on their own.

Referring now to FIG. 29B, to encourage alignment and coaptation between the first catheter 3100 and the second catheter 3400, the biasing stent 3120 of the first catheter 3100 and/or the biasing stent 3420 of the second catheter 3400 may be transitioned from the low-profile configuration to the extended configuration. In some embodiments, only the biasing stent 3120 of the first catheter 3100 may be transitioned from the low-profile configuration to the extended configuration to encourage alignment and coaptation between the first catheter 3100 and the second catheter 3400. In some embodiments, only the biasing stent 3420 of the second catheter 3400 may be transitioned from the low-profile configuration to the extended configuration to encourage alignment and coaptation between the first catheter 3100 and the second catheter 3400. In embodiments, the biasing stent 3120 and/or the biasing stent 3420 may transition from the low-profile configuration to the extended configuration by any device or method discussed with reference to FIGS. 26A-26C and 28A-28E.

In embodiments in which the biasing stents 3120 and 3420 are naturally biased from the low-profile configuration to the extended configuration, the biasing stents 3120 and 3420 may begin to extend radially away from the catheter bodies 3102 and 3402, respectively. For instance, the biasing stent 3120 may extend toward the maximum height of the extended configuration until the biasing stent 3120 contacts a wall of the blood vessel 3300. Accordingly, the biasing stent 3120 may apply a biasing force against the wall of the blood vessel 3300, resulting in a biasing reaction force to be applied to the catheter 3100 to push the active side 3134 and/or the one or more magnetic arrays against a wall of the blood vessel 3300. More specifically, as the lateral center point 3137 (FIG. 26B) of the biasing stent 3120 is diametrically opposite the active side 3134 of the treatment portion 3135, the biasing reaction force may direct the active side 3134 of the treatment portion 3135 against a wall of the blood vessel 3300 at a position diametrically opposite the lateral center point 3137 (FIG. 26B) of the biasing stent 3120.

Similarly, the biasing stent 3420 of the second catheter 3400 may transition to the extended configuration to contact a wall of the second blood vessel 3302, resulting in a biasing reaction force that directs the active side 3434 of the treatment portion 3435 of the second catheter 3400 against a wall of the second blood vessel 3302. For example, the biasing stent 3420 of the second catheter 3400 may be deployed when the active side 3434 of the treatment portion 3435 of the second catheter 3400 is substantially aligned with the active side 3134 of the treatment portion 3135 of the first catheter 3100. Accordingly, the biasing stents 3120 and 3420 may strengthen the coaptation between the first catheter 3100 and the second catheter 3400. For example, and as described above, the biasing stent 3120 of the first catheter 3100 may bias, in addition to the active side 3134 of the treatment portion 3135, the one or more arrays of magnets 3104, 3106, 3150 against the wall of the first blood vessel 3300, and the biasing stent 3420 of the second catheter 3400 may bias, in addition to the active side 3434 of the treatment portion 3435, the one or more arrays of magnets 3404, 3406, 3450 against the wall of the second blood vessel 3302 thereby increasing attraction between the one or more arrays of magnets 3104, 3105, 3150 of the first catheter 3100 with the one or more arrays of magnets 3404, 3406, 3450 of the second catheter 3400 by decreasing distance between them. For example, once in closer approximation, the one or more arrays of magnets 3104, 3106, 3150 of the first catheter 3100 and the one or more arrays of magnets 3404, 3406, 3450 of the second catheter 3400 may be able to mate, overcoming any previous barriers, such as the distance between magnets, the flexibility of the catheters 3100, 3400, the distance between the vessels 3300, 3302, the tortuous anatomy of the first and second blood vessels 3300, 3302, and/or the like. The mating of the one or more arrays of magnets 3104, 3106, 3150 of the first catheter 3100 and the one or more arrays of magnets 3404, 3406, 3450 of the second catheter 3400 may coapt and align the catheters 3100 and 3400. After the first catheter 3100 and the second catheter 3400 are coapted and aligned, the electrode 3108 may be advanced and energized to ablate the wall of the first blood vessel 3300 and the wall of the second blood vessel 3302 and advance into the recess 3408 of the second catheter 3400, thereby forming a fistula between the first and second blood vessels 3300, 3302.

In other embodiments, only one of the catheters 3100, 3400 may be used in a method for forming a fistula between the first blood vessel 3300 and the second blood vessel 3302. For instance, a contrast dye may be injected into the second blood vessel 3302, such that the second blood vessel 3302 is visible under fluoroscopy. The first catheter 3100 may then be advanced in the first blood vessel 3300 until the treatment portion 3135 is generally aligned with a desired site to form a fistula between the first and second blood vessels 3300, 3302. The biasing stent 3120 may be made of a material that exhibits high radiopacity, allowing the biasing stent 3120 of the first catheter 3100 to be visualized under fluoroscopy, aiding a user in identifying the position of the biasing stent 3120 and the treatment portion 3135 of the first catheter 3100 in the first blood vessel 3300. The biasing stent 3120 of the first catheter 3100 may transition from the low-profile configuration to the extended configuration by any device or method discussed with reference to FIGS. 26A-26C and 28A-28E. In doing so, the biasing stent 3120 of the first catheter 3100 may bias the active side 3134 of the treatment portion 3135 against the wall of the first blood vessel 3300 at a desired site to form a fistula between the first blood vessel 3300 and the second blood vessel 3302. The electrode 3108 may then be advanced and energized to ablate the wall of the first blood vessel 3300 and the wall of the second blood vessel 3302, thereby forming a fistula between the first and second blood vessels 3300, 3302.

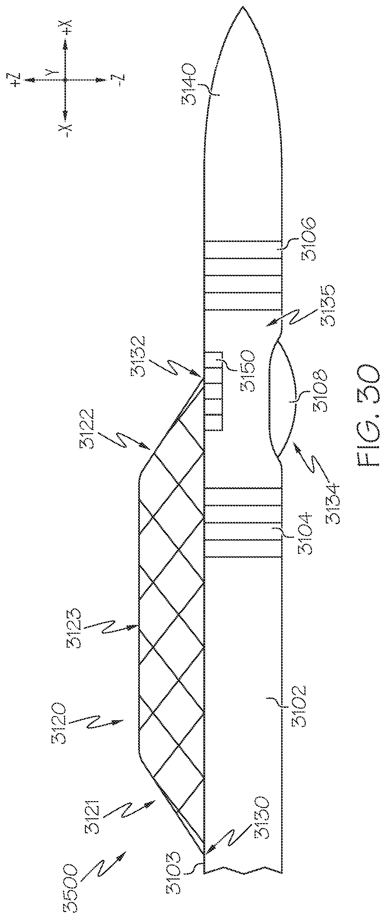

Referring now to FIG. 30, a side view of a catheter 3500 is depicted. The catheter 3500 may resemble the catheter 3100 discussed in FIGS. 26A-29B in all aspects except as discussed herein. For instance, similar to the catheter 3100, the catheter 3500 may include the catheter body 3102 defining the treatment portion 3135 and the one or more arrays of magnets 3104, 3106, 3150. The treatment portion 3135 may include the active side 3134 diametrically opposite the non-active side 3103. The catheter 3500 includes the biasing stent 3120 that may radially arch away from the catheter body 3102 between the proximal point 3130 and the distal point 3132. Unlike the catheter 3100 discussed above, the distal point 3132 of the catheter 3500 may be positioned along the non-active side 3103 of the treatment portion 3135. In such embodiments, the first array of magnets 3104 may be positioned between the proximal point 3130 and the distal point 3132. In embodiments, the distal point 3132 may be longitudinally positioned (e.g. in the direction of the x-axis of the coordinate axes of FIG. 30) along the third array of magnets 3150. In embodiments, the location of the distal point 3132 and/or the positioning and/or length of the third array of magnets 3150 may be adjusted such that the third array of magnets 150 is positioned between (e.g. in the direction of the x-axis of the coordinate axes of FIG. 30) the proximal point 3130 and the distal point 3132. In some embodiments, the proximal point 3130 may be positioned along the non-active side 3103 of the treatment portion 3135 and the distal point 3132 may be at a proximal end (e.g. in the -x direction of the coordinate axes of FIG. 30) of the distal tip 3140 such that the second array of magnets 3106 is positioned between the proximal point 3130 and the distal point 3132. In such embodiments, the third array of magnets 3150 may be positioned between the proximal point 3130 and the distal point 3132. Similar to the catheter 3100, the biasing stent 3120 of the catheter 3500 may be coupled to, or movable within a track of, the catheter body 3102 at the proximal point 3130, and the biasing stent 3120 may be coupled to, or movable within a track of, the catheter body 3102 at the distal point 3132.

### Systems and Methods

Various systems and methods will now be described including the various embodiments of the above-described catheters. It is noted that while only specific embodiments may be illustrated within the figures. The present system and methods may be applicable to any of the catheter systems described herein.

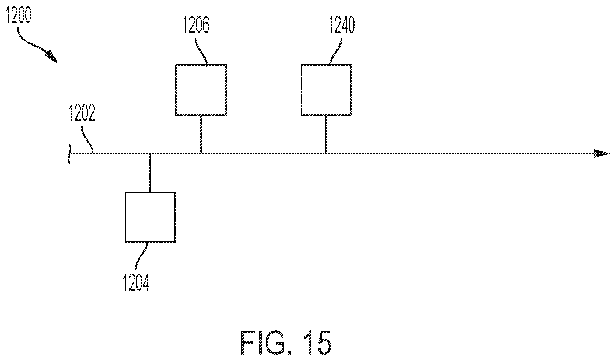

FIG. 15 generally schematically depicts communication between various modules within a system 1200 for endovascular treatment of a blood vessel. In particular, the system 1200 includes a communication path 1202, a control unit 1204, an imaging device 1206, and a display 1240. It is noted that in various embodiments a fewer or greater number of modules may be included within the system 1200 without departing from the scope of the present disclosure. Additionally, the system includes one or more catheters such as any of the two catheter or single catheter systems described herein above. That is the system may include a single catheter system configured to generate a fistula or deliver another type of vascular treatment to a target location within a vessel or a dual catheter system configured to generate a fistula between the two catheters or deliver some other type of vascular treatment.

The various modules of the system 1200 may be communicatively coupled to one another over the communication path 1202. The communication path 1202 may be formed from any medium that is capable of transmitting a signal such as, for example, conductive wires, conductive traces, optical waveguides, or the like. Moreover, the communication path 1202 may be formed from a combination of mediums capable of transmitting signals. In some embodiments, the communication path 1202 includes a combination of conductive traces, conductive wires, connectors, and buses that cooperate to permit the transmission of electrical data signals between the various components of the components such as processors, memories, sensors, input devices, output devices, and communication devices. Additionally, it is noted that the term "signal" means a waveform (e.g., electrical, optical, magnetic, mechanical or electromagnetic), such as DC, AC, sinusoidal-wave, triangular-wave, square-wave, vibration, and the like, capable of traveling through a medium.

The control unit 1204 can be any type of computing device and includes one or more processors and one or more memory modules. The one or more processors may include any device capable of executing machine-readable instructions stored on a non-transitory computer-readable medium, such as those stored on the one or more memory modules. Accordingly, each of the one or more processors may include a controller, an integrated circuit, a microchip, a computer, and/or any other computing device.

The one or more memory modules of the control unit 1204 are communicatively coupled to the one or more processors. The one or more memory modules may be configured as volatile and/or nonvolatile memory and, as such, may include random access memory (including SRAM, DRAM, and/or other types of RAM), flash memory, secure digital (SD) memory, registers, compact discs (CD), digital versatile discs (DVD), and/or other types of non-transitory computer-readable mediums. Depending on the particular embodiment, these non-transitory computer-readable mediums may reside within the control unit 1304, as shown, and/or external to the control unit 1304. The one or more memory modules may be configured to store logic (i.e., machine readable instructions) that, when executed by the one or more processors, allow the control unit to perform various functions that will be described in greater detail below.

The imaging device 1206 may be any imaging device configured to capture image data of the one or more catheters and surrounding vasculature as the catheter is advanced through the blood vessel. For example, and as described above, the imaging device 1306 may be an intravascular imaging device (e.g., IVUS, ICE, OCT, etc.) coupled to the housing of the catheter. Intravascular imaging devices are described in greater detail above. In other embodiments, the imaging device 1306 may be an external imaging device such as, for example an ultrasound device (e.g., a 2D ultrasound device and/or a 3D ultrasound device).

The imaging device 1206 may be communicatively coupled to the control unit over the communication path. Based on the data received from the imaging device 1306, the control unit may be able to process the image data to determine the rotational orientation of the catheter, and more specifically, the rotational orientation of the treatment portion of the catheter. In two catheter systems, the control unit may be able to determine proper alignment (e.g., longitudinal, rotational, and distance) between the two catheters for delivery of a vascular treatment.

As noted above, the system 1200 further includes a display 1240 communicatively coupled to the other modules of the system 1200 over the communication path 1202. The display 1240 may be any type of display configured to display image data from the imaging device 1206. In some embodiments, the control unit 1204 may process image data and with the display, project indicators onto the image to indicate, for example, rotational alignment, longitudinal alignment, distance between blood vessels, blood vessel labels (artery, catheter, perforator, etc.), etc. In embodiments wherein the imaging device comprises Doppler functionality, the control unit may be configured to display Doppler information include flow rate, volume, vessel pressure, etc. In various embodiments, the control unit may display the treatment portion of the catheter in real time as the treatment portion is advanced through the vasculature of the patient.

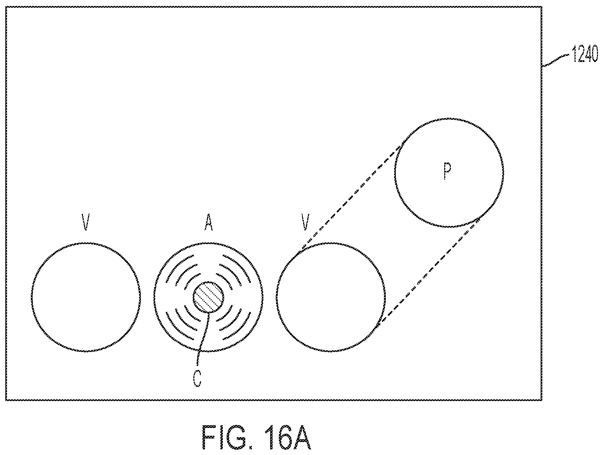

As discussed herein, methods may include selection of a blood vessel for access. As noted above, access to a vein or artery may be provided at the wrist or elsewhere. The catheter may be advanced through the blood vessel to a desired location, such as proximate to a perforator. For example, with reference to FIGS. 16A-16D. a depiction of a display 1240 showing axial cross-section image data from the imaging device 1206 is generally depicted. The imaging device 1206 may show the catheter C being advanced through an artery A, comitant veins V may be positioned on either side of the artery A. The catheter C may be advanced until a perforator P to one of the veins becomes visible on the display. The catheter C may continue to be advanced or retracted until the perforator is shown to meld into the vein V from which it extends. That may be the desired position for vascular treatment (e.g., fistula formation) as it is close to the origin of the perforator P.

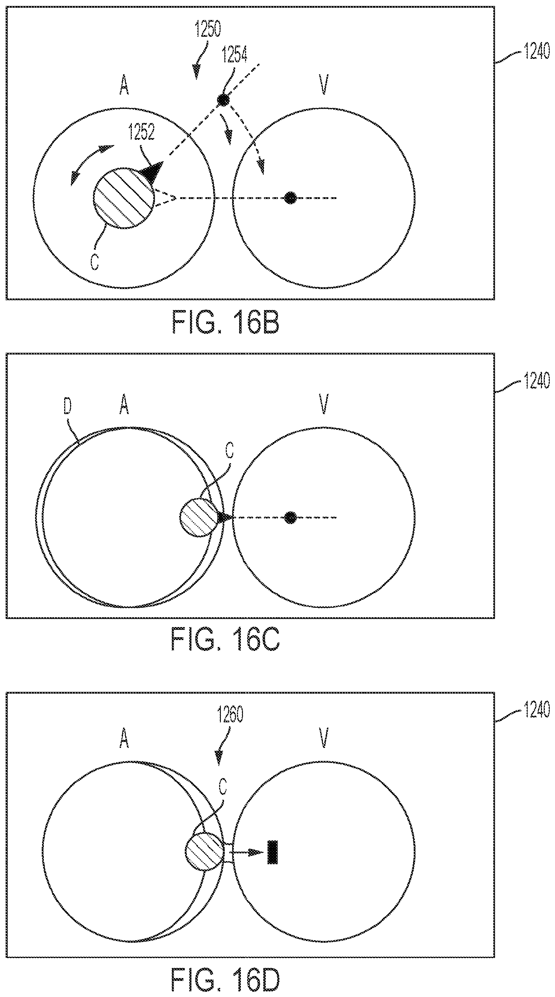

As noted above, the control unit 1204 may be configured to determine a rotational positon of the catheter, and more specifically the treatment portion of the catheter. For example, and as noted above the catheter C may include one or more location sensors (e.g., including information from an intravascular imaging device as described herein) and/or echogenic markers that may be discernable by (e.g., through image recognition processing) or communicatively coupled to the control unit 1204. The one or more location sensors and/or echogenic markers may allow the system to follow and/or track the orientation and/or location of the treatment portion (e.g., the electrode) of the catheter C and produce an overlay such as illustrated in FIG. 16B to guide a physician or other user. In particular, FIG. 16B illustrates an overlay displaying an indicator 1250 which illustrates the rotational position of the treatment portion of the catheter C. For example, arrow 1252 illustrates the position and cutting direction of the treatment portion such that the position and cutting direction of the treatment portion is readily discernible on the display 1240. Additionally, the overlay may depict a cutting depth indicator 1254 which may provide an indication of the overall cutting depth of the treatment portion of the catheter C. FIG. 16B further illustrates rotation of the catheter to the desired orientation so as to be directed toward the treatment location within the blood vessel. During fistula formation, the treatment location may be the portion of the host blood vessel positioned closest to the target blood vessel.

Once in the desired alignment as the operator may determine from the display and overlay projected on the display, the operator can deploy the biasing mechanism D, such as discussed above, to bias the treatment portion into contact with the treatment location of the blood vessel, as illustrated in FIG. 16C. At FIG. 16D, the operator may then apply the vascular treatment, in the illustrated embodiment, a fistula 1260 is formed. Doppler and/or fluoroscopy may then be used to confirm treatment success.

It is noted that while the above-provided example is directed to fistula formation using a single catheter, other treatments are contemplated and possible. Additionally, systems incorporating two catheters may similarly be used. In such cases, each catheter may include an intravascular imaging device and an overlay may provide rotational orientation of both of the catheters.

However, as noted herein, in various embodiments, the imaging device may not be an intravascular imaging device. In such embodiments, and as will be described in greater detail below, an actuator may be coupled to the imaging device and communicatively coupled to the control unit such that the control unit can control motion of the imaging device through the actuator. In such embodiments, the control unit will follow a position of the treatment portion of the catheter with the imaging device such that real-time imaging of the treatment portion of the catheter may be shown on the display without the need for direct operator control of the imaging device.

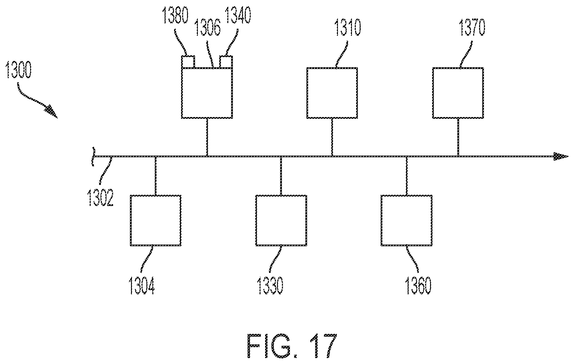

FIG. 17 schematically illustrates an alternative embodiment of a system 1300 for endovascular treatment of a blood vessel. Similar to system 1200 described above, the system 1300 may include a communication path 1302, a control unit 1304, an imaging device 1306, and a display 1310. Unless as otherwise described below, the communication path 1302, the control unit 1304, and the display 1310 may be substantially identical to those described in relation to system 1200, above. In addition, the system 1300 may further include a user input device 1330, an actuator 1340, an electromagnetic field generator 1360, one or more location sensors 1380, and an energy source 1370. It is noted that in various embodiments a fewer or greater number of modules may be included within the system 1300 without departing from the scope of the present disclosure. Additionally, the system 1300 includes one or more catheters such as the catheters described herein above. That is, the system 1300 may include a single catheter system configured to generate a fistula or deliver another type of vascular treatment to a target location within a vessel or a dual catheter system configured to generate a fistula between the two catheters or deliver some other type of vascular treatment. Additionally, it is noted that while various modules are described in relation to system 1300, such modules may be incorporated within system 1200 described above, without departing from the scope of the present disclosure.

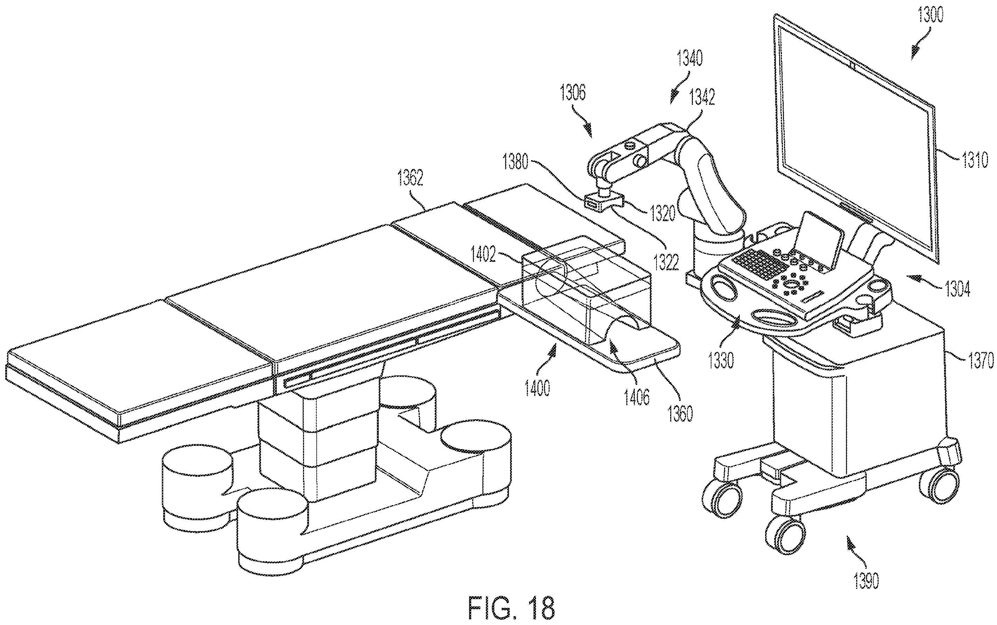

FIG. 18 illustrates generally portions of the system 1300 for endovascular treatment of a blood vessel as may be provide within an exam room or doctor's office. As illustrated, various modules of the system may be mounted to a moveable cart 1390 that is able to be rolled from room to room. Accordingly, treatment locations for endovascular treatment may be improved since the system 1300 may be moved to a user. In other embodiments, there may not be a moveable cart. The moveable cart 1300 may support a variety of components of the system include, but not limited to, the control unit 1304, the imaging device 1306, the user input device 1304, the actuator 1340, etc.

Referring still to FIG. 18, as noted above, the system 1300 may include an imaging device 1306 communicatively coupled to the control unit 1304 over the communication path 1302. The imaging device 1306 may be an intravascular imaging device, as described above, or an external imaging device as illustrated in FIG. 18. The imaging device 1306, may be any device configured to provide images of a blood vessel of a subject. For example, in at least one embodiment, the imaging device is an ultrasound imaging device. In some embodiments, the imaging device is 2D ultrasound device or a 3D ultrasound device capable of capturing images of the desired blood vessel(s) along a frontal (coronal) plane, axial (transverse/cross-sectional) plane, and/or a sagittal plane. The ultrasound device may capable of performing a variety of Doppler functions including, but not limited to, color Doppler, power Doppler, and Doppler vector flow. Using Doppler functions, the system 1300 may be able to analyze vascular flow to determiner arteries and veins. The system 1300 may illustrate these different vessels using different colored overlays, for example, on the display 1310 to allow an operator to quickly and efficiently determine which vessel is an artery and/or vein. Additionally, Doppler functionality may be used to ensure treatment success. For example, Doppler functionality may be able to determine successful fistula creation. For example, Doppler may be used to identify or confirm blood flow through the fistula formed between the two blood vessels. It is noted that other imaging devices/solutions may be used including, fluoroscopy.

Referring now to FIG. 18, the imaging device 1306 includes an ultrasound probe 1340. The ultrasound probe 1340 may be coupled to the moveable cart 1390 via the actuator 1340 (e.g., a robotic arm 1342). In other embodiments, the ultrasound probe 1340 may not be coupled to the moveable cart 1390.

Referring collectively to FIGS. 17 and 18, in some embodiments, the imaging device 1306 may include a catheter tracking sensor 1380 coupled to the ultrasound probe 1320. The control unit 1304 may receive a catheter tracking signal from the catheter tracking sensor 1380 and determine a location of a treatment portion of the catheter. As will be described in greater detail herein, in some embodiments the ultrasound probe 1320 may be coupled to an actuator 1340 configured to move the ultrasound probe 1380 with the treatment portion of the catheter to follow a location of the treatment portion of the catheter in real time (e.g., as the catheter is advanced through the blood vessel). In some embodiments, the control unit 1304 may be configured to perform image recognition on an ultrasound image to recognize a treatment portion of a catheter to determine the location of the treatment portion of the catheter relative to the imaging device 1306. For example, as described above, the one or more catheters may include echogenic markers that the control unit may be configured to recognize with the imaging device 1306. Based on recognizing the echogenic markers, the control unit 1304 may control motion of the imaging device 1406, through the actuator 134, to follow the location of the treatment portion of the catheter in real time as the catheter is advanced through the blood vessel. In addition, the control unit may adjust settings of the ultrasound probe 1320 to automatically focus on the treatment portion of the catheter and surrounding vasculature and display focused images on the display 1310. For example, based on the location of the echogenic markers, the control unit may automatically track a depth of the echogenic markers and adjust image quality settings.

In some embodiments, the catheter tracking sensor 1380 may interact with a location sensor incorporated into the one or more catheters. For example, the catheter tracking sensor 1380 may be able to detect a signal output by the location sensor incorporated into the catheter to follow the location of the treatment portion of the catheter. In some embodiments, the system 1300 may include an electromagnetic field generator board 1360 that will generate an electromagnet field to facilitate tracking between the catheter tracking sensor 1340 and the location sensor of the catheter. Such electromagnetic field generator board 1360 may be situated, for example, underneath a treatment portion of the user to generator an electromagnetic field around the treatment portion of the user. Referring to FIG. 18, the electromagnetic field generator board 1360 may be coupled to a subject support surface 1362. The control unit 1304, may be operable to control activation and deactivation of the electromagnetic field generator board 1360.

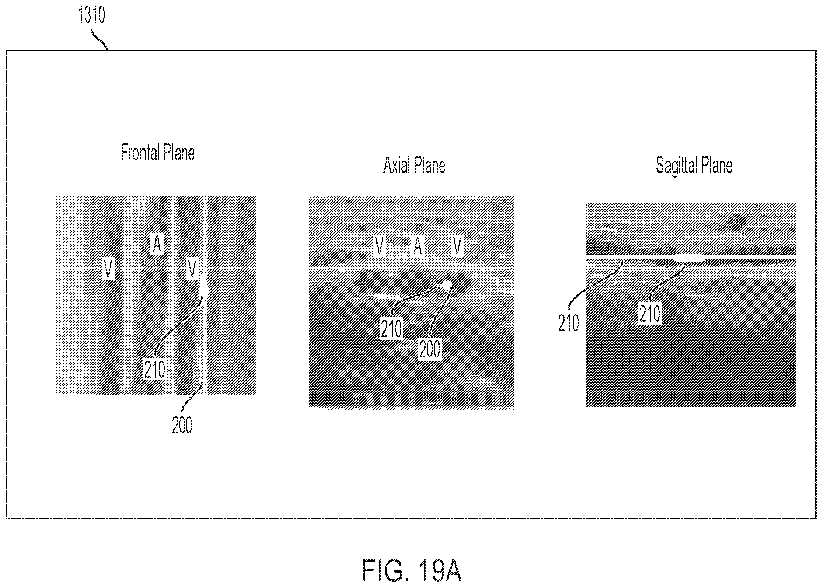

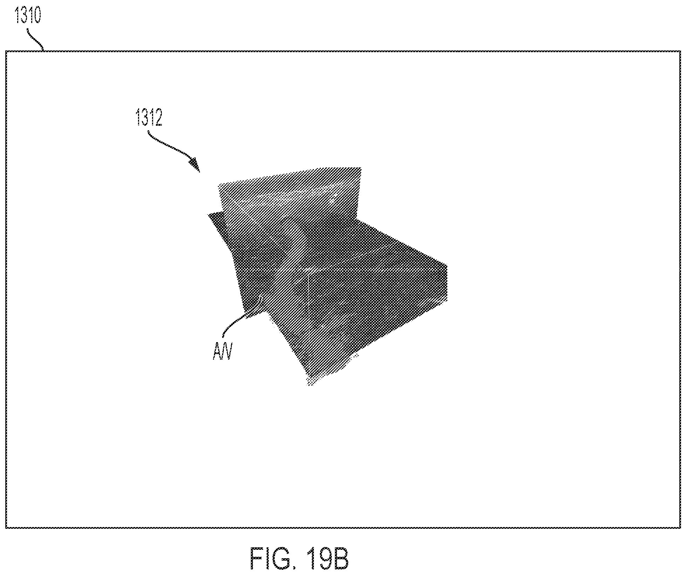

As noted herein, based on the signals of the location sensor and/or the tracking sensor 1340, the control unit 1304 may determine a location of the treatment portion of the catheter and may be configured to automatically focus the settings of the imaging device to display various views of the treatment portion of the catheter including a sagittal view, an axial view, and/or a frontal view. For example, based on the signal of the location sensor, the control unit may automatically track a depth of the sensor and adjust image quality settings. Such views may cut through a center of the treatment portion of the catheter, such that each view shows a cross-sectional view of the catheter along in the sagittal plane, axial plane, and/or the frontal plane. FIG. 19A illustrates a display 1310 showing frontal plane view of a catheter 200 having a treatment portion being advanced through a vein V positioned proximate to an artery A in a frontal plane, axial plane, and a sagittal plane. In some embodiments, the control unit 200 will cause all three view to be display simultaneously. In other embodiments, the control unit 200 may only display two or fewer views. As noted herein, the control unit may 200 be configured to recognize various portions of the vasculature and provide an overly identifying the vasculature. Such overlay may include labels, colors, etc. For example, the overlay may provide a blue overlay to arteries to indicate arterial flow and a blue overlay to veins to indicate venous flow. In some embodiments, the control unit may be configured to generate a 3-Dimensional model of the vasculature of the subject. Referring to FIG. 19B an example 3-Dimensional model 1312 of a portion of the vasculature of the subject. For example, where a catheter having a location sensor and/or echogenic marker is tracked through an artery or vein using an imaging device (e.g., 2D or 3D ultrasound device), the control unit 200 may generate a 3-Dimensional model 1312 of the artery or vein A/V and display the same on the display 1310. Surrounding veins and arteries may also be identified and generated as part of the 3-Dimensional map. Arteries and veins may appear as different colors (e.g., red or blue), or otherwise labeled, to allow an operator to distinguish between the two.

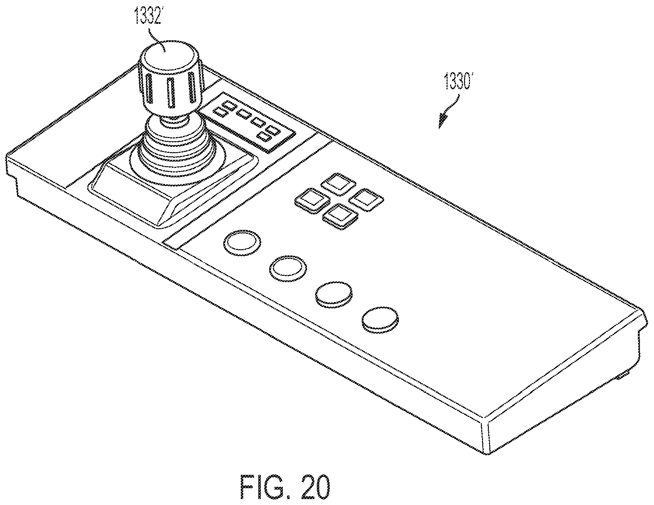

Referring again to FIGS. 17 and 18, the system 1300 may further include one or more user input devices 1330 communicatively coupled to the control unit 1304. The one or more user input devices 1330 may include any device capable of transforming mechanical, optical, audible, or electrical signals into a data signal capable of being transmitted with the communication path 1302. Specifically, a user input device 1330 may include any number of movable objects that transform physical motion into a data signal that can be transmitted over the communication path 1302 such as, for example, joystick, a button, a keyboard, a switch, a knob, a microphone, or the like. FIG. 18 illustrates the user input device 1330 mounted to the moveable cart. Accordingly, an operator may input commands to the control unit 1304 through the user input device 1330. Such commands may include but are not limited, manual control of the imaging device, selecting particular views, or particular overlays. Referring to FIG. 20, an alternative user input device 1330 is generally depicted. Such user input device 1330' is illustrated as including a joystick 1332'. Based on a user input from the one or more user input devices 1330', the control unit may be configured to switch to a manual operation mode from an automatic following mode to allow for manual control of the ultrasound probe 1380 based on input from the one or more user input devices 1330'.

As noted above, the system 1300 may further include an actuator 1340 communicatively coupled to the control unit 1304 and physically coupled to the imaging device 1306 (e.g., ultrasound probe 1320). As noted herein, the control unit 1304 may be configured to move the imaging device 1306 with the actuator 1340. FIG. 18, illustrates an embodiment, wherein the imaging device 1340 includes an ultrasound probe 1320 (e.g., 3D ultrasound probe and/or a 2D ultrasound probe.) The actuator 1340 may include a robotic arm 1342 coupled to the ultrasound probe 1340. The robotic arm 1342 may be capable of 6 (or more) degrees of freedom of motion to control motion of the ultrasound probe 1320. The robotic arm 1342 may be coupled to the moveable cart 1390 so as to be moveable with the moveable cart 1390.

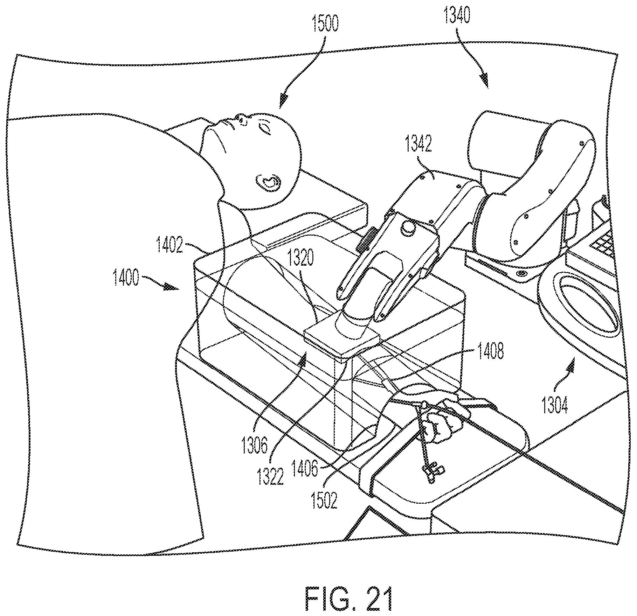

When using an external ultrasound imaging device, the ultrasound probe 1320 may include a subject contact surface 1322. The subject contact surface 1322 may be contacted to a treatment zone (e.g., arm, leg, torso, etc.) of a subject through a flexible subject interface/fluid barrier. That is, the subject contact surface 1322 may directly contact a treatment zone (e.g., arm, leg, etc.) of a patient or may directly contact the flexible fluid barrier 1408, which is directly contacted with the treatment zone of the subject. Such fluid barrier may be provided s part of a media bath 1400 configured to be placed over the treatment zone of a subject. For example, the media bath 1400, such as illustrated in FIG. 18, may include a fluid housing 1402 configured to hold fluid around the treatment zone of a subject. The fluid housing 1402 may include a shaped opening 1406 through which a treatment zone (e.g., arm, leg, etc.) of a subject may be disposed. For example, FIG. 21 illustrates a subject 1500 having an arm 1502 disposed within the shaped opening 1406. A flexible fluid barrier 1408 (e.g., plastic) may be situated between the subject 1500 and the fluid placed within the fluid housing 1402 and conform to the shape of the treatment zone of the subject 1500.

Once the subject 1500 is positioned, the robotic arm 1342 may be controlled either manually or automatically based on logic executed by the control unit 1304, to place the ultrasound probe within the media bath 1400 and in contact the subject contact surface 1322 with the subject 1500. In various embodiments, the system 1300 may be used without catheters to first map a vasculature of the subject to seek a desired location for vascular treatment (e.g., fistula formation). As noted herein, the system 1300 may be placed in an automatic catheter following mode, wherein the control unit 1304 automatically controls the robotic arm 1342 to cause the ultrasound probe 1306 to follow a position of a treatment portion of the catheter as it is advanced through the vasculature of subject to a target treatment location.

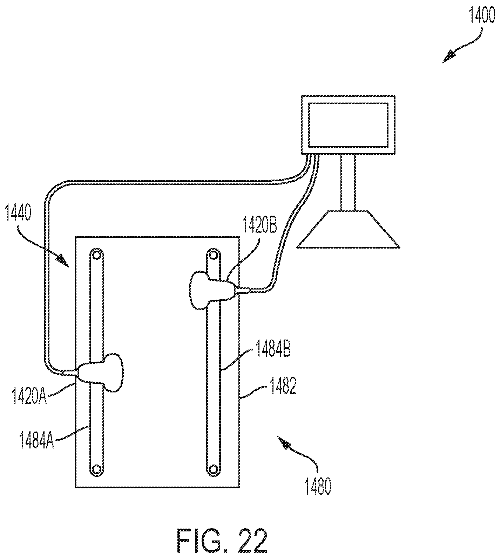

FIG. 22 illustrates an alternative embodiment of a system 1400, wherein an actuator 1440, and the imaging device 1306 is incorporated into a fluid housing 1482 of a media bath 1480. In such embodiments, the fluid housing 1482 may be provided with tracks 1484A/1484B along which one or more ultrasound probes 1420A/1420B (e.g., 2 probes) can track back and forth. In such embodiment, the actuator 1440 may include one or more linear actuators that interact with the one or more ultrasound probes 1420A/1420B to cause the one or more ultrasound probes to move 1420A/1420B along the tracks 1484A/1484B. In some cases, such as embodiments wherein two catheters are separately placed within blood vessels within the subject, each probe may be separately controlled to separately track each catheter and to provide image data of the treatment portion of each catheter.

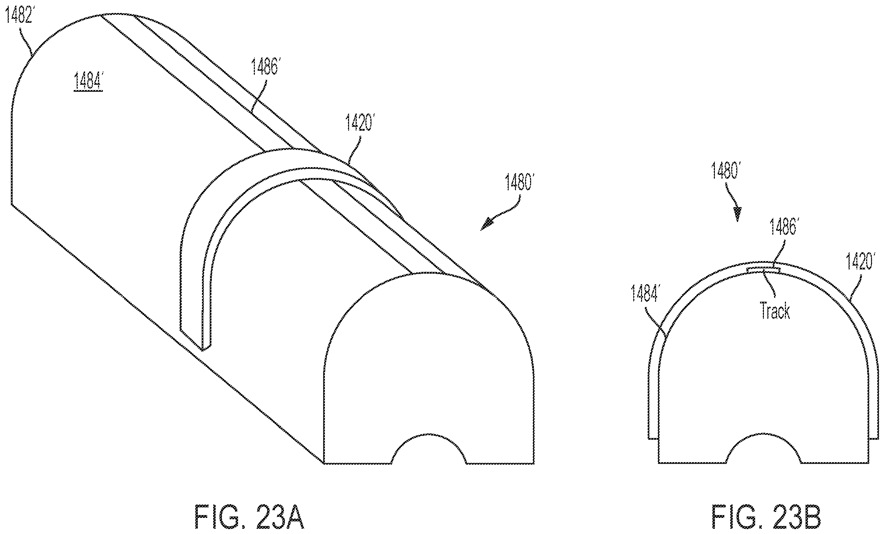

FIGS. 23A and 23B illustrate an alternative media bath 1480' including a fluid housing 1482' and ultrasound probe 1420'. In the illustrated embodiment, the fluid housing 1482' defines a curved surface 1484' over which a curved ultrasound probe 1420' travels. For example, a track 1486' may be coupled to the fluid housing 1482' at an apex of the curved surface 1484'. A linear actuator may be used to cause the curved ultrasound probe to traverse the cured surface 1484' of the fluid housing 1482'.

Referring again to FIGS. 17 and 18, the system 1300 may further include an energy source 1370 communicatively coupled to the control unit 1304. The energy source 1370 may be operatively coupled to the one more catheters via an electrical lead. The energy source 1370 may be an RF energy source to provide energy to an electrode of the treatment portion of the catheter, as described above. The one or more user input devices 1330 may be used to input commands into the control unit 1304 to excite the electrode for fistula formation. The energy source 1370 may, in some embodiments, be mounted to the moveable cart 1390.

As noted above, in some embodiments, the systems as provided herein may be used to scan a perspective anatomical region to build a venous and/or arterial 2D or 3D map and display such map on a display. For example, and as noted above, Doppler functionality may be used to allow the system to determine arterial and venous blood flows (e.g., Doppler functions may measure flow direction, velocity, etc. to allow for determination). The control unit may execute logic to build and 2D or 3D arterial map. In some embodiments, the generated 2D or 3D map may use different colors (e.g., red/blue) to illustrate venous and/or arterial blood flow. Furthermore, when a catheter is advanced through the system it may be shown on the generated the map as it is advanced through the vasculature. Such mapping may be integrated into a larger vessel map (e.g., vessel map of entire arm, leg, body, etc.) to allow a physician to contemplate an entire anatomy of a subject to determine proper treatment locations/zones. Success of treatment may be identified or confirmed using Doppler and indicated on the 2D or 3D map. For example, where a fistula is created, Doppler functionality may be used to identify new flow between adjacent vessels to determine a fistula has been created and adjust the 2D or 3D map to illustrate the same.

In some embodiments, though not shown during vascular treatment, a guidewire having an integrated tracking sensor close to its tip may be inserted into the desired vein or artery and advanced to a target treatment location under guidance of the imaging device. The catheter may then be advanced to the target treatment location over the guidewire using the one or more location sensors as described herein, or the one or more echogenic markers or rings, the treatment portion of the catheter may be tracked and displayed using the display device in real time with or without the use of fluoroscopy.

As noted herein in various embodiments overlays may be positioned over images from the imaging device and displayed on the display to provide indications as to rotational alignment, longitudinal alignment, and distance (e.g., between blood vessels and/or catheters). Additionally, the overlays may also allow a user to determine if the treatment portion in contact with the treatment location within the blood vessel. For example, and as described in greater detail above, a biasing mechanism may be activated to bias the catheter into the correct position within the vessel to deliver treatment (e.g., form a fistula).

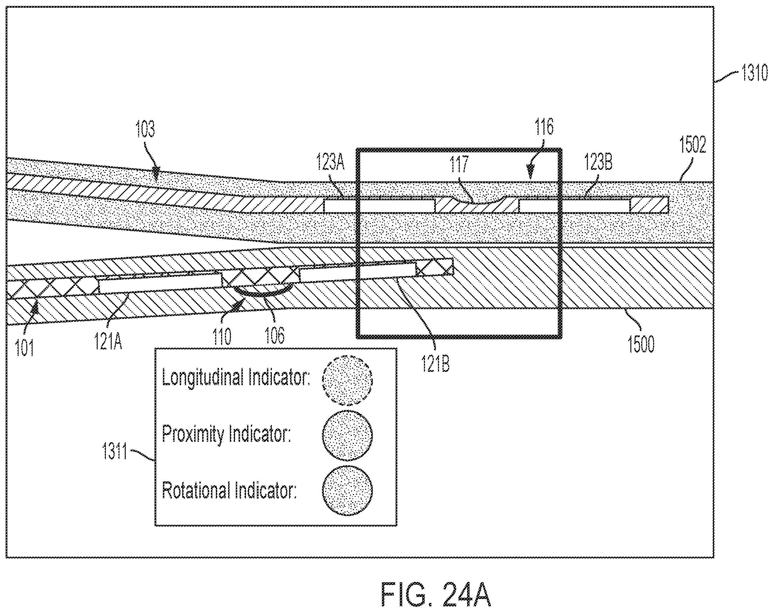

FIGS. 24A-24D illustrate alignment of a two catheter system, such as that described above. The catheters include a 101 first catheter advanced through a first blood vessel 1500 and a second catheter 103 advanced through a second blood vessel 1502. The first catheter 101 having a first treatment portion 110 (e.g., an electrode 106) and one or more location sensors 121A/121B positioned in close proximity to the first treatment portion 110. The second catheter 103 has a second treatment portion 116 (e.g., recess 117) and one or more location sensors 123A/123B positioned in close proximity to the second treatment portion 116. Displayed on the display 1310 is a frontal plane view of the first and second catheters 101/103 within the blood vessels 1500/1502. An indicator 1311 may be displayed on the display 1310 to indicate one or more alignment indicators. For example a longitudinal alignment indicator indicating longitudinal alignment of the first and second catheter 101/103, a proximity indicator, indicating whether the first and second catheter 101/103 are close enough to one another to deliver treatment (e.g., form a fistula), and a rotational indicator, indicating whether the first and second treatment portions 110/116 are rotationally aligned with one another.

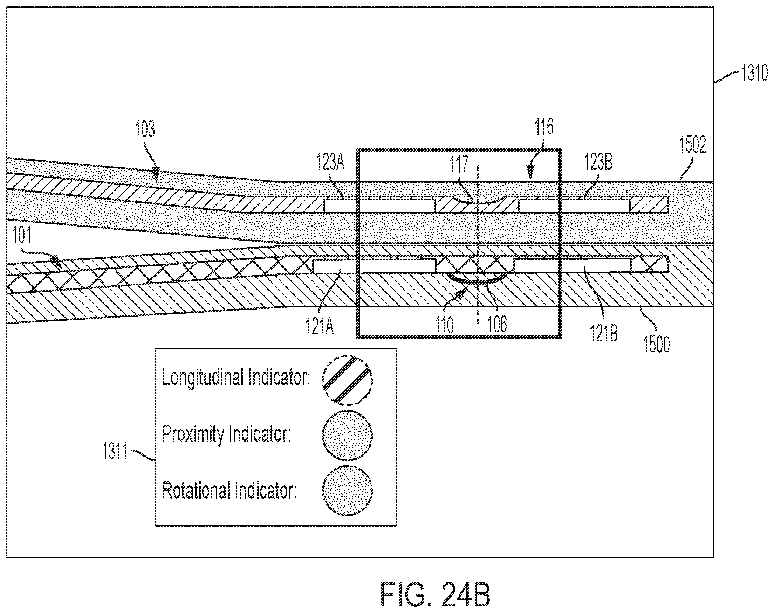

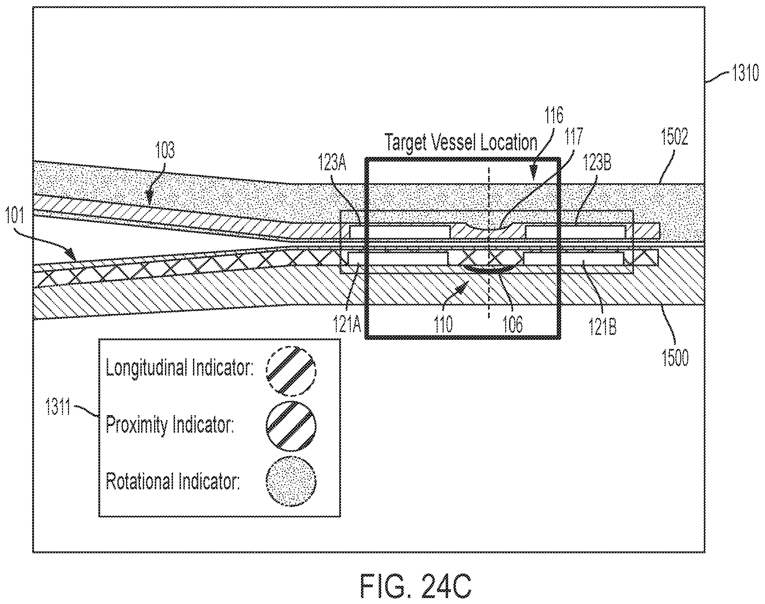

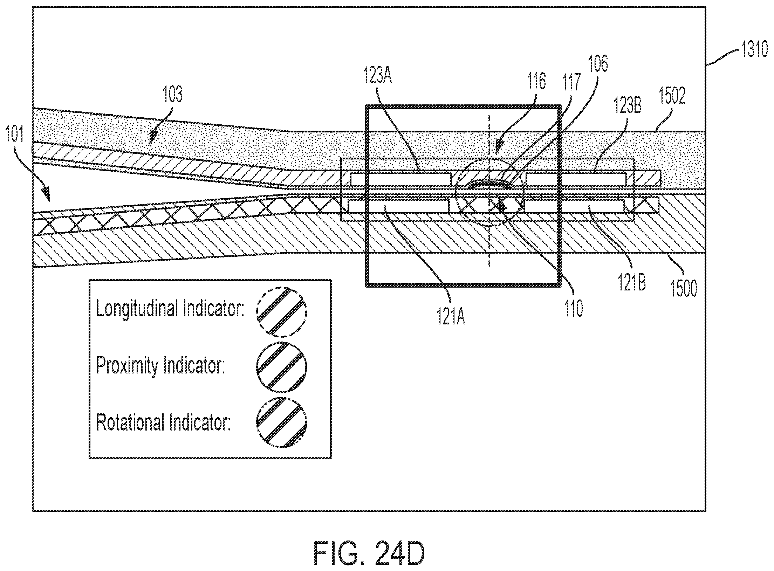

In determining proximity, the control unit may track a location of each of the first and second treatment portions 110/116 based on signals from the one or more location sensors 121A/121B/123A/123B and/or the one more tracking sensors discussed above. Based on these signals, the control unit may determine whether or not the catheters are positioned within a predetermined distance such that fistula formation is position (e.g., less than 2 mm). FIG. 24B illustrates activation of the longitudinal indicator when it is determined by the control unit that the first and second treatment portions 110/116 are longitudinally aligned with one another. FIG. 24C illustrate that the first and second catheters 101/103 having been moved to a suitable proximity to one another such that a fistula may be formed. FIG. 24D illustrate that both the first catheter 101 and the second catheter 103 have proper rotational alignment such that a fistula may be formed. At this point, the electrode 106 of the treatment portion 110 of the first catheter 101 may be activated to ablate tissue sandwiched between the first treatment portion 110 and the second treatment portion 116. Doppler functionality of the imaging device may then be used to determine blood flow between the first blood vessel 1500 and the second blood vessel 1502 to confirm fistula formation.

It is noted, that the external imaging devices described herein may be similarly used for tracking and locating a single catheter system.

As noted herein, devices and methods as provided herein may be used for purposes other than fistula formation. For example, the devices as provided herein may be used for vasculature mapping purpose, arterializing purposes (e.g., arterializing a vein for ischemia in the leg), vessel occlusion, angioplasty, thrombectomy, atherectomy, crossing, drug coated balloon angioplasty, stenting (uncovered and covered), lytic therapy, etc. In addition, methods provided herein, may include multiple treatments and or multiple treatment sites.

It should now be understood that embodiments as described herein are directed the systems, methods, and catheters for endovascular treatment of a blood vessel. In particular, embodiments as described herein include imaging devices (e.g., external or endovascular imaging devices) that provide real-time imaging of a catheter to allow an operator to quickly and efficiently determine the position and alignment of a treatment portion of a catheter. Moreover, embodiments described herein may allow for use of a single catheter for such treatment as fistula formation. Thus simplifying such procedures for operators and patients alike.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A catheter (101, 2000) for endovascular treatment of a blood vessel, the catheter (101, 2000) comprising:
a housing (113, 2008); a treatment portion (116, 2013); a fistula forming element (110, 2014); and an intravascular ultrasound imaging device (240, 2040),
wherein the intravascular ultrasound imaging device (240, 2040) further comprises an array of solid-state transducers (2012)and further comprises one or more flexible circuit elements (2022) electrically coupled to the array of solid-state transducers (2012);
the catheter being **characterized in that**
the one or more flexible circuit elements further comprise one or more multiplexing application-specific integrated circuits configured to enable front-end processing of data generated by the array of solid-state transducers (2012).

2. The catheter (101, 2000) of claim 1,
wherein the array of solid-state transducers (2012) are disposed distal the fistula forming element (110, 2014); or
wherein the array of solid-state transducers (2012) are disposed proximal the fistula forming element (110, 2014); or
wherein the array of solid-state transducers (2012) are coupled to the housing (113, 2008) of the catheter (101, 2000); or
wherein the array of solid-state transducers (2012) are disposed circumferentially around the housing (113, 2008) of the catheter (101, 2000).

3. The catheter (101, 2000) of claim 1,
wherein the one or more flexible circuit elements (2022) are positioned within the housing (113, 2008) of the catheter (101, 2000); or
wherein the one or more flexible circuit elements (2022) are positioned distal the fistula forming element (110, 2014) and proximal the array of solid-state transducers (2012);
or
wherein the one or more flexible circuit elements (2022) are positioned proximal the array of solid-state transducers (2012) and the array of solid-state transducers (2012) are positioned proximal the fistula forming element (110, 2014).

4. The catheter (101, 2000) of claim 1,
wherein the one or more flexible circuit elements (2022) are positioned within the housing (113, 2008) of the catheter (101, 2000) and the catheter (101, 2000) further comprises an intravascular ultrasound imaging device wire (2024) extending proximally from the one or more flexible circuit elements (2022) through the housing (113, 2008) of the catheter (101, 2000).

5. The catheter of claim 1,
wherein the fistula forming element (110, 2014) is an electrode configured to ablate tissue.

6. The catheter (101, 2000) of claim 5,
further comprising an electrode housing (2016), wherein: the electrode housing (2016) is positioned along the treatment portion (116, 2013) of the catheter (101, 2000); and the electrode housing (2016) is at least partially positioned within the housing of the catheter (101, 2000).

7. The catheter (101, 2000) of claim 6,
wherein the electrode (106) is housed within the electrode housing (2016) when the electrode (106) is in a low-profile configuration; or
wherein the electrode (106) is configured to radially extend from the electrode housing (2016) and the housing (113, 2008) of the catheter (101, 2000) when the electrode (106) is in an extended configuration; or
wherein the electrode (106) is spring biased from a low-profile configuration, wherein the electrode (106) is housed within the electrode housing (2016), to an extended configuration, wherein the electrode (106) radially extends from the electrode housing (2016) and the housing (113, 2008) of the catheter (101, 2000).

8. The catheter (101, 2000) of claim 6, further comprising an intravascular ultrasound imaging device wire extending proximally from the one or more flexible circuit elements (2022) through the housing (113, 2008) of the catheter (101, 2000).

9. The catheter (101, 2000) of claim 8,
wherein the electrode housing (2016) further comprises a channel (2026) extending therethrough, the array of solid-state transducers (2012) are positioned distal the electrode housing (2016), the one or more flexible circuit elements (2022) are positioned distal the electrode housing (2016), and the intravascular ultrasound imaging device wire (2024) extends through the channel (2026) extending through the electrode housing (2016); or
wherein the catheter (101, 2000), further comprises: a first lumen (2050) extending through the housing (113, 2008) of the catheter (101, 2000), and a second lumen (2060) extending through the housing (113, 2008) of the catheter (101, 2000), wherein: the electrode (106) further comprises an electrode wire (2020) electrically coupled to the electrode (106) and extending proximally from the electrode (106) through the housing (113, 2008) of the catheter (101, 2000) in the first lumen (2050), and the intravascular ultrasound imaging device wire (2024) extends through the second lumen (2060).

10. The catheter (101, 2000) of claim 8,
further comprising: a first lumen (2050) extending through the housing (113, 2008) of the catheter (101, 2000), and a second lumen (2060) extending through the housing (113, 2008) of the catheter (101, 2000),
wherein the electrode (106) further comprises an electrode wire (2020) electrically coupled to the electrode (106) and extending proximally from the electrode (106) through the housing (113, 2008) of the catheter (101, 2000) in the first lumen (2050), and the intravascular ultrasound imaging device wire (2024) extends through the second lumen (2060), wherein the first lumen (2050) further comprises an insulation sleeve.

11. The catheter (101, 2000) of claim 1,
further comprising a biasing mechanism (220) coupled to the housing (113, 2008), wherein the biasing mechanism (220) is configured to contact a wall of the blood vessel (1180, 1182) to bias the treatment portion (116, 2013) into contact with the wall of the blood vessel (1180, 1182).

12. The catheter (101, 2000) of claim 11,
wherein the biasing mechanism (220) is configured to contact a first radial portion of the wall of the blood vessel (1180, 1182) to bias the treatment portion (116, 2013) toward a second radial portion of the wall of the blood vessel (1180, 1182) opposite the first radial portion; or
wherein the biasing mechanism (220) is a balloon; or
wherein the biasing mechanism (220) is an expandable cage; or
wherein the biasing mechanism (220) comprises one or more expandable wires (611) moveable between a collapsed position and an expanded position wherein at least a portion of the one or more expandable wires (611) are spaced from an outer wall of the housing (113, 2008) of the catheter (101, 2000).

13. The catheter (101, 2000) of claim 10,
wherein the housing (113, 2008) further comprises a rapid exchange tip (2002) at a distal end of the catheter (101, 2000); or
wherein the housing (113, 2008) is comprised of one or more polymers, wherein preferably the housing (113, 2008) is comprised of silicone rubber, nylon, polyurethane, polyethylene terephthalate, latex, or thermoplastic elastomers, or wherein preferably the housing (113, 2008) is further comprised of a braided polymer.

## Patentansprüche

1. Katheter (101, 2000) zur endovaskulären Behandlung eines Blutgefäßes, der Katheter (101, 2000) umfassend:
ein Gehäuse (113, 2008); einen Behandlungsabschnitt (116, 2013); ein Fistelbildungselement (110, 2014); und eine intravaskuläre Ultraschallbildgebungsvorrichtung (240, 2040),
wobei die intravaskuläre Ultraschallbildgebungsvorrichtung (240, 2040) weiter eine Anordnung von Festkörperwandlern (2012) umfasst und weiter ein oder mehrere flexible Schaltungselemente (2022) umfasst, die elektrisch mit der Anordnung von Festkörperwandlern (2012) gekoppelt sind;
wobei der Katheter **dadurch gekennzeichnet ist, dass**
das eine oder die mehreren flexiblen Schaltungselemente weiter eine oder mehrere multiplexende anwendungsspezifische integrierte Schaltungen umfassen, die dazu ausgebildet sind, eine Front-End-Verarbeitung von Daten zu ermöglichen, die von der Anordnung von Festkörperwandlern (2012) erzeugt werden.

2. Katheter (101, 2000) nach Anspruch 1,
wobei die Anordnung von Festkörperwandlern (2012) distal zum Fistelbildungselement (110, 2014) angeordnet ist; oder
wobei die Anordnung von Festkörperwandlern (2012) proximal zum Fistelbildungselement (110, 2014) angeordnet ist; oder
wobei die Anordnung von Festkörperwandlern (2012) mit dem Gehäuse (113, 2008) des Katheters (101, 2000) gekoppelt ist; oder
wobei die Anordnung von Festkörperwandlern (2012) umlaufend um das Gehäuse (113, 2008) des Katheters (101, 2000) angeordnet ist.

3. Katheter (101, 2000) nach Anspruch 1,
wobei das eine oder die mehreren flexiblen Schaltungselemente (2022) innerhalb des Gehäuses (113, 2008) des Katheters (101, 2000) positioniert sind; oder
wobei das eine oder die mehreren flexiblen Schaltungselemente (2022) distal zum Fistelbildungselement (110, 2014) und proximal zur Anordnung von Festkörperwandlern (2012) positioniert sind; oder
wobei das eine oder die mehreren flexiblen Schaltungselemente (2022) proximal zur Anordnung von Festkörperwandlern (2012) positioniert sind und die Anordnung von Festkörperwandlern (2012) proximal zum Fistelbildungselement (110, 2014) positioniert ist.

4. Katheter (101, 2000) nach Anspruch 1,
wobei das eine oder die mehreren flexiblen Schaltungselemente (2022) innerhalb des Gehäuses (113, 2008) des Katheters (101, 2000) positioniert sind und der Katheter (101, 2000) weiter einen Draht (2024) der intravaskulären Ultraschallbildgebungsvorrichtung umfasst, der sich proximal von dem einen oder den mehreren flexiblen Schaltungselementen (2022) durch das Gehäuse (113, 2008) des Katheters (101, 2000) erstreckt.

5. Katheter nach Anspruch 1,
wobei das Fistelbildungselement (110, 2014) eine Elektrode ist, die dazu ausgebildet ist, Gewebe abzutragen.

6. Katheter (101, 2000) nach Anspruch 5,
weiter umfassend ein Elektrodengehäuse (2016), wobei: das Elektrodengehäuse (2016) entlang des Behandlungsabschnitts (116, 2013) des Katheters (101, 2000) positioniert ist; und das Elektrodengehäuse (2016) mindestens teilweise innerhalb des Gehäuses des Katheters (101, 2000) positioniert ist.

7. Katheter (101, 2000) nach Anspruch 6,
wobei die Elektrode (106) innerhalb des Elektrodengehäuses (2016) untergebracht ist, wenn sich die Elektrode (106) in einer flachen Konfiguration befindet; oder
wobei die Elektrode (106) dazu ausgebildet ist, sich radial vom Elektrodengehäuse (2016) und dem Gehäuse (113, 2008) des Katheters (101, 2000) zu erstrecken, wenn sich die Elektrode (106) in einer ausgefahrenen Konfiguration befindet; oder
wobei die Elektrode (106) durch eine Federkraft von einer flachen Konfiguration, in der die Elektrode (106) im Elektrodengehäuse (2016) untergebracht ist, in eine ausgefahrene Konfiguration, in der die Elektrode (106) sich radial von dem Elektrodengehäuse (2016) und dem Gehäuse (113, 2008) des Katheters (101, 2000) erstreckt, vorgespannt ist.

8. Katheter (101, 2000) nach Anspruch 6, weiter umfassend
einen Draht der intravaskulären Ultraschallbildgebungsvorrichtung, der sich proximal von dem einen oder den mehreren flexiblen Schaltungselementen (2022) durch das Gehäuse (113, 2008) des Katheters (101, 2000) erstreckt.

9. Katheter (101, 2000) nach Anspruch 8,
wobei das Elektrodengehäuse (2016) weiter einen sich dort hindurch erstreckenden Kanal (2026) umfasst, die Anordnung von Festkörperwandlern (2012) distal zum Elektrodengehäuse (2016) positioniert ist, das eine oder die mehreren flexiblen Schaltungselemente (2022) distal zum Elektrodengehäuse (2016) positioniert sind und der Draht (2024) der intravaskulären Ultraschallbildgebungsvorrichtung sich durch den Kanal (2026) erstreckt, der sich durch das Elektrodengehäuse (2016) erstreckt; oder
wobei der Katheter (101, 2000) weiter umfasst: ein erstes Lumen (2050), das sich durch das Gehäuse (113, 2008) des Katheters (101, 2000) erstreckt, und ein zweites Lumen (2060), das sich durch das Gehäuse (113, 2008) des Katheters (101, 2000) erstreckt, wobei: die Elektrode (106) weiter einen Elektrodendraht (2020) umfasst, der elektrisch mit der Elektrode (106) gekoppelt ist und sich proximal von der Elektrode (106) durch das Gehäuse (113, 2008) des Katheters (101, 2000) im ersten Lumen (2050) erstreckt, und der Draht (2024) der intravaskulären Ultraschallbildgebungsvorrichtung sich durch das zweite Lumen (2060) erstreckt.

10. Katheter (101, 2000) nach Anspruch 8,
weiter umfassend: ein erstes Lumen (2050), das sich durch das Gehäuse (113, 2008) des Katheters (101, 2000) erstreckt, und ein zweites Lumen (2060), das sich durch das Gehäuse (113, 2008) des Katheters (101, 2000) erstreckt,
wobei die Elektrode (106) weiter einen Elektrodendraht (2020) umfasst, der elektrisch mit der Elektrode (106) gekoppelt ist und sich proximal von der Elektrode (106) durch das Gehäuse (113, 2008) des Katheters (101, 2000) im ersten Lumen (2050) erstreckt, und der Draht (2024) der intravaskulären Ultraschallbildgebungsvorrichtung sich durch das zweite Lumen (2060) erstreckt, wobei das erste Lumen (2050) weiter eine Isolierhülse umfasst.

11. Katheter (101, 2000) nach Anspruch 1,
weiter umfassend einen Vorspannmechanismus (220), der mit dem Gehäuse (113, 2008) gekoppelt ist, wobei der Vorspannmechanismus (220) dazu ausgebildet ist, eine Wand des Blutgefäßes (1180, 1182) zu kontaktieren, um den Behandlungsabschnitt (116, 2013) in Kontakt mit der Wand des Blutgefäßes (1180, 1182) vorzuspannen.

12. Katheter (101, 2000) nach Anspruch 11,
wobei der Vorspannmechanismus (220) dazu ausgebildet ist, einen ersten radialen Abschnitt der Wand des Blutgefäßes (1180, 1182) zu kontaktieren, um den Behandlungsabschnitt (116, 2013) in Richtung eines zweiten radialen Abschnitts der Wand des Blutgefäßes (1180, 1182), der dem ersten radialen Abschnitt gegenüberliegt, vorzuspannen; oder
wobei der Vorspannmechanismus (220) ein Ballon ist; oder
wobei der Vorspannmechanismus (220) ein expandierbarer Käfig ist; oder
wobei der Vorspannmechanismus (220) einen oder mehrere expandierbare Drähte (611) umfasst, die zwischen einer kollabierten Position und einer expandierten Position beweglich sind, wobei mindestens ein Abschnitt des einen oder der mehreren expandierbaren Drähte (611) von einer Außenwand des Gehäuses (113, 2008) des Katheters (101, 2000) beabstandet ist.

13. Katheter (101, 2000) nach Anspruch 10,
wobei das Gehäuse (113, 2008) weiter eine Schnellwechselspitze (2002) an einem distalen Ende des Katheters (101, 2000) umfasst; oder
wobei das Gehäuse (113, 2008) aus einem oder mehreren Polymeren besteht, wobei das Gehäuse (113, 2008) vorzugsweise aus Silikonkautschuk, Nylon, Polyurethan, Polyethylenterephthalat, Latex oder thermoplastischen Elastomeren besteht, oder wobei das Gehäuse (113, 2008) vorzugsweise weiter aus einem geflochtenen Polymer besteht.

## Revendications

1. Cathéter (101, 2000) pour le traitement endovasculaire d'un vaisseau sanguin, le cathéter (101, 2000) comprenant :
un boîtier (113, 2008) ; une partie (116 ; 2013) traitement ; un élément (110, 2014) formant fistule ; et un dispositif (240, 2040) d'imagerie intravasculaire par ultrasons,
dans lequel le dispositif (240, 2040) d'imagerie intravasculaire par ultrasons comprend en outre un réseau de transducteurs (2012) à semi-conducteur et comprend en outre un ou plusieurs éléments (2022) de circuit flexibles couplés électriquement au réseau de transducteurs (2012) à semi-conducteur ;
le cathéter étant **caractérisé en ce que**
les un ou plusieurs éléments de circuit flexible comprennent en outre un ou plusieurs circuits intégrés à application spécifique de multiplexage configurés pour permettre le traitement frontal de données générées par le réseau de transducteurs (2012) à semi-conducteurs.

2. Cathéter (101, 2000) selon la revendication 1,
dans lequel le réseau de transducteurs (2012) à semi-conducteur est disposé de manière distale par rapport à l'élément formant fistule (110, 2014) ; ou
dans lequel le réseau de transducteurs (2012) à semi-conducteur est disposé de manière proximale par rapport à l'élément formant fistule (110, 2014) ; ou
dans lequel le réseau de transducteurs (2012) à semi-conducteur est couplé au boîtier (113, 2008) du cathéter (101, 2000) ; ou
dans lequel le réseau de transducteurs (2012) à semi-conducteur est disposé de manière circonférentielle autour du boîtier (113, 2008) du cathéter (101, 2000).

3. Cathéter (101, 2000) selon la revendication 1,
dans lequel les un ou plusieurs éléments (2022) de circuit flexible sont positionnés à l'intérieur du boîtier (113, 2008) du cathéter (101, 2000) ; ou
dans lequel les un ou plusieurs éléments (2022) de circuit flexible sont positionnés de manière distale par rapport à l'élément (110, 2014) formant fistule et de manière proximale par rapport au réseau de transducteurs (2012) à semi-conducteurs ; ou
dans lequel les un ou plusieurs éléments (2022) de circuit flexible sont positionnés de manière proximale par rapport au réseau de transducteurs (2012) à semi-conducteur et le réseau de transducteurs (2012) à semi-conducteur est positionné de manière proximale par rapport à l'élément (110, 2014) formant fistule.

4. Cathéter (101, 2000) selon la revendication 1,
dans lequel les un ou plusieurs éléments (2022) de circuit flexible sont positionnés à l'intérieur du boîtier (113, 2008) du cathéter (101, 2000) et le cathéter (101, 2000) comprend en outre un fil (2024) de dispositif d'imagerie intravasculaire par ultrasons s'étendant de manière proximale à partir des un ou plusieurs éléments (2022) de circuit flexible à travers le boîtier (113, 2008) du cathéter (101, 2000).

5. Cathéter selon la revendication 1,
dans lequel l'élément (110, 2014) formant fistule est une électrode configurée pour réaliser une ablation de tissu.

6. Cathéter (101, 2000) selon la revendication 5,
comprenant en outre un boîtier (2016) d'électrode, dans lequel : le boîtier (2016) d'électrode est positionné le long de la partie (116, 2013) traitement du cathéter (101, 2000) ; et le boîtier (2016) d'électrode est au moins partiellement positionné à l'intérieur du boîtier du cathéter (101, 2000).

7. Cathéter (101, 2000) selon la revendication 6,
dans lequel l'électrode (106) est logée à l'intérieur du boîtier (2016) d'électrode lorsque l'électrode (106) est dans une configuration à profil bas ; ou
dans lequel l'électrode (106) est configurée pour s'étendre radialement à partir du boîtier (2016) d'électrode et du boîtier (113, 2008) du cathéter (101, 2000) lorsque l'électrode (106) est dans une configuration étendue ; ou
dans lequel l'électrode (106) est sollicitée par ressort à partir d'une configuration à profil bas, dans lequel l'électrode (106) est logée à l'intérieur du boîtier (2016) d'électrode, jusqu'à une configuration étendue, dans lequel l'électrode (106) s'étend radialement à partir du boîtier (2016) d'électrode et du boîtier (113, 2008) du cathéter (101, 2000).

8. Cathéter (101, 2000) selon la revendication 6, comprenant en outre
un fil de dispositif d'imagerie intravasculaire par ultrasons s'étendant de manière proximale à partir des un ou plusieurs éléments (2022) de circuit flexible à travers le boîtier (113, 2008) du cathéter (101, 2000).

9. Cathéter (101, 2000) selon la revendication 8,
dans lequel le boîtier (2016) d'électrode comprend en outre un canal (2026) s'étendant à travers celui-ci, le réseau de transducteurs (2012) à semi-conducteurs est positionné de manière distale par rapport au boîtier (2016) d'électrode, les un ou plusieurs éléments (2022) de circuit flexible sont positionnés de manière distale par rapport au boîtier (2016) d'électrode, et le fil (2024) de dispositif d'imagerie intravasculaire par ultrasons s'étend à travers le canal (2026) s'étendant à travers le boîtier (2016) d'électrode ; ou
dans lequel le cathéter (101, 2000), comprend en outre : une première lumière (2050) s'étendant à travers le boîtier (113, 2008) du cathéter (101, 2000), et une seconde lumière (2060) s'étendant à travers le boîtier (113, 2008) du cathéter (101, 2000), dans lequel : l'électrode (106) comprend en outre un fil (2020) d'électrode couplé électriquement à l'électrode (106) et s'étendant de manière proximale à partir de l'électrode (106) à travers le boîtier (113, 2008) du cathéter (101, 2000) dans la première lumière (2050), et le fil (2024) de dispositif d'imagerie intravasculaire par ultrasons s'étend à travers la seconde lumière (2060).

10. Cathéter (101, 2000) selon la revendication 8,
comprenant en outre : une première lumière (2050) s'étendant à travers le boîtier (113, 2008) du cathéter (101, 2000), et une seconde lumière (2060) s'étendant à travers le boîtier (113, 2008) du cathéter (101, 2000),
dans lequel l'électrode (106) comprend en outre un fil (2020) d'électrode couplé électriquement à l'électrode (106) et s'étendant de manière proximale à partir de l'électrode (106) à travers le boîtier (113, 2008) du cathéter (101, 2000) dans la première lumière (2050), et le fil (2024) de dispositif d'imagerie intravasculaire par ultrasons s'étend à travers la seconde lumière (2060), dans lequel la première lumière (2050) comprend en outre un manchon d'isolation.

11. Cathéter (101, 2000) selon la revendication 1,
comprenant en outre un mécanisme de sollicitation (220) couplé au boîtier (113, 2008), dans lequel le mécanisme de sollicitation (220) est configuré pour entrer en contact avec une paroi du vaisseau (1180, 1182) sanguin pour solliciter la partie (116, 2013) traitement en contact avec la paroi du vaisseau (1180, 1182) sanguin.

12. Cathéter (101, 2000) selon la revendication 11,
dans lequel le mécanisme de sollicitation (220) est configuré pour entrer en contact avec une première partie radiale de la paroi du vaisseau (1180, 1182) sanguin pour solliciter la partie (116, 2013) traitement vers une seconde partie radiale de la paroi du vaisseau (1180, 1182) sanguin opposée à la première partie radiale ; ou
dans lequel le mécanisme de sollicitation (220) est un ballonnet ; ou
dans lequel le mécanisme de sollicitation (220) est une cage expansible ; ou
dans lequel le mécanisme de sollicitation (220) comprend un ou plusieurs fils (611) expansibles pouvant être déplacés entre une position repliée et une position déployée dans laquelle au moins une partie des un ou plusieurs fils (611) expansibles est espacée d'une paroi externe du boîtier (113, 2008) du cathéter (101, 2000).

13. Cathéter (101, 2000) selon la revendication 10,
dans lequel le boîtier (113, 2008) comprend en outre un embout (2002) d'échange rapide à une extrémité distale du cathéter (101, 2000) ; ou
dans lequel le boîtier (113, 2008) est composé d'un ou de plusieurs polymères, dans lequel de préférence le boîtier (113, 2008) est composé de caoutchouc de silicone, de nylon, de polyuréthane, de poly(téréphtalate d'éthylène), de latex, ou d'élastomères thermoplastiques, ou dans lequel de préférence le boîtier (113, 2008) est en outre composé d'un polymère tressé.
